# EUROPÄISCHE PATENTANMELDUNG

(11) **EP 1 918 378 A1**
(43) Veröffentlichungstag der Anmeldung: **07.05.2008**
(21) Anmeldenummer: 07123778.8
(22) Anmeldetag: 16.12.2004
(51) Int. Cl.: C12N 15/77, C12N 9/02, C12N 1/21

(54) **Psod-Expressionseinheiten**

(30) Priorität: 18.12.2003 DE 10359660
(62) Teilanmeldung aus: 04803950.7
(71) Anmelder: BASF SE, 67056 Ludwigshafen (DE)
(72) Erfinder: Kröger, Burkhard, 67117 Limburgerhof (DE); Zelder, Oskar, 67346 Speyer (DE); Klopprogge, Corinna, 68239 Mannheim (DE); Schröder, Hartwig, 69226 Nußloch (DE); Haefner, Stefan, 67346 Speyer (DE)

(57) **Zusammenfassung**

Die vorliegende Erfindung betrifft die Verwendung von Nukleinsäuresequenzen zur Regulation der Transkription und Expression von Genen, die neuen Promotoren und Expressionseinheiten selbst, Verfahren zur Veränderung oder Verursachung der Transkriptionsrate und/oder Expressionsrate von Genen, Expressionskasetten, enthaltend die Expressionseinheiten, genetisch veränderte Mikroorganismen mit veränderter oder verursachter Trankriptionsrate und/oder Expressionsrate sowie Verfahren zur Herstellung von biosynthetischen Produkten durch Kultivierung der genetisch veränderten Mikroorganismen.

<110>  BASF Aktiengesellschaft

<120>  Psod-Expressionseinheiten

<130>  PF 55185/Mec

<140> 20030320
<141>

<160> 44

<210> 1
<211> 173
<212> DNA
<213> Corynebacterium glutamicum

<220>
<223> SUPEROXID\DISMUTASE\RXA03119\

<400> 1
gctgccaatt attccgggct tgtgacccgc tacccgataa ataggtcggc tgaaaaattt 60

cgttgcaata tcaacaaaaa ggcctatcat tgggaggtgt cgcaccaagt acttttgcga 120

agcgccatct gacggatttt caaaagatgt atatgctcgg tgcggaaacc tac        173


<210> 2
<211> 191
<212> DNA
<213> Corynebacterium glutamicum

<220>
<223> SUPEROXID\DISMUTASE\RXA03119\

<400> 2
agctgccaat tattccgggc ttgtgacccg ctacccgata aataggtcgg ctgaaaaatt 60

tcgttgcaat atcaacaaaa aggcctatca ttgggaggtg tcgcaccaag tacttttgcg 120

aagcgccatc tgacggattt tcaaaagatg tatatgctcg gtgcggaaac ctacgaaagg 180

atttttaccc c                                                      191


<210> 3
<211> 1365
<212> DNA
<213> Corynebacterium glutamicum

<220>
<223> RXA00077

<400> 3
atgaatgatg agaatattca aagctccaac tatcagccat tcccgagttt tgacgattgg 60

aaacagatcg aggtgtcgct cttagatgtc atcgaatcct cacgccattt ttctgatttg 120

aaagatagca ctgatcgttc tgcgttagat gctgcgctag agagagcaaa aagagctgcc 180

gcagttgata ccaatgccat agaaggaatc ttccaaactg atcgcggttt tacccataca 240

gttgcaacgc aggtaggggc ttgggagcaa caaatggcga tgaaaggcaa acatgttaag 300

cctgcgtttg acgatactct agaaggcttt gagtatgttc tcgatgcagt aactggtaga 360

actccaatct ctcagcaatg gattagaaat ttgcacgccg tcattctgcg gagccaagaa 420

agccacgagg tttttacagc cgttggagtc caaaatcagg cgcttcagaa aggcgagtat 480

aaaactcagc caaatagtcc acagcgctca gatggatctg tacatgcata cgccccagtt 540

gaagatactc ctgctgaaat ggctagattt atttcagaac ttgaatctaa ggaattctta 600

gcagccgaga aggttattca agctgcctat gcccactatg ctttcgtatg tattcatcct 660

tttgcagatg ggaatggacg agttgcacga gccttggcta gtgtttttct atacaaagat 720

cctggtgtcc ctctcgtaat ctaccaagat caacgcagag attacatcca tgctctagaa 780

gcagcggaca agaataaccc gctcctgctg attagattct ttgctgaacg agtgaccgat 840

actattaact ctattatcgt tgatctcact accccgatcg cgggtaaatc tggttcggct 900

aagctttcgg atgcgctacg ccccactcgc gtattaccag aattacatga tgctgcacat 960

aggctccaag aaagtttatt tacagaaatc cgatctcgat tggatgaaga aggaaaaagg 1020

aatgggttgg agtttctact tcaacggatt tttatcggtt ccccattcaa tctgccagag 1080

ggctataacg ctttccctga tagctattgt ctgaccttag ctttcaatag caactctcca 1140

aaacaaatct tccacccgct atccatagta atagcagctc gagatgggaa aagagcgagc 1200

agcgacctcg tggcagctac ttctattgga tacaactttc acgcttacgg acgtgaagtc 1260

gagcctgttg ttactgaaag ctttcgagaa cgtgtgaaaa tttacgccga cgggattgta 1320

gatcacttct taaccgaact ggctaaaaag tttcaacaga attaa 1365

<210> 4
<211> 454
<212> PRT
<213> Corynebacterium glutamicum

<400> 4
Met Asn Asp Glu Asn Ile Gln Ser Ser Asn Tyr Gln Pro Phe Pro Ser
1               5                   10                  15

Phe Asp Asp Trp Lys Gln Ile Glu Val Ser Leu Leu Asp Val Ile Glu
            20                  25                  30

Ser Ser Arg His Phe Ser Asp Leu Lys Asp Ser Thr Asp Arg Ser Ala
        35                  40                  45

Leu Asp Ala Ala Leu Glu Arg Ala Lys Arg Ala Ala Ala Val Asp Thr
    50                  55                  60

Asn Ala Ile Glu Gly Ile Phe Gln Thr Asp Arg Gly Phe Thr His Thr
65                  70                  75                  80

Val Ala Thr Gln Val Gly Ala Trp Glu Gln Gln Met Ala Met Lys Gly
                85                  90                  95

Lys His Val Lys Pro Ala Phe Asp Asp Thr Leu Glu Gly Phe Glu Tyr
            100                 105                 110

Val Leu Asp Ala Val Thr Gly Arg Thr Pro Ile Ser Gln Gln Trp Ile
        115                 120                 125

Arg Asn Leu His Ala Val Ile Leu Arg Ser Gln Glu Ser His Glu Val
    130                 135                 140

Phe Thr Ala Val Gly Val Gln Asn Gln Ala Leu Gln Lys Gly Glu Tyr
145                 150                 155                 160

Lys Thr Gln Pro Asn Ser Pro Gln Arg Ser Asp Gly Ser Val His Ala
                165                 170                 175

Tyr Ala Pro Val Glu Asp Thr Pro Ala Glu Met Ala Arg Phe Ile Ser
            180                 185                 190

Glu Leu Glu Ser Lys Glu Phe Leu Ala Ala Glu Lys Val Ile Gln Ala
        195                 200                 205

Ala Tyr Ala His Tyr Ala Phe Val Cys Ile His Pro Phe Ala Asp Gly
    210                 215                 220

Asn Gly Arg Val Ala Arg Ala Leu Ala Ser Val Phe Leu Tyr Lys Asp
225                 230                 235                 240

Pro Gly Val Pro Leu Val Ile Tyr Gln Asp Gln Arg Arg Asp Tyr Ile
                245                 250                 255

His Ala Leu Glu Ala Ala Asp Lys Asn Asn Pro Leu Leu Leu Ile Arg
            260                 265                 270

Phe Phe Ala Glu Arg Val Thr Asp Thr Ile Asn Ser Ile Ile Val Asp
        275                 280                 285

Leu Thr Thr Pro Ile Ala Gly Lys Ser Gly Ser Ala Lys Leu Ser Asp
    290                 295                 300

Ala Leu Arg Pro Thr Arg Val Leu Pro Glu Leu His Asp Ala Ala His
305                 310                 315                 320

Arg Leu Gln Glu Ser Leu Phe Thr Glu Ile Arg Ser Arg Leu Asp Glu
                325                 330                 335

Glu Gly Lys Arg Asn Gly Leu Glu Phe Leu Leu Gln Arg Ile Phe Ile
            340                 345                 350

Gly Ser Pro Phe Asn Leu Pro Glu Gly Tyr Asn Ala Phe Pro Asp Ser
        355                 360                 365

Tyr Cys Leu Thr Leu Ala Phe Asn Ser Asn Ser Pro Lys Gln Ile Phe
    370                 375                 380

His Pro Leu Ser Ile Val Ile Ala Ala Arg Asp Gly Lys Arg Ala Ser
385                 390                 395                 400

Ser Asp Leu Val Ala Ala Thr Ser Ile Gly Tyr Asn Phe His Ala Tyr
                405                 410                 415

Gly Arg Glu Val Glu Pro Val Val Thr Glu Ser Phe Arg Glu Arg Val
            420                 425                 430

Lys Ile Tyr Ala Asp Gly Ile Val Asp His Phe Leu Thr Glu Leu Ala
        435                 440                 445

Lys Lys Phe Gln Gln Asn
    450


<210> 5
<211> 35
<212> DNA
<213> Corynebacterium glutamicum

<220>
<223> SEQ_ID5

<400> 5
gagagagaga cgcgtcccag tggctgagac gcatc                                35


<210> 6
<211> 34
<212> DNA
<213> Corynebacterium glutamicum

<220>
<223> SEQ_ID_6

<400> 6
ctctctctgt cgacgaattc aatcttacgg cctg                                 34


<210> 7
<211> 4323
<212> DNA
<213> Corynebacterium glutamicum

<220>
<223> SEQ_ID_7

<220>

<221>  misc_feature
<222>  (457)..(1248)
<223>  KanR

<220>
<221>  misc_feature
<222>  (1515)..(2375)
<223>  Ori-EC   (pMB)

<220>
<221>  misc_feature
<222>  (1515)..(2375)
<223>  Ori-EC   (pMB)  complement

<220>
<221>  misc_feature
<222>  (2418)..(3839)
<223>  SacB  complement

<220>
<221>  misc_feature
<222>  (3840)..(4302)
<223>  PsacB   complement

<400> 7
tcgagaggcc tgacgtcggg cccggtacca cgcgtcatat gactagttcg gacctaggga 60

tatcgtcgac atcgatgctc ttctgcgtta attaacaatt gggatcctct agacccggga 120

tttaaatcgc tagcgggctg ctaaaggaag cggaacacgt agaaagccag tccgcagaaa 180

cggtgctgac cccggatgaa tgtcagctac tgggctatct ggacaaggga aaacgcaagc 240

gcaaagagaa agcaggtagc ttgcagtggg cttacatggc gatagctaga ctgggcggtt 300

ttatggacag caagcgaacc ggaattgcca gctggggcgc cctctggtaa ggttgggaag 360

ccctgcaaag taaactggat ggctttcttg ccgccaagga tctgatggcg caggggatca 420

agatctgatc aagagacagg atgaggatcg tttcgcatga ttgaacaaga tggattgcac 480

gcaggttctc cggccgcttg ggtggagagg ctattcggct atgactgggc acaacagaca 540

atcggctgct ctgatgccgc cgtgttccgg ctgtcagcgc aggggcgccc ggttcttttt 600

gtcaagaccg acctgtccgg tgccctgaat gaactgcagg acgaggcagc gcggctatcg 660

tggctggcca cgacgggcgt tccttgcgca gctgtgctcg acgttgtcac tgaagcggga 720

agggactggc tgctattggg cgaagtgccg gggcaggatc tcctgtcatc tcaccttgct 780

cctgccgaga aagtatccat catggctgat gcaatgcggc ggctgcatac gcttgatccg 840

gctacctgcc cattcgacca ccaagcgaaa catcgcatcg agcgagcacg tactcggatg 900

gaagccggtc ttgtcgatca ggatgatctg gacgaagagc atcaggggct cgcgccagcc 960

gaactgttcg ccaggctcaa ggcgcgcatg cccgacggcg aggatctcgt cgtgacccat 1020

ggcgatgcct gcttgccgaa tatcatggtg gaaaatggcc gcttttctgg attcatcgac 1080

tgtggccggc tgggtgtggc ggaccgctat caggacatag cgttggctac ccgtgatatt 1140

gctgaagagc ttggcggcga atgggctgac cgcttcctcg tgctttacgg tatcgccgct 1200

cccgattcgc agcgcatcgc cttctatcgc cttcttgacg agttcttctg agcgggactc 1260

tggggttcga aatgaccgac caagcgacgc ccaacctgcc atcacgagat ttcgattcca 1320

ccgccgcctt ctatgaaagg ttgggcttcg gaatcgtttt ccgggacgcc ggctggatga 1380

tcctccagcg cggggatctc atgctggagt tcttcgccca cgctagcggc gcgccggccg 1440

gcccggtgtg aaataccgca cagatgcgta aggagaaaat accgcatcag gcgctcttcc 1500

gcttcctcgc tcactgactc gctgcgctcg gtcgttcggc tgcggcgagc ggtatcagct 1560

cactcaaagg cggtaatacg gttatccaca gaatcagggg ataacgcagg aaagaacatg 1620
tgagcaaaag gccagcaaaa ggccaggaac cgtaaaaagg ccgcgttgct ggcgtttttc 1680
cataggctcc gcccccctga cgagcatcac aaaaatcgac gctcaagtca gaggtggcga 1740
aacccgacag gactataaag ataccaggcg tttccccctg gaagctccct cgtgcgctct 1800
cctgttccga ccctgccgct taccggatac ctgtccgcct ttctcccttc gggaagcgtg 1860
gcgctttctc atagctcacg ctgtaggtat ctcagttcgg tgtaggtcgt tcgctccaag 1920
ctgggctgtg tgcacgaacc ccccgttcag cccgaccgct gcgccttatc cggtaactat 1980
cgtcttgagt ccaacccggt aagacacgac ttatcgccac tggcagcagc cactggtaac 2040
aggattagca gagcgaggta tgtaggcggt gctacagagt tcttgaagtg gtggcctaac 2100
tacggctaca ctagaaggac agtatttggt atctgcgctc tgctgaagcc agttaccttc 2160
ggaaaaagag ttggtagctc ttgatccggc aaacaaacca ccgctggtag cggtggtttt 2220
tttgtttgca agcagcagat tacgcgcaga aaaaaaggat ctcaagaaga tcctttgatc 2280
ttttctacgg ggtctgacgc tcagtggaac gaaaactcac gttaagggat tttggtcatg 2340
agattatcaa aaaggatctt cacctagatc cttttaaagg ccggccgcgg ccgccatcgg 2400
cattttcttt tgcgttttta tttgttaact gttaattgtc cttgttcaag gatgctgtct 2460
ttgacaacag atgttttctt gcctttgatg ttcagcagga agctcggcgc aaacgttgat 2520
tgtttgtctg cgtagaatcc tctgtttgtc atatagcttg taatcacgac attgtttcct 2580
ttcgcttgag gtacagcgaa gtgtgagtaa gtaaaggtta catcgttagg atcaagatcc 2640
atttttaaca caaggccagt tttgttcagc ggcttgtatg ggccagttaa agaattagaa 2700
acataaccaa gcatgtaaat atcgttagac gtaatgccgt caatcgtcat ttttgatccg 2760
cgggagtcag tgaacaggta ccatttgccg ttcattttaa agacgttcgc gcgttcaatt 2820
tcatctgtta ctgtgttaga tgcaatcagc ggtttcatca cttttttcag tgtgtaatca 2880
tcgtttagct caatcatacc gagagcgccg tttgctaact cagccgtgcg ttttttatcg 2940
ctttgcagaa gtttttgact ttcttgacgg aagaatgatg tgcttttgcc atagtatgct 3000
ttgttaaata aagattcttc gccttggtag ccatcttcag ttccagtgtt tgcttcaaat 3060
actaagtatt tgtggccttt atcttctacg tagtgaggat ctctcagcgt atggttgtcg 3120
cctgagctgt agttgccttc atcgatgaac tgctgtacat tttgatacgt ttttccgtca 3180
ccgtcaaaga ttgatttata atcctctaca ccgttgatgt tcaaagagct gtctgatgct 3240
gatacgttaa cttgtgcagt tgtcagtgtt tgtttgccgt aatgtttacc ggagaaatca 3300
gtgtagaata aacggatttt tccgtcagat gtaaatgtgg ctgaacctga ccattcttgt 3360
gtttggtctt ttaggataga atcatttgca tcgaatttgt cgctgtcttt aaagacgcgg 3420
ccagcgtttt tccagctgtc aatagaagtt tcgccgactt tttgatagaa catgtaaatc 3480
gatgtgtcat ccgcattttt aggatctccg gctaatgcaa agacgatgtg gtagccgtga 3540
tagtttgcga cagtgccgtc agcgttttgt aatggccagc tgtcccaaac gtccaggcct 3600
tttgcagaag agatattttt aattgtggac gaatcaaatt cagaaacttg atatttttca 3660
tttttttgct gttcagggat ttgcagcata tcatggcgtg taatatggga aatgccgtat 3720
gtttccttat atggcttttg gttcgtttct ttcgcaaacg cttgagttgc gcctcctgcc 3780
agcagtgcgg tagtaaaggt taatactgtt gcttgttttg caaacttttt gatgttcatc 3840
gttcatgtct ccttttttat gtactgtgtt agcggtctgc ttcttccagc cctcctgttt 3900

gaagatggca agttagttac gcacaataaa aaaagaccta aaatatgtaa ggggtgacgc 3960

caaagtatac actttgccct ttacacattt taggtcttgc ctgctttatc agtaacaaac 4020

ccgcgcgatt tacttttcga cctcattcta ttagactctc gtttggattg caactggtct 4080

attttcctct tttgtttgat agaaaatcat aaaaggattt gcagactacg ggcctaaaga 4140

actaaaaaat ctatctgttt cttttcattc tctgtatttt ttatagtttc tgttgcatgg 4200

gcataaagtt gcctttttaa tcacaattca gaaaatatca taatatctca tttcactaaa 4260

taatagtgaa cggcaggtat atgtgatggg ttaaaaagga tcggcggccg ctcgatttaa 4320

atc 4323

<210> 8
<211> 5860
<212> DNA
<213> Corynebacterium glutamicum

<220>
<223> PCIS\LYSC

<220>
<221> misc_feature
<222> (155)..(1420)
<223> lysC

<220>
<221> misc_feature
<222> (1974)..(2765)
<223> KanR

<220>
<221> misc_feature
<222> (3032)..(3892)
<223> Ori-EC complement

<220>
<221> misc_feature
<222> (3913)..(3934)
<223> sacB downstream complement

<220>
<221> misc_feature
<222> (3935)..(5356)
<223> sacB (Bacillus Subtilis) complement

<220>
<221> misc_feature
<222> (5357)..(5819)
<223> Promotor sacB complement

<400> 8
cccggtacca cgcgtcccag tggctgagac gcatccgcta aagccccagg aaccctgtgc 60

agaaagaaaa cactcctctg gctaggtaga cacagtttat aaaggtagag ttgagcgggt 120

aactgtcagc acgtagatcg aaaggtgcac aaaggtggcc ctggtcgtac agaaatatgg 180

cggttcctcg cttgagagtg cggaacgcat tagaaacgtc gctgaacgga tcgttgccac 240

caagaaggct ggaaatgatg tcgtggttgt ctgctccgca atgggagaca ccacggatga 300

acttctagaa cttgcagcgg cagtgaatcc cgttccgcca gctcgtgaaa tggatatgct 360

cctgactgct ggtgagcgta tttctaacgc tctcgtcgcc atggctattg agtcccttgg 420

cgcagaagcc caatctttca cgggctctca ggctggtgtg ctcaccaccg agcgccacgg 480

aaacgcacgc attgttgatg tcactccagg tcgtgtgcgt gaagcactcg atgagggcaa 540

gatctgcatt gttgctggtt tccagggtgt taataaagaa acccgcgatg tcaccacgtt 600

gggtcgtggt ggttctgaca ccactgcagt tgcgttggca gctgctttga acgctgatgt 660
gtgtgagatt tactcggacg ttgacggtgt gtataccgct gacccgcgca tcgttcctaa 720
tgcacagaag ctggaaaagc tcagcttcga agaaatgctg gaacttgctg ctgttggctc 780
caagattttg gtgctgcgca gtgttgaata cgctcgtgca ttcaatgtgc cacttcgcgt 840
acgctcgtct tatagtaatg atcccggcac tttgattgcc ggctctatgg aggatattcc 900
tgtggaagaa gcagtcctta ccggtgtcgc aaccgacaag tccgaagcca aagtaaccgt 960
tctgggtatt tccgataagc caggcgaggc tgcgaaggtt ttccgtgcgt tggctgatgc 1020
agaaatcaac attgacatgg ttctgcagaa cgtctcttct gtagaagacg gcaccaccga 1080
catcaccttc acctgccctc gttccgacgg ccgccgcgcg atggagatct tgaagaagct 1140
tcaggttcag ggcaactgga ccaatgtgct ttacgacgac caggtcggca aagtctccct 1200
cgtgggtgct ggcatgaagt ctcacccagg tgttaccgca gagttcatgg aagctctgcg 1260
cgatgtcaac gtgaacatcg aattgatttc cacctctgag attcgtattt ccgtgctgat 1320
ccgtgaagat gatctggatg ctgctgcacg tgcattgcat gagcagttcc agctgggcgg 1380
cgaagacgaa gccgtcgttt atgcaggcac cggacgctaa agttttaaag gagtagtttt 1440
acaatgacca ccatcgcagt tgttggtgca accggccagg tcggccaggt tatgcgcacc 1500
cttttggaag agcgcaattt cccagctgac actgttcgtt tctttgcttc cccacgttcc 1560
gcaggccgta agattgaatt cgtcgacatc gatgctcttc tgcgttaatt aacaattggg 1620
atcctctaga cccgggattt aaatcgctag cgggctgcta aaggaagcgg aacacgtaga 1680
aagccagtcc gcagaaacgg tgctgacccc ggatgaatgt cagctactgg gctatctgga 1740
caagggaaaa cgcaagcgca aagagaaagc aggtagcttg cagtgggctt acatggcgat 1800
agctagactg ggcggtttta tggacagcaa gcgaaccgga attgccagct ggggcgccct 1860
ctggtaaggt tgggaagccc tgcaaagtaa actggatggc tttcttgccg ccaaggatct 1920
gatggcgcag gggatcaaga tctgatcaag agacaggatg aggatcgttt cgcatgattg 1980
aacaagatgg attgcacgca ggttctccgg ccgcttgggt ggagaggcta ttcggctatg 2040
actgggcaca acagacaatc ggctgctctg atgccgccgt gttccggctg tcagcgcagg 2100
ggcgcccggt tctttttgtc aagaccgacc tgtccggtgc cctgaatgaa ctgcaggacg 2160
aggcagcgcg gctatcgtgg ctggccacga cgggcgttcc ttgcgcagct gtgctcgacg 2220
ttgtcactga agcgggaagg gactggctgc tattgggcga agtgccgggg caggatctcc 2280
tgtcatctca ccttgctcct gccgagaaag tatccatcat ggctgatgca atgcggcggc 2340
tgcatacgct tgatccggct acctgcccat tcgaccacca agcgaaacat cgcatcgagc 2400
gagcacgtac tcggatggaa gccggtcttg tcgatcagga tgatctggac gaagagcatc 2460
aggggctcgc gccagccgaa ctgttcgcca ggctcaaggc gcgcatgccc gacggcgagg 2520
atctcgtcgt gacccatggc gatgcctgct tgccgaatat catggtggaa aatggccgct 2580
tttctggatt catcgactgt ggccggctgg gtgtggcgga ccgctatcag gacatagcgt 2640
tggctacccg tgatattgct gaagagcttg gcggcgaatg ggctgaccgc ttcctcgtgc 2700
tttacggtat cgccgctccc gattcgcagc gcatcgcctt ctatcgcctt cttgacgagt 2760
tcttctgagc gggactctgg ggttcgaaat gaccgaccaa gcgacgccca acctgccatc 2820
acgagatttc gattccaccg ccgccttcta tgaaaggttg ggcttcggaa tcgttttccg 2880
ggacgccggc tggatgatcc tccagcgcgg ggatctcatg ctggagttct tcgcccacgc 2940

tagcggcgcg ccggccggcc cggtgtgaaa taccgcacag atgcgtaagg agaaaatacc 3000
gcatcaggcg ctcttccgct tcctcgctca ctgactcgct gcgctcggtc gttcggctgc 3060
ggcgagcggt atcagctcac tcaaaggcgg taatacggtt atccacagaa tcaggggata 3120
acgcaggaaa gaacatgtga gcaaaaggcc agcaaaaggc caggaaccgt aaaaaggccg 3180
cgttgctggc gtttttccat aggctccgcc cccctgacga gcatcacaaa aatcgacgct 3240
caagtcagag gtggcgaaac ccgacaggac tataaagata ccaggcgttt ccccctggaa 3300
gctccctcgt gcgctctcct gttccgaccc tgccgcttac cggatacctg tccgcctttc 3360
tcccttcggg aagcgtggcg ctttctcata gctcacgctg taggtatctc agttcggtgt 3420
aggtcgttcg ctccaagctg ggctgtgtgc acgaaccccc cgttcagccc gaccgctgcg 3480
ccttatccgg taactatcgt cttgagtcca acccggtaag acacgactta tcgccactgg 3540
cagcagccac tggtaacagg attagcagag cgaggtatgt aggcggtgct acagagttct 3600
tgaagtggtg gcctaactac ggctacacta gaaggacagt atttggtatc tgcgctctgc 3660
tgaagccagt taccttcgga aaaagagttg gtagctcttg atccggcaaa caaaccaccg 3720
ctggtagcgg tggttttttt gtttgcaagc agcagattac gcgcagaaaa aaaggatctc 3780
aagaagatcc tttgatcttt tctacggggt ctgacgctca gtggaacgaa aactcacgtt 3840
aagggatttt ggtcatgaga ttatcaaaaa ggatcttcac ctagatcctt ttaaaggccg 3900
gccgcggccg ccatcggcat tttcttttgc gtttttattt gttaactgtt aattgtcctt 3960
gttcaaggat gctgtctttg acaacagatg ttttcttgcc tttgatgttc agcaggaagc 4020
tcggcgcaaa cgttgattgt ttgtctgcgt agaatcctct gtttgtcata tagcttgtaa 4080
tcacgacatt gtttcctttc gcttgaggta cagcgaagtg tgagtaagta aaggttacat 4140
cgttaggatc aagatccatt tttaacacaa ggccagtttt gttcagcggc ttgtatgggc 4200
cagttaaaga attagaaaca taaccaagca tgtaaatatc gttagacgta atgccgtcaa 4260
tcgtcatttt tgatccgcgg gagtcagtga acaggtacca tttgccgttc attttaaaga 4320
cgttcgcgcg ttcaatttca tctgttactg tgttagatgc aatcagcggt ttcatcactt 4380
ttttcagtgt gtaatcatcg tttagctcaa tcataccgag agcgccgttt gctaactcag 4440
ccgtgcgttt tttatcgctt tgcagaagtt tttgactttc ttgacggaag aatgatgtgc 4500
ttttgccata gtatgctttg ttaaataaag attcttcgcc ttggtagcca tcttcagttc 4560
cagtgtttgc ttcaaatact aagtatttgt ggcctttatc ttctacgtag tgaggatctc 4620
tcagcgtatg gttgtcgcct gagctgtagt tgccttcatc gatgaactgc tgtacatttt 4680
gatacgtttt tccgtcaccg tcaaagattg atttataatc ctctacaccg ttgatgttca 4740
aagagctgtc tgatgctgat acgttaactt gtgcagttgt cagtgtttgt ttgccgtaat 4800
gtttaccgga gaaatcagtg tagaataaac ggatttttcc gtcagatgta aatgtggctg 4860
aacctgacca ttcttgtgtt tggtctttta ggatagaatc atttgcatcg aatttgtcgc 4920
tgtctttaaa gacgcggcca gcgtttttcc agctgtcaat agaagtttcg ccgacttttt 4980
gatagaacat gtaaatcgat gtgtcatccg catttttagg atctccggct aatgcaaaga 5040
cgatgtggta gccgtgatag tttgcgacag tgccgtcagc gttttgtaat ggccagctgt 5100
cccaaacgtc caggcctttt gcagaagaga tatttttaat tgtggacgaa tcaaattcag 5160
aaacttgata tttttcattt ttttgctgtt cagggatttg cagcatatca tggcgtgtaa 5220

tatgggaaat gccgtatgtt tccttatatg gcttttggtt cgtttctttc gcaaacgctt 5280

gagttgcgcc tcctgccagc agtgcggtag taaaggttaa tactgttgct tgttttgcaa 5340

actttttgat gttcatcgtt catgtctcct tttttatgta ctgtgttagc ggtctgcttc 5400

ttccagccct cctgtttgaa gatggcaagt tagttacgca caataaaaaa agacctaaaa 5460

tatgtaaggg gtgacgccaa agtatacact ttgcccttta cacattttag gtcttgcctg 5520

ctttatcagt aacaaacccg cgcgatttac ttttcgacct cattctatta gactctcgtt 5580

tggattgcaa ctggtctatt ttcctctttt gtttgataga aaatcataaa aggatttgca 5640

gactacgggc ctaaagaact aaaaaatcta tctgtttctt ttcattctct gtatttttta 5700

tagtttctgt tgcatgggca taaagttgcc tttttaatca caattcagaa aatatcataa 5760

tatctcattt cactaaataa tagtgaacgg caggtatatg tgatgggtta aaaaggatcg 5820

gcggccgctc gatttaaatc tcgagaggcc tgacgtcggg 5860

<210> 9
<211> 38
<212> DNA
<213> Corynebacterium glutamicum

<220>
<223> SEQ_ID_9

<400> 9
cggcaccacc gacatcatct tcacctgccc tcgttccg 38

<210> 10
<211> 38
<212> DNA
<213> Corynebacterium glutamicum

<220>
<223> SEQ_ID_10

<400> 10
cggaacgagg gcaggtgaag atgatgtcgg tggtgccg 38

<210> 11
<211> 1263
<212> DNA
<213> Corynebacterium glutamicum

<220>
<223> LYSC-GEN

<400> 11
gtggccctgg tcgtacagaa atatggcggt tcctcgcttg agagtgcgga acgcattaga 60

aacgtcgctg aacggatcgt tgccaccaag aaggctggaa atgatgtcgt ggttgtctgc 120

tccgcaatgg gagacaccac ggatgaactt ctagaacttg cagcggcagt gaatcccgtt 180

ccgccagctc gtgaaatgga tatgctcctg actgctggtg agcgtatttc taacgctctc 240

gtcgccatgg ctattgagtc ccttggcgca gaagcccaat ctttcacggg ctctcaggct 300

ggtgtgctca ccaccgagcg ccacggaaac gcacgcattg ttgatgtcac tccaggtcgt 360

gtgcgtgaag cactcgatga gggcaagatc tgcattgttg ctggtttcca gggtgttaat 420

aaagaaaccc gcgatgtcac cacgttgggt cgtggtggtt ctgacaccac tgcagttgcg 480

ttggcagctg ctttgaacgc tgatgtgtgt gagatttact cggacgttga cggtgtgtat 540

accgctgacc cgcgcatcgt tcctaatgca cagaagctgg aaaagctcag cttcgaagaa 600

atgctggaac ttgctgctgt tggctccaag attttggtgc tgcgcagtgt tgaatacgct 660

cgtgcattca atgtgccact tcgcgtacgc tcgtcttata gtaatgatcc cggcactttg 720

attgccggct ctatggagga tattcctgtg gaagaagcag tccttaccgg tgtcgcaacc 780

gacaagtccg aagccaaagt aaccgttctg ggtatttccg ataagccagg cgaggctgcg 840

aaggttttcc gtgcgttggc tgatgcagaa atcaacattg acatggttct gcagaacgtc 900

tcttctgtag aagacggcac caccgacatc accttcacct gccctcgttc cgacggccgc 960

cgcgcgatgg agatcttgaa gaagcttcag gttcagggca actggaccaa tgtgctttac 1020

gacgaccagg tcggcaaagt ctccctcgtg ggtgctggca tgaagtctca cccaggtgtt 1080

accgcagagt tcatggaagc tctgcgcgat gtcaacgtga acatcgaatt gatttccacc 1140

tctgagattc gtatttccgt gctgatccgt gaagatgatc tggatgctgc tgcacgtgca 1200

ttgcatgagc agttccagct gggcggcgaa gacgaagccg tcgtttatgc aggcaccgga 1260

cgc 1263

<210> 12
<211> 5860
<212> DNA
<213> Corynebacterium glutamicum

<220>
<223> PCIS\LYSC\THR311ILE

<220>
<221> misc_feature
<222> (155)..(1420)
<223> LysC

<220>
<221> misc_feature
<222> (1974)..(2765)
<223> KanR

<220>
<221> misc_feature
<222> (3032)..(3892)
<223> Ori-EC (pMB)

<220>
<221> misc_feature
<222> (3913)..(3934)
<223> C_region : sacB downstream (complement)

<220>
<221> misc_feature
<222> (3935)..(5356)
<223> sacB Bacillus Subtilis (complement)

<220>
<221> misc_feature
<222> (5357)..(5819)
<223> Promotor sacB (complement)

<400> 12
cccggtacca cgcgtcccag tggctgagac gcatccgcta aagccccagg aaccctgtgc 60

agaaagaaaa cactcctctg gctaggtaga cacagtttat aaaggtagag ttgagcgggt 120

aactgtcagc acgtagatcg aaaggtgcac aaaggtggcc ctggtcgtac agaaatatgg 180

cggttcctcg cttgagagtg cggaacgcat tagaaacgtc gctgaacgga tcgttgccac 240

caagaaggct ggaaatgatg tcgtggttgt ctgctccgca atgggagaca ccacggatga 300

acttctagaa cttgcagcgg cagtgaatcc cgttccgcca gctcgtgaaa tggatatgct 360

cctgactgct ggtgagcgta tttctaacgc tctcgtcgcc atggctattg agtcccttgg 420

cgcagaagcc caatctttca cgggctctca ggctggtgtg ctcaccaccg agcgccacgg 480
aaacgcacgc attgttgatg tcactccagg tcgtgtgcgt gaagcactcg atgagggcaa 540
gatctgcatt gttgctggtt tccagggtgt taataaagaa acccgcgatg tcaccacgtt 600
gggtcgtggt ggttctgaca ccactgcagt tgcgttggca gctgctttga acgctgatgt 660
gtgtgagatt tactcggacg ttgacggtgt gtataccgct gacccgcgca tcgttcctaa 720
tgcacagaag ctggaaaagc tcagcttcga agaaatgctg gaacttgctg ctgttggctc 780
caagattttg gtgctgcgca gtgttgaata cgctcgtgca ttcaatgtgc cacttcgcgt 840
acgctcgtct tatagtaatg atcccggcac tttgattgcc ggctctatgg aggatattcc 900
tgtggaagaa gcagtcctta ccggtgtcgc aaccgacaag tccgaagcca aagtaaccgt 960
tctgggtatt tccgataagc caggcgaggc tgcgaaggtt ttccgtgcgt tggctgatgc 1020
agaaatcaac attgacatgg ttctgcagaa cgtctcttct gtagaagacg gcaccaccga 1080
catcatcttc acctgccctc gttccgacgg ccgccgcgcg atggagatct tgaagaagct 1140
tcaggttcag ggcaactgga ccaatgtgct ttacgacgac caggtcggca aagtctccct 1200
cgtgggtgct ggcatgaagt ctcacccagg tgttaccgca gagttcatgg aagctctgcg 1260
cgatgtcaac gtgaacatcg aattgatttc cacctctgag attcgtattt ccgtgctgat 1320
ccgtgaagat gatctggatg ctgctgcacg tgcattgcat gagcagttcc agctgggcgg 1380
cgaagacgaa gccgtcgttt atgcaggcac cggacgctaa agttttaaag gagtagtttt 1440
acaatgacca ccatcgcagt tgttggtgca accggccagg tcggccaggt tatgcgcacc 1500
cttttggaag agcgcaattt cccagctgac actgttcgtt tctttgcttc cccacgttcc 1560
gcaggccgta agattgaatt cgtcgacatc gatgctcttc tgcgttaatt aacaattggg 1620
atcctctaga cccgggattt aaatcgctag cgggctgcta aaggaagcgg aacacgtaga 1680
aagccagtcc gcagaaacgg tgctgacccc ggatgaatgt cagctactgg gctatctgga 1740
caagggaaaa cgcaagcgca aagagaaagc aggtagcttg cagtgggctt acatggcgat 1800
agctagactg ggcggtttta tggacagcaa gcgaaccgga attgccagct ggggcgccct 1860
ctggtaaggt tgggaagccc tgcaaagtaa actggatggc tttcttgccg ccaaggatct 1920
gatggcgcag gggatcaaga tctgatcaag agacaggatg aggatcgttt cgcatgattg 1980
aacaagatgg attgcacgca ggttctccgg ccgcttgggt ggagaggcta ttcggctatg 2040
actgggcaca acagacaatc ggctgctctg atgccgccgt gttccggctg tcagcgcagg 2100
ggcgcccggt tctttttgtc aagaccgacc tgtccggtgc cctgaatgaa ctgcaggacg 2160
aggcagcgcg gctatcgtgg ctggccacga cgggcgttcc ttgcgcagct gtgctcgacg 2220
ttgtcactga agcgggaagg gactggctgc tattgggcga agtgccgggg caggatctcc 2280
tgtcatctca ccttgctcct gccgagaaag tatccatcat ggctgatgca atgcggcggc 2340
tgcatacgct tgatccggct acctgcccat tcgaccacca agcgaaacat cgcatcgagc 2400
gagcacgtac tcggatggaa gccggtcttg tcgatcagga tgatctggac gaagagcatc 2460
aggggctcgc gccagccgaa ctgttcgcca ggctcaaggc gcgcatgccc gacggcgagg 2520
atctcgtcgt gacccatggc gatgcctgct tgccgaatat catggtggaa aatggccgct 2580
tttctggatt catcgactgt ggccggctgg gtgtggcgga ccgctatcag gacatagcgt 2640
tggctacccg tgatattgct gaagagcttg gcggcgaatg ggctgaccgc ttcctcgtgc 2700
tttacggtat cgccgctccc gattcgcagc gcatcgcctt ctatcgcctt cttgacgagt 2760

tcttctgagc gggactctgg ggttcgaaat gaccgaccaa gcgacgccca acctgccatc 2820
acgagatttc gattccaccg ccgccttcta tgaaaggttg ggcttcggaa tcgttttccg 2880
ggacgccggc tggatgatcc tccagcgcgg ggatctcatg ctggagttct tcgcccacgc 2940
tagcggcgcg ccggccggcc cggtgtgaaa taccgcacag atgcgtaagg agaaaatacc 3000
gcatcaggcg ctcttccgct tcctcgctca ctgactcgct gcgctcggtc gttcggctgc 3060
ggcgagcggt atcagctcac tcaaaggcgg taatacggtt atccacagaa tcaggggata 3120
acgcaggaaa gaacatgtga gcaaaaggcc agcaaaaggc caggaaccgt aaaaaggccg 3180
cgttgctggc gtttttccat aggctccgcc cccctgacga gcatcacaaa aatcgacgct 3240
caagtcagag gtggcgaaac ccgacaggac tataaagata ccaggcgttt ccccctggaa 3300
gctccctcgt gcgctctcct gttccgaccc tgccgcttac cggatacctg tccgcctttc 3360
tcccttcggg aagcgtggcg ctttctcata gctcacgctg taggtatctc agttcggtgt 3420
aggtcgttcg ctccaagctg ggctgtgtgc acgaaccccc cgttcagccc gaccgctgcg 3480
ccttatccgg taactatcgt cttgagtcca acccggtaag acacgactta tcgccactgg 3540
cagcagccac tggtaacagg attagcagag cgaggtatgt aggcggtgct acagagttct 3600
tgaagtggtg gcctaactac ggctacacta gaaggacagt atttggtatc tgcgctctgc 3660
tgaagccagt taccttcgga aaaagagttg gtagctcttg atccggcaaa caaaccaccg 3720
ctggtagcgg tggttttttt gtttgcaagc agcagattac gcgcagaaaa aaaggatctc 3780
aagaagatcc tttgatcttt tctacggggt ctgacgctca gtggaacgaa aactcacgtt 3840
aagggatttt ggtcatgaga ttatcaaaaa ggatcttcac ctagatcctt ttaaaggccg 3900
gccgcggccg ccatcggcat tttcttttgc gtttttattt gttaactgtt aattgtcctt 3960
gttcaaggat gctgtctttg acaacagatg ttttcttgcc tttgatgttc agcaggaagc 4020
tcggcgcaaa cgttgattgt ttgtctgcgt agaatcctct gtttgtcata tagcttgtaa 4080
tcacgacatt gtttcctttc gcttgaggta cagcgaagtg tgagtaagta aaggttacat 4140
cgttaggatc aagatccatt tttaacacaa ggccagtttt gttcagcggc ttgtatgggc 4200
cagttaaaga attagaaaca taaccaagca tgtaaatatc gttagacgta atgccgtcaa 4260
tcgtcatttt tgatccgcgg gagtcagtga acaggtacca tttgccgttc attttaaaga 4320
cgttcgcgcg ttcaatttca tctgttactg tgttagatgc aatcagcggt ttcatcactt 4380
ttttcagtgt gtaatcatcg tttagctcaa tcataccgag agcgccgttt gctaactcag 4440
ccgtgcgttt tttatcgctt tgcagaagtt tttgactttc ttgacggaag aatgatgtgc 4500
ttttgccata gtatgctttg ttaaataaag attcttcgcc ttggtagcca tcttcagttc 4560
cagtgtttgc ttcaaatact aagtatttgt ggcctttatc ttctacgtag tgaggatctc 4620
tcagcgtatg gttgtcgcct gagctgtagt tgccttcatc gatgaactgc tgtacatttt 4680
gatacgtttt tccgtcaccg tcaaagattg atttataatc ctctacaccg ttgatgttca 4740
aagagctgtc tgatgctgat acgttaactt gtgcagttgt cagtgtttgt ttgccgtaat 4800
gtttaccgga gaaatcagtg tagaataaac ggatttttcc gtcagatgta aatgtggctg 4860
aacctgacca ttcttgtgtt tggtctttta ggatagaatc atttgcatcg aatttgtcgc 4920
tgtctttaaa gacgcggcca gcgtttttcc agctgtcaat agaagtttcg ccgacttttt 4980
gatagaacat gtaaatcgat gtgtcatccg catttttagg atctccggct aatgcaaaga 5040

cgatgtggta gccgtgatag tttgcgacag tgccgtcagc gttttgtaat ggccagctgt 5100

cccaaacgtc caggcctttt gcagaagaga tatttttaat tgtggacgaa tcaaattcag 5160

aaacttgata tttttcattt ttttgctgtt cagggatttg cagcatatca tggcgtgtaa 5220

tatgggaaat gccgtatgtt tccttatatg gcttttggtt cgtttctttc gcaaacgctt 5280

gagttgcgcc tcctgccagc agtgcggtag taaaggttaa tactgttgct tgttttgcaa 5340

actttttgat gttcatcgtt catgtctcct tttttatgta ctgtgttagc ggtctgcttc 5400

ttccagccct cctgtttgaa gatggcaagt tagttacgca caataaaaaa agacctaaaa 5460

tatgtaaggg gtgacgccaa agtatacact ttgcccttta cacattttag gtcttgcctg 5520

ctttatcagt aacaaacccg cgcgatttac ttttcgacct cattctatta gactctcgtt 5580

tggattgcaa ctggtctatt ttcctctttt gtttgataga aaatcataaa aggatttgca 5640

gactacgggc ctaaagaact aaaaaatcta tctgtttctt ttcattctct gtatttttta 5700

tagtttctgt tgcatgggca taaagttgcc tttttaatca caattcagaa aatatcataa 5760

tatctcattt cactaaataa tagtgaacgg caggtatatg tgatgggtta aaaaggatcg 5820

gcggccgctc gatttaaatc tcgagaggcc tgacgtcggg 5860

<210> 13
<211> 23
<212> DNA
<213> Corynebacterium glutamicum

<220>
<223> SOD8

<400> 13
accctggcgg aaaccctgag tcg 23

<210> 14
<211> 65
<212> DNA
<213> Corynebacterium glutamicum

<220>
<223> SOD1

<400> 14
tacgaccagg gccacgggta aaaaatcctt tcgtaggttt ccgcaccgag catatacatc 60

ttttg 65

<210> 15
<211> 40
<212> DNA
<213> Corynebacterium glutamicum

<220>
<223> LYSC2

<400> 15
cctacgaaag gattttttac ccgtggccct ggtcgtacag 40

<210> 16
<211> 21
<212> DNA
<213> Corynebacterium glutamicum

<220>
<223> LYSC6

<400> 16
gattagtgga acctcgtcgt c 21

<210> 17
<211> 19
<212> DNA
<213> Corynebacterium glutamicum

<220>
<223> SOD4

<400> 17
gcggcgcagg attttctaa                                                    19


<210> 18
<211> 22
<212> DNA
<213> Corynebacterium glutamicum

<220>
<223> LYSC4

<400> 18
tcggttgcct gagtaatgtc tt                                                22


<210> 19
<211> 5720
<212> DNA
<213> Corynebacterium glutamicum

<220>
<223> PK19

<220>
<221>  misc_feature
<222>  (2706 )..(3968 )
<223>  lysC  complement

<220>
<221>  misc_feature
<222>  (3969 )..(4166 )
<223>  Psod  complement

<220>
<221>  misc_feature
<222>  (4538 )..(5236 )
<223>  5' lysC complement

<220>
<221>  misc_feature
<222>  (5626 )..(6420 )
<223>  KanR

<220>
<221>  misc_feature
<222>  (6449)..(6911 )
<223>  PsacB

<220>
<221>  misc_feature
<222>  (6912 )..(6)
<223>  sacB

<400> 19
ggtcgactct agaggatccc cgggtaccga gctcgaattc actggccgtc gttttacaac 60

gtcgtgactg ggaaaaccct ggcgttaccc aacttaatcg ccttgcagca catccccctt 120

tcgccagctg gcgtaatagc gaagaggccc gcaccgatcg cccttcccaa cagttgcgca 180

gcctgaatgg cgaatggcga taagctagct tcacgctgcc gcaagcactc agggcgcaag 240

ggctgctaaa ggaagcggaa cacgtagaaa gccagtccgc agaaacggtg ctgaccccgg 300

atgaatgtca gctactgggc tatctggaca agggaaaacg caagcgcaaa gagaaagcag 360

gtagcttgca gtgggcttac atggcgatag ctagactggg cggttttatg gacagcaagc 420
gaaccggaat tgccagctgg ggcgccctct ggtaaggttg ggaagccctg caaagtaaac 480
tggatggctt tcttgccgcc aaggatctga tggcgcaggg gatcaagatc tgatcaagag 540
acaggatgag gatcgtttcg catgattgaa caagatggat tgcacgcagg ttctccggcc 600
gcttgggtgg agaggctatt cggctatgac tgggcacaac agacaatcgg ctgctctgat 660
gccgccgtgt tccggctgtc agcgcagggg cgcccggttc tttttgtcaa gaccgacctg 720
tccggtgccc tgaatgaact ccaagacgag gcagcgcggc tatcgtggct ggccacgacg 780
ggcgttcctt gcgcagctgt gctcgacgtt gtcactgaag cgggaaggga ctggctgcta 840
ttgggcgaag tgccggggca ggatctcctg tcatctcacc ttgctcctgc cgagaaagta 900
tccatcatgg ctgatgcaat gcggcggctg catacgcttg atccggctac ctgcccattc 960
gaccaccaag cgaaacatcg catcgagcga gcacgtactc ggatggaagc cggtcttgtc 1020
gatcaggatg atctggacga agagcatcag gggctcgcgc cagccgaact gttcgccagg 1080
ctcaaggcgc ggatgcccga cggcgaggat ctcgtcgtga cccatggcga tgcctgcttg 1140
ccgaatatca tggtggaaaa tggccgcttt tctggattca tcgactgtgg ccggctgggt 1200
gtggcggacc gctatcagga catagcgttg gctacccgtg atattgctga agagcttggc 1260
ggcgaatggg ctgaccgctt cctcgtgctt tacggtatcg ccgctcccga ttcgcagcgc 1320
atcgccttct atcgccttct tgacgagttc ttctgagcgg gactctgggg ttcgctagag 1380
gatcgatcct ttttaaccca tcacatatac ctgccgttca ctattattta gtgaaatgag 1440
atattatgat attttctgaa ttgtgattaa aaaggcaact ttatgcccat gcaacagaaa 1500
ctataaaaaa tacagagaat gaaaagaaac agatagattt tttagttctt taggcccgta 1560
gtctgcaaat ccttttatga ttttctatca aacaaaagag gaaaatagac cagttgcaat 1620
ccaaacgaga gtctaataga atgaggtcga aaagtaaatc gcgcgggttt gttactgata 1680
aagcaggcaa gacctaaaat gtgtaaaggg caaagtgtat actttggcgt caccccttac 1740
atattttagg tcttttttta ttgtgcgtaa ctaacttgcc atcttcaaac aggagggctg 1800
gaagaagcag accgctaaca cagtacataa aaaaggagac atgaacgatg aacatcaaaa 1860
agtttgcaaa acaagcaaca gtattaacct ttactaccgc actgctggca ggaggcgcaa 1920
ctcaagcgtt tgcgaaagaa acgaaccaaa agccatataa ggaaacatac ggcatttccc 1980
atattacacg ccatgatatg ctgcaaatcc ctgaacagca aaaaaatgaa aaatatcaag 2040
tttctgaatt tgattcgtcc acaattaaaa atatctcttc tgcaaaaggc ctggacgttt 2100
gggacagctg gccattacaa aacgctgacg gcactgtcgc aaactatcac ggctaccaca 2160
tcgtctttgc attagccgga gatcctaaaa atgcggatga cacatcgatt tacatgttct 2220
atcaaaaagt cggcgaaact tctattgaca gctggaaaaa cgctggccgc gtctttaaag 2280
acagcgacaa attcgatgca aatgattcta tcctaaaaga ccaaacacaa gaatggtcag 2340
gttcagccac atttacatct gacggaaaaa tccgtttatt ctacactgat ttctccggta 2400
aacattacgg caaacaaaca ctgacaactg cacaagttaa cgtatcagca tcagacagct 2460
ctttgaacat caacggtgta gaggattata aatcaatctt tgacggtgac ggaaaaacgt 2520
atcaaaatgt acagcagttc atcgatgaag gcaactacag ctcaggcgac aaccatacgc 2580
tgagagatcc tcactacgta gaagataaag gccacaaata cttagtattt gaagcaaaca 2640
ctggaactga agatggctac caaggcgaag aatctttatt taacaaagca tactatggca 2700

aaagcacatc attcttccgt caagaaagtc aaaaacttct gcaaagcgat aaaaaacgca 2760
cggctgagtt agcaaacggc gctctcggta tgattgagct aaacgatgat tacacactga 2820
aaaaagtgat gaaaccgctg attgcatcta acacagtaac agatgaaatt gaacgcgcga 2880
acgtctttaa aatgaacggc aaatggtacc tgttcactga ctcccgcgga tcaaaaatga 2940
cgattgacgg cattacgtct aacgatattt acatgcttgg ttatgtttct aattctttaa 3000
ctggcccata caagccgctg aacaaaactg gccttgtgtt aaaaatggat cttgatccta 3060
acgatgtaac ctttacttac tcacacttcg ctgtacctca agcgaaagga aacaatgtcg 3120
tgattacaag ctatatgaca aacagaggat tctacgcaga caaacaatca acgtttgcgc 3180
cgagcttcct gctgaacatc aaaggcaaga aaacatctgt tgtcaaagac agcatccttg 3240
aacaaggaca attaacagtt aacaaataaa aacgcaaaag aaaatgccga tgggtaccga 3300
gcgaaatgac cgaccaagcg acgcccaacc tgccatcacg agatttcgat tccaccgccg 3360
ccttctatga aaggttgggc ttcggaatcg ttttccggga cgccctcgcg gacgtgctca 3420
tagtccacga cgcccgtgat tttgtagccc tggccgacgg ccagcaggta ggccgacagg 3480
ctcatgccgg ccgccgccgc cttttcctca atcgctcttc gttcgtctgg aaggcagtac 3540
accttgatag gtgggctgcc cttcctggtt ggcttggttt catcagccat ccgcttgccc 3600
tcatctgtta cgccggcggt agccggccag cctcgcagag caggattccc gttgagcacc 3660
gccaggtgcg aataagggac agtgaagaag gaacacccgc tcgcgggtgg gcctacttca 3720
cctatcctgc ccggctgacg ccgttggata caccaaggaa agtctacacg aaccctttgg 3780
caaaatcctg tatatcgtgc gaaaaaggat ggatataccg aaaaaatcgc tataatgacc 3840
ccgaagcagg gttatgcagc ggaaaagcgc tgcttccctg ctgttttgtg gaatatctac 3900
cgactggaaa caggcaaatg caggaaatta ctgaactgag gggacaggcg agagacgatg 3960
ccaaagagct cctgaaaatc tcgataactc aaaaaatacg cccggtagtg atcttatttc 4020
attatggtga aagttggaac ctcttacgtg ccgatcaacg tctcattttc gccaaaagtt 4080
ggcccagggc ttcccggtat caacagggac accaggattt atttattctg cgaagtgatc 4140
ttccgtcaca ggtatttatt cggcgcaaag tgcgtcgggt gatgctgcca acttactgat 4200
ttagtgtatg atggtgtttt tgaggtgctc cagtggcttc tgtttctatc agctcctgaa 4260
aatctcgata actcaaaaaa tacgcccggt agtgatctta tttcattatg gtgaaagttg 4320
gaacctctta cgtgccgatc aacgtctcat tttcgccaaa agttggccca gggcttcccg 4380
gtatcaacag ggacaccagg atttatttat tctgcgaagt gatcttccgt cacaggtatt 4440
tattcggcgc aaagtgcgtc gggtgatgct gccaacttac tgatttagtg tatgatggtg 4500
tttttgaggt gctccagtgg cttctgtttc tatcagggct ggatgatcct ccagcgcggg 4560
gatctcatgc tggagttctt cgcccacccc aaaaggatct aggtgaagat cctttttgat 4620
aatctcatga ccaaaatccc ttaacgtgag ttttcgttcc actgagcgtc agaccccgta 4680
gaaaagatca aaggatcttc ttgagatcct ttttttctgc gcgtaatctg ctgcttgcaa 4740
acaaaaaaac caccgctacc agcggtggtt tgtttgccgg atcaagagct accaactctt 4800
tttccgaagg taactggctt cagcagagcg cagataccaa atactgttct tctagtgtag 4860
ccgtagttag gccaccactt caagaactct gtagcaccgc ctacatacct cgctctgcta 4920
atcctgttac cagtggctgc tgccagtggc gataagtcgt gtcttaccgg gttggactca 4980

agacgatagt taccggataa ggcgcagcgg tcgggctgaa cggggggttc gtgcacacag 5040

cccagcttgg agcgaacgac ctacaccgaa ctgagatacc tacagcgtga gctatgagaa 5100

agcgccacgc ttcccgaagg gagaaaggcg gacaggtatc cggtaagcgg cagggtcgga 5160

acaggagagc gcacgaggga gcttccaggg ggaaacgcct ggtatcttta tagtcctgtc 5220

gggtttcgcc acctctgact tgagcgtcga tttttgtgat gctcgtcagg ggggcggagc 5280

ctatggaaaa acgccagcaa cgcggccttt ttacggttcc tggccttttg ctggcctttt 5340

gctcacatgt tctttcctgc gttatcccct gattctgtgg ataaccgtat taccgccttt 5400

gagtgagctg ataccgctcg ccgcagccga acgaccgagc gcagcgagtc agtgagcgag 5460

gaagcggaag agcgcccaat acgcaaaccg cctctccccg cgcgttggcc gattcattaa 5520

tgcagctggc acgacaggtt tcccgactgg aaagcgggca gtgagcgcaa cgcaattaat 5580

gtgagttagc tcactcatta ggcaccccag gctttacact ttatgcttcc ggctcgtatg 5640

ttgtgtggaa ttgtgagcgg ataacaattt cacacaggaa acagctatga ccatgattac 5700

gccaagcttg catgcctgca 5720

<210> 20
<211> 27
<212> DNA
<213> Corynebacterium glutamicum

<220>
<223> LYSC23

<400> 20
caccgcggct ttggacatca ctgctac 27

<210> 21
<211> 26
<212> DNA
<213> Corynebacterium glutamicum

<220>
<223> LYSC24

<400> 21
cctggggctt tagcggatgc gtctca 26

<210> 22
<211> 8324
<212> DNA
<213> Corynebacterium glutamicum

<220>
<223> PK19

<220>
<221> misc_feature
<222> (2706 )..(3968 )
<223> lysC complement

<220>
<221> misc_feature
<222> (3969 )..(4166 )
<223> Psod complement

<220>
<221> misc_feature
<222> (4538 )..(5236 )
<223> 5' lysC complement

<220>
<221> misc_feature
<222> (5626 )..(6420 )

<223>  KanR

<220>
<221>  misc_feature
<222>  (6449)..(6911 )
<223>  PsacB

<220>
<221>  misc_feature
<222>  (6912 )..(6)
<223>  sacB

<400> 22
aaacgcaaaa gaaaatgccg atgggtaccg agcgaaatga ccgaccaagc gacgcccaac 60

ctgccatcac gagatttcga ttccaccgcc gccttctatg aaaggttggg cttcggaatc 120

gttttccggg acgccctcgc ggacgtgctc atagtccacg acgcccgtga ttttgtagcc 180

ctggccgacg gccagcaggt aggccgacag gctcatgccg gccgccgccg ccttttcctc 240

aatcgctctt cgttcgtctg gaaggcagta caccttgata ggtgggctgc ccttcctggt 300

tggcttggtt tcatcagcca tccgcttgcc ctcatctgtt acgccggcgg tagccggcca 360

gcctcgcaga gcaggattcc cgttgagcac cgccaggtgc gaataaggga cagtgaagaa 420

ggaacacccg ctcgcgggtg ggcctacttc acctatcctg cccggctgac gccgttggat 480

acaccaagga aagtctacac gaaccctttg gcaaaatcct gtatatcgtg cgaaaaagga 540

tggatatacc gaaaaaatcg ctataatgac cccgaagcag ggttatgcag cggaaaagcg 600

ctgcttccct gctgttttgt ggaatatcta ccgactggaa acaggcaaat gcaggaaatt 660

actgaactga ggggacaggc gagagacgat gccaaagagc tcctgaaaat ctcgataact 720

caaaaaatac gcccggtagt gatcttattt cattatggtg aaagttggaa cctcttacgt 780

gccgatcaac gtctcatttt cgccaaaagt tggcccaggg cttcccggta tcaacaggga 840

caccaggatt tatttattct gcgaagtgat cttccgtcac aggtatttat tcggcgcaaa 900

gtgcgtcggg tgatgctgcc aacttactga tttagtgtat gatggtgttt ttgaggtgct 960

ccagtggctt ctgtttctat cagctcctga aaatctcgat aactcaaaaa atacgcccgg 1020

tagtgatctt atttcattat ggtgaaagtt ggaacctctt acgtgccgat caacgtctca 1080

ttttcgccaa aagttggccc agggcttccc ggtatcaaca gggacaccag gatttattta 1140

ttctgcgaag tgatcttccg tcacaggtat ttattcggcg caaagtgcgt cgggtgatgc 1200

tgccaactta ctgatttagt gtatgatggt gtttttgagg tgctccagtg gcttctgttt 1260

ctatcagggc tggatgatcc tccagcgcgg ggatctcatg ctggagttct tcgcccaccc 1320

caaaaggatc taggtgaaga tcctttttga taatctcatg accaaaatcc cttaacgtga 1380

gttttcgttc cactgagcgt cagaccccgt agaaaagatc aaaggatctt cttgagatcc 1440

tttttttctg cgcgtaatct gctgcttgca aacaaaaaaa ccaccgctac cagcggtggt 1500

ttgtttgccg gatcaagagc taccaactct ttttccgaag gtaactggct tcagcagagc 1560

gcagatacca aatactgttc ttctagtgta gccgtagtta ggccaccact tcaagaactc 1620

tgtagcaccg cctacatacc tcgctctgct aatcctgtta ccagtggctg ctgccagtgg 1680

cgataagtcg tgtcttaccg ggttggactc aagacgatag ttaccggata aggcgcagcg 1740

gtcgggctga acggggggtt cgtgcacaca gcccagcttg gagcgaacga cctacaccga 1800

actgagatac ctacagcgtg agctatgaga aagcgccacg cttcccgaag ggagaaaggc 1860

ggacaggtat ccggtaagcg gcagggtcgg aacaggagag cgcacgaggg agcttccagg 1920

gggaaacgcc tggtatcttt atagtcctgt cgggtttcgc cacctctgac ttgagcgtcg 1980
atttttgtga tgctcgtcag gggggcggag cctatggaaa aacgccagca acgcggcctt 2040
tttacggttc ctggcctttt gctggccttt tgctcacatg ttctttcctg cgttatcccc 2100
tgattctgtg gataaccgta ttaccgcctt tgagtgagct gataccgctc gccgcagccg 2160
aacgaccgag cgcagcgagt cagtgagcga ggaagcggaa gagcgcccaa tacgcaaacc 2220
gcctctcccc gcgcgttggc cgattcatta atgcagctgg cacgacaggt ttcccgactg 2280
gaaagcgggc agtgagcgca acgcaattaa tgtgagttag ctcactcatt aggcacccca 2340
ggctttacac tttatgcttc cggctcgtat gttgtgtgga attgtgagcg gataacaatt 2400
tcacacagga aacagctatg accatgatta cgccaagctt gcatgcctgc aggtcgactc 2460
tagaggatcc ccgggtaccg agctcgaatt cgccctttcg gttgcctgag taatgtcttc 2520
tacctcgatt tccgtgccac ggaattcaat cttacggcct gcggaacgtg gggaagcaaa 2580
gaaacgaaca gtgtcagctg ggaaattgcg ctcttccaaa agggtgcgca taacctggcc 2640
gacctggccg gttgcaccaa caactgcgat ggtggtcatt gtaaaactac tcctttaaaa 2700
ctttagcgtc cggtgcctgc ataaacgacg gcttcgtctt cgccgcccag ctggaactgc 2760
tcatgcaatg cacgtgcagc agcatccaga tcatcttcac ggatcagcac ggaaatacga 2820
atctcagagg tggaaatcaa ttcgatgttc acgttgacat cgcgcagagc ttccatgaac 2880
tctgcggtaa cacctgggtg agacttcatg ccagcaccca cgagggagac tttgccgacc 2940
tggtcgtcgt aaagcacatt ggtccagttg ccctgaacct gaagcttctt caagatctcc 3000
atcgcgcggc ggccgtcgga acgagggcag gtgaagatga tgtcggtggt gccgtcttct 3060
acagaagaga cgttctgcag aaccatgtca atgttgattt ctgcatcagc caacgcacgg 3120
aaaaccttcg cagcctcgcc tggcttatcg gaaataccca gaacggttac tttggcttcg 3180
gacttgtcgg ttgcgacacc ggtaaggact gcttcttcca caggaatatc ctccatagag 3240
ccggcaatca aagtgccggg atcattacta taagacgagc gtacgcgaag tggcacattg 3300
aatgcacgag cgtattcaac actgcgcagc accaaaatct tggagccaac agcagcaagt 3360
tccagcattt cttcgaagct gagcttttcc agcttctgtg cattaggaac gatgcgcggg 3420
tcagcggtat acacaccgtc aacgtccgag taaatctcac acacatcagc gttcaaagca 3480
gctgccaacg caactgcagt ggtgtcagaa ccaccacgac ccaacgtggt gacatcgcgg 3540
gtttctttat taacaccctg gaaaccagca acaatgcaga tcttgccctc atcgagtgct 3600
tcacgcacac gacctggagt gacatcaaca atgcgtgcgt ttccgtggcg ctcggtggtg 3660
agcacaccag cctgagagcc cgtgaaagat tgggcttctg cgccaaggga ctcaatagcc 3720
atggcgacga gagcgttaga aatacgctca ccagcagtca ggagcatatc catttcacga 3780
gctggcggaa cgggattcac tgccgctgca agttctagaa gttcatccgt ggtgtctccc 3840
attgcggagc agacaaccac gacatcattt ccagccttct tggtggcaac gatccgttca 3900
gcgacgtttc taatgcgttc cgcactctca agcgaggaac cgccatattt ctgtacgacc 3960
agggccacgg gtaaaaaatc ctttcgtagg tttccgcacc gagcatatac atcttttgaa 4020
aatccgtcag atggcgcttc gcaaaagtac ttggtgcgac acctcccaat gataggcctt 4080
tttgttgata ttgcaacgaa atttttcagc cgacctattt atcgggtagc gggtcacaag 4140
cccggaataa ttggcagcta agtagggttg aagggcataa ggcttcctca attttcgaaa 4200
ggaacattcc tgttatggca tggtttttcg cacccgaacc cgtgatggtt accgctgatg 4260

aggcgcttaa aggtggcagg catcctgttt tagaaaatcc tgcgccgcaa gggcgaattc 4320
actggccgtc gttttacaac gtcgtgactg ggaaaaccct ggcgttaccc aacttaatcg 4380
ccttgcagca catccccctt tcgccagctg gcgtaatagc gaagaggccc gcaccgatcg 4440
cccttcccaa cagttgcgca gcctgaatgg cgaatggcga taagctagat gcatgctcga 4500
gcggccgcca gtgtgatgga tatctgcaga attcgccctt ggggctttag cggatgcgtc 4560
tcagccactg ggacgcgttc caataaacag accatatatt gatattcgat ttaatatttg 4620
agacaaaagt gacaggtgct acttcgcgag caactcttta gtcaactacc ctgaatcaag 4680
tgcaaagcaa tctgctcgcc gtgcttttcg ccctagcatc ggcattaaca atcgcatggg 4740
gcaccgtggt cagacaccgg atcgcgctcc gcaccccaaa agatggctcc ctaaggagct 4800
cacctttact caatgctctg atgacaccga tgtggtgggc aggcatgagt accgcgatgc 4860
tggcatattt cttacaaaca gtagcacttg gtttcggcac cctcttggta gtgcaaccag 4920
tgcttgtcct gtcgctgatg ttcacgctgc cgctctcagc acgattcaat ggctaccgac 4980
tacgccgaac tgaaatcttc tgggctaccc tcctcaccgt agccgtgggc atcatgatcg 5040
ttttgggacg cccccttccc ggaaaccccc acccccactc gatcgatgga ttccagtact 5100
tttagtcggc gttgcagtaa tgggtggaat gtggctgctt gcggaatacg tattaaagaa 5160
ggacaaagcc ctcatccttg gtcttgtgac gggtgcattg tttggctacg tagcagtgat 5220
gtccaaagcc gcggtgaagg gcgaattcca gcacactggc ggccgttact agcttcacgc 5280
tgccgcaagc actcagggcg caagggctgc taaaggaagc ggaacacgta gaaagccagt 5340
ccgcagaaac ggtgctgacc ccggatgaat gtcagctact gggctatctg gacaagggaa 5400
aacgcaagcg caaagagaaa gcaggtagct tgcagtgggc ttacatggcg atagctagac 5460
tgggcggttt tatggacagc aagcgaaccg gaattgccag ctggggcgcc ctctggtaag 5520
gttgggaagc cctgcaaagt aaactggatg gctttcttgc cgccaaggat ctgatggcgc 5580
aggggatcaa gatctgatca agagacagga tgaggatcgt ttcgcatgat tgaacaagat 5640
ggattgcacg caggttctcc ggccgcttgg gtggagaggc tattcggcta tgactgggca 5700
caacagacaa tcggctgctc tgatgccgcc gtgttccggc tgtcagcgca ggggcgcccg 5760
gttctttttg tcaagaccga cctgtccggt gccctgaatg aactccaaga cgaggcagcg 5820
cggctatcgt ggctggccac gacgggcgtt ccttgcgcag ctgtgctcga cgttgtcact 5880
gaagcgggaa gggactggct gctattgggc gaagtgccgg ggcaggatct cctgtcatct 5940
caccttgctc ctgccgagaa agtatccatc atggctgatg caatgcggcg gctgcatacg 6000
cttgatccgg ctacctgccc attcgaccac caagcgaaac atcgcatcga gcgagcacgt 6060
actcggatgg aagccggtct tgtcgatcag gatgatctgg acgaagagca tcaggggctc 6120
gcgccagccg aactgttcgc caggctcaag gcgcggatgc ccgacggcga ggatctcgtc 6180
gtgacccatg gcgatgcctg cttgccgaat atcatggtgg aaaatggccg cttttctgga 6240
ttcatcgact gtggccggct gggtgtggcg gaccgctatc aggacatagc gttggctacc 6300
cgtgatattg ctgaagagct tggcggcgaa tgggctgacc gcttcctcgt gctttacggt 6360
atcgccgctc ccgattcgca gcgcatcgcc ttctatcgcc ttcttgacga gttcttctga 6420
gcgggactct ggggttcgct agaggatcga tcctttttaa cccatcacat atacctgccg 6480
ttcactatta tttagtgaaa tgagatatta tgatattttc tgaattgtga ttaaaaaggc 6540

aactttatgc ccatgcaaca gaaactataa aaaatacaga gaatgaaaag aaacagatag 6600

attttttagt tctttaggcc cgtagtctgc aaatcctttt atgattttct atcaaacaaa 6660

agaggaaaat agaccagttg caatccaaac gagagtctaa tagaatgagg tcgaaaagta 6720

aatcgcgcgg gtttgttact gataaagcag gcaagaccta aaatgtgtaa agggcaaagt 6780

gtatactttg gcgtcacccc ttacatattt taggtctttt tttattgtgc gtaactaact 6840

tgccatcttc aaacaggagg gctggaagaa gcagaccgct aacacagtac ataaaaaagg 6900

agacatgaac gatgaacatc aaaaagtttg caaaacaagc aacagtatta acctttacta 6960

ccgcactgct ggcaggaggc gcaactcaag cgtttgcgaa agaaacgaac caaaagccat 7020

ataaggaaac atacggcatt tcccatatta cacgccatga tatgctgcaa atccctgaac 7080

agcaaaaaaa tgaaaaatat caagttcctg aatttgattc gtccacaatt aaaaatatct 7140

cttctgcaaa aggcctggac gtttgggaca gctggccatt acaaaacgct gacggcactg 7200

tcgcaaacta tcacggctac cacatcgtct ttgcattagc cggagatcct aaaaatgcgg 7260

atgacacatc gatttacatg ttctatcaaa aagtcggcga aacttctatt gacagctgga 7320

aaaacgctgg ccgcgtcttt aaagacagcg acaaattcga tgcaaatgat tctatcctaa 7380

aagaccaaac acaagaatgg tcaggttcag ccacatttac atctgacgga aaaatccgtt 7440

tattctacac tgatttctcc ggtaaacatt acggcaaaca aacactgaca actgcacaag 7500

ttaacgtatc agcatcagac agctctttga acatcaacgg tgtagaggat tataaatcaa 7560

tctttgacgg tgacggaaaa acgtatcaaa atgtacagca gttcatcgat gaaggcaact 7620

acagctcagg cgacaaccat acgctgagag atcctcacta cgtagaagat aaaggccaca 7680

aatacttagt atttgaagca aacactggaa ctgaagatgg ctaccaaggc gaagaatctt 7740

tatttaacaa agcatactat ggcaaaagca catcattctt ccgtcaagaa agtcaaaaac 7800

ttctgcaaag cgataaaaaa cgcacggctg agttagcaaa cggcgctctc ggtatgattg 7860

agctaaacga tgattacaca ctgaaaaaag tgatgaaacc gctgattgca tctaacacag 7920

taacagatga aattgaacgc gcgaacgtct ttaaaatgaa cggcaaatgg tacctgttca 7980

ctgactcccg cggatcaaaa atgacgattg acggcattac gtctaacgat atttacatgc 8040

ttggttatgt ttctaattct ttaactggcc catacaagcc gctgaacaaa actggccttg 8100

tgttaaaaat ggatcttgat cctaacgatg taacctttac ttactcacac ttcgctgtac 8160

ctcaagcgaa aggaaacaat gtcgtgatta caagctatat gacaaacaga ggattctacg 8220

cagacaaaca atcaacgttt gcgccgagct tcctgctgaa catcaaaggc aagaaaacat 8280

ctgttgtcaa agacagcatc cttgaacaag gacaattaac agtt 8324

<210> 23
<211> 52
<212> DNA
<213> Corynebacterium glutamicum

<220>
<223> SEQ_ID_23

<400> 23
cccgggatcc gctagcggcg cgccggccgg cccggtgtga aataccgcac ag 52

<210> 24
<211> 53
<212> DNA
<213> Corynebacterium glutamicum

<220>
<223> SEQ_ID_24

<400> 24
tctagactcg agcggccgcg gccggccttt aaattgaaga cgaaagggcc tcg          53


<210> 25
<211> 47
<212> DNA
<213> Corynebacterium glutamicum

<220>
<223> SEQ_ID_25

<400> 25
gagatctaga cccggggatc cgctagcggg ctgctaaagg aagcgga                 47


<210> 26
<211> 38
<212> DNA
<213> Corynebacterium glutamicum

<220>
<223> SEQ_ID_26

<400> 26
gagaggcgcg ccgctagcgt gggcgaagaa ctccagca                           38


<210> 27
<211> 34
<212> DNA
<213> Corynebacterium glutamicum

<220>
<223> SEQ_ID_27

<400> 27
gagagggcgg ccgcgcaaag tcccgcttcg tgaa                               34


<210> 28
<211> 34
<212> DNA
<213> Corynebacterium glutamicum

<220>
<223> SEQ_ID_28

<400> 28
gagagggcgg ccgctcaagt cggtcaagcc acgc                               34


<210> 29
<211> 140
<212> DNA
<213> Corynebacterium glutamicum

<220>
<223> SEQ_ID_29

<400> 29
tcgaatttaa atctcgagag gcctgacgtc gggcccggta ccacgcgtca tatgactagt 60

tcggacctag ggatatcgtc gacatcgatg ctcttctgcg ttaattaaca attgggatcc 120

tctagacccg ggatttaaat                                               140


<210> 30
<211> 140
<212> DNA
<213> Corynebacterium glutamicum

<220>
<223> SEQ_ID_30

<400> 30
gatcatttaa atcccgggtc tagaggatcc caattgttaa ttaacgcaga agagcatcga 60

tgtcgacgat atccctaggt ccgaactagt catatgacgc gtggtaccgg gcccgacgtc 120

aggcctctcg agatttaaat 140


<210> 31
<211> 5091
<212> DNA
<213> Corynebacterium glutamicum

<220>
<223> PCLIK5MCS

<220>
<221> misc_feature
<222> (469 )..(1260 )
<223> KanR

<220>
<221> misc_feature
<222> (1527 )..(2387 )
<223> Ori EC pMB (complement)

<220>
<221> misc_feature
<222> (2533 )..(3207 )
<223> Orf1

<220>
<221> misc_feature
<222> (3541 )..(4662 )
<223> Rep Protein

<400> 31
tcgatttaaa tctcgagagg cctgacgtcg ggcccggtac cacgcgtcat atgactagtt 60

cggacctagg gatatcgtcg acatcgatgc tcttctgcgt taattaacaa ttgggatcct 120

ctagacccgg gatttaaatc gctagcgggc tgctaaagga agcggaacac gtagaaagcc 180

agtccgcaga aacggtgctg accccggatg aatgtcagct actgggctat ctggacaagg 240

gaaaacgcaa gcgcaaagag aaagcaggta gcttgcagtg ggcttacatg gcgatagcta 300

gactgggcgg ttttatggac agcaagcgaa ccggaattgc cagctggggc gccctctggt 360

aaggttggga agccctgcaa agtaaactgg atggctttct tgccgccaag gatctgatgg 420

cgcaggggat caagatctga tcaagagaca ggatgaggat cgtttcgcat gattgaacaa 480

gatggattgc acgcaggttc tccggccgct tgggtggaga ggctattcgg ctatgactgg 540

gcacaacaga caatcggctg ctctgatgcc gccgtgttcc ggctgtcagc gcaggggcgc 600

ccggttcttt ttgtcaagac cgacctgtcc ggtgccctga atgaactgca ggacgaggca 660

gcgcggctat cgtggctggc cacgacgggc gttccttgcg cagctgtgct cgacgttgtc 720

actgaagcgg gaagggactg gctgctattg ggcgaagtgc cggggcagga tctcctgtca 780

tctcaccttg ctcctgccga gaaagtatcc atcatggctg atgcaatgcg gcggctgcat 840

acgcttgatc cggctacctg cccattcgac caccaagcga aacatcgcat cgagcgagca 900

cgtactcgga tggaagccgg tcttgtcgat caggatgatc tggacgaaga gcatcagggg 960

ctcgcgccag ccgaactgtt cgccaggctc aaggcgcgca tgcccgacgg cgaggatctc 1020

gtcgtgaccc atggcgatgc ctgcttgccg aatatcatgg tggaaaatgg ccgcttttct 1080

ggattcatcg actgtggccg gctgggtgtg gcggaccgct atcaggacat agcgttggct 1140

acccgtgata ttgctgaaga gcttggcggc gaatgggctg accgcttcct cgtgctttac 1200
ggtatcgccg ctcccgattc gcagcgcatc gccttctatc gccttcttga cgagttcttc 1260
tgagcgggac tctggggttc gaaatgaccg accaagcgac gcccaacctg ccatcacgag 1320
atttcgattc caccgccgcc ttctatgaaa ggttgggctt cggaatcgtt ttccgggacg 1380
ccggctggat gatcctccag cgcggggatc tcatgctgga gttcttcgcc cacgctagcg 1440
gcgcgccggc cggcccggtg tgaaataccg cacagatgcg taaggagaaa ataccgcatc 1500
aggcgctctt ccgcttcctc gctcactgac tcgctgcgct cggtcgttcg gctgcggcga 1560
gcggtatcag ctcactcaaa ggcggtaata cggttatcca cagaatcagg ggataacgca 1620
ggaaagaaca tgtgagcaaa aggccagcaa aaggccagga accgtaaaaa ggccgcgttg 1680
ctggcgtttt tccataggct ccgcccccct gacgagcatc acaaaaatcg acgctcaagt 1740
cagaggtggc gaaacccgac aggactataa agataccagg cgtttccccc tggaagctcc 1800
ctcgtgcgct ctcctgttcc gaccctgccg cttaccggat acctgtccgc ctttctccct 1860
tcgggaagcg tggcgctttc tcatagctca cgctgtaggt atctcagttc ggtgtaggtc 1920
gttcgctcca agctgggctg tgtgcacgaa ccccccgttc agcccgaccg ctgcgcctta 1980
tccggtaact atcgtcttga gtccaacccg gtaagacacg acttatcgcc actggcagca 2040
gccactggta acaggattag cagagcgagg tatgtaggcg gtgctacaga gttcttgaag 2100
tggtggccta actacggcta cactagaagg acagtatttg gtatctgcgc tctgctgaag 2160
ccagttacct tcggaaaaag agttggtagc tcttgatccg gcaaacaaac caccgctggt 2220
agcggtggtt tttttgtttg caagcagcag attacgcgca gaaaaaaagg atctcaagaa 2280
gatcctttga tcttttctac ggggtctgac gctcagtgga acgaaaactc acgttaaggg 2340
attttggtca tgagattatc aaaaaggatc ttcacctaga tccttttaaa ggccggccgc 2400
ggccgcgcaa agtcccgctt cgtgaaaatt ttcgtgccgc gtgattttcc gccaaaaact 2460
ttaacgaacg ttcgttataa tggtgtcatg accttcacga cgaagtacta aaattggccc 2520
gaatcatcag ctatggatct ctctgatgtc gcgctggagt ccgacgcgct cgatgctgcc 2580
gtcgatttaa aaacggtgat cggatttttc cgagctctcg atacgacgga cgcgccagca 2640
tcacgagact gggccagtgc cgcgagcgac ctagaaactc tcgtggcgga tcttgaggag 2700
ctggctgacg agctgcgtgc tcggccagcg ccaggaggac gcacagtagt ggaggatgca 2760
atcagttgcg cctactgcgg tggcctgatt cctccccggc ctgacccgcg aggacggcgc 2820
gcaaaatatt gctcagatgc gtgtcgtgcc gcagccagcc gcgagcgcgc caacaaacgc 2880
cacgccgagg agctggaggc ggctaggtcg caaatggcgc tggaagtgcg tcccccgagc 2940
gaaattttgg ccatggtcgt cacagagctg gaagcggcag cgagaattat cgcgatcgtg 3000
gcggtgcccg caggcatgac aaacatcgta aatgccgcgt ttcgtgtgcc gtggccgccc 3060
aggacgtgtc agcgccgcca ccacctgcac cgaatcggca gcagcgtcgc gcgtcgaaaa 3120
agcgcacagg cggcaagaag cgataagctg cacgaatacc tgaaaaatgt tgaacgcccc 3180
gtgagcggta actcacaggg cgtcggctaa cccccagtcc aaacctggga gaaagcgctc 3240
aaaaatgact ctagcggatt cacgagacat tgacacaccg gcctggaaat tttccgctga 3300
tctgttcgac acccatcccg agctcgcgct gcgatcacgt ggctggacga gcgaagaccg 3360
ccgcgaattc ctcgctcacc tgggcagaga aaatttccag ggcagcaaga cccgcgactt 3420

cgccagcgct tggatcaaag acccggacac ggagaaacac agccgaagtt ataccgagtt 3480
ggttcaaaat cgcttgcccg gtgccagtat gttgctctga cgcacgcgca gcacgcagcc 3540
gtgcttgtcc tggacattga tgtgccgagc caccaggccg gcgggaaaat cgagcacgta 3600
aaccccgagg tctacgcgat tttggagcgc tgggcacgcc tggaaaaagc gccagcttgg 3660
atcggcgtga atccactgag cgggaaatgc cagctcatct ggctcattga tccggtgtat 3720
gccgcagcag gcatgagcag cccgaatatg cgcctgctgg ctgcaacgac cgaggaaatg 3780
acccgcgttt tcggcgctga ccaggctttt tcacataggc tgagccgtgg ccactgcact 3840
ctccgacgat cccagccgta ccgctggcat gcccagcaca atcgcgtgga tcgcctagct 3900
gatcttatgg aggttgctcg catgatctca ggcacagaaa aacctaaaaa acgctatgag 3960
caggagtttt ctagcggacg ggcacgtatc gaagcggcaa gaaaagccac tgcggaagca 4020
aaagcacttg ccacgcttga agcaagcctg ccgagcgccg ctgaagcgtc tggagagctg 4080
atcgacggcg tccgtgtcct ctggactgct ccagggcgtg ccgcccgtga tgagacggct 4140
tttcgccacg ctttgactgt gggataccag ttaaaagcgg ctggtgagcg cctaaaagac 4200
accaagggtc atcgagccta cgagcgtgcc tacaccgtcg ctcaggcggt cggaggaggc 4260
cgtgagcctg atctgccgcc ggactgtgac cgccagacgg attggccgcg acgtgtgcgc 4320
ggctacgtcg ctaaaggcca gccagtcgtc cctgctcgtc agacagagac gcagagccag 4380
ccgaggcgaa aagctctggc cactatggga agacgtggcg gtaaaaaggc cgcagaacgc 4440
tggaaagacc caaacagtga gtacgcccga gcacagcgag aaaaactagc taagtccagt 4500
caacgacaag ctaggaaagc taaaggaaat cgcttgacca ttgcaggttg gtttatgact 4560
gttgagggag agactggctc gtggccgaca atcaatgaag ctatgtctga atttagcgtg 4620
tcacgtcaga ccgtgaatag agcacttaag gtctgcgggc attgaacttc cacgaggacg 4680
ccgaaagctt cccagtaaat gtgccatctc gtaggcagaa aacggttccc ccgtagggtc 4740
tctctcttgg cctcctttct aggtcgggct gattgctctt gaagctctct aggggggctc 4800
acaccatagg cagataacgt tccccaccgg ctcgcctcgt aagcgcacaa ggactgctcc 4860
caaagatctt caaagccact gccgcgactg ccttcgcgaa gccttgcccc gcggaaattt 4920
cctccaccga gttcgtgcac acccctatgc caagcttctt tcaccctaaa ttcgagagat 4980
tggattctta ccgtggaaat tcttcgcaaa aatcgtcccc tgatcgccct tgcgacgttg 5040
gcgtcggtgc cgctggttgc gcttggcttg accgacttga tcagcggccg c 5091

<210> 32
<211> 28
<212> DNA
<213> Corynebacterium glutamicum

<220>
<223> SEQ_ID_32

<400> 32
gcgcggtacc tagactcacc ccagtgct 28

<210> 33
<211> 30
<212> DNA
<213> Corynebacterium glutamicum

<220>
<223> SEQ_ID_33

<400> 33
ctctactagt ttagatgtag aactcgatgt                                        30


<210> 34
<211> 6349
<212> DNA
<213> Corynebacterium glutamicum

<220>
<223> PCLIK5MCS

<220>
<221>  misc_feature
<222>  (42 )..(177 )
<223>  PmetA

<220>
<221>  misc_feature
<222>  (178 )..(1311 )
<223>  metA

<220>
<221>  misc_feature
<222>  (1727 )..(2518 )
<223>  KanR

<220>
<221>  misc_feature
<222>  (2785 )..(3645 )
<223>  Orf1

<220>
<221>  misc_feature
<222>  (3791 )..(4465 )
<223>  Ori-EC   complement

<220>
<221>  misc_feature
<222>  (4799 )..(5920 )
<223>  Rep Protein

<400> 34
tcgatttaaa tctcgagagg cctgacgtcg ggcccggtac ctagactcac cccagtgctt 60

aaagcgctgg gtttttcttt ttcagactcg tgagaatgca aactagacta gacagagctg 120

tccatataca ctggacgaag ttttagtctt gtccacccag aacaggcggt tattttcatg 180

cccaccctcg cgccttcagg tcaacttgaa atccaagcga tcggtgatgt ctccaccgaa 240

gccggagcaa tcattacaaa cgctgaaatc gcctatcacc gctggggtga ataccgcgta 300

gataaagaag gacgcagcaa tgtcgttctc atcgaacacg ccctcactgg agattccaac 360

gcagccgatt ggtgggctga cttgctcggt cccggcaaag ccatcaacac tgatatttac 420

tgcgtgatct gtaccaacgt catcggtggt tgcaacggtt ccaccggacc tggctccatg 480

catccagatg gaaatttctg gggtaatcgc ttccccgcca cgtccattcg tgatcaggta 540

aacgccgaaa aacaattcct cgacgcactc ggcatcacca cggtcgccgc agtacttggt 600

ggttccatgg gtggtgcccg caccctagag tgggccgcaa tgtacccaga aactgttggc 660

gcagctgctg ttcttgcagt ttctgcacgc gccagcgcct ggcaaatcgg cattcaatcc 720

gcccaaatta aggcgattga aaacgaccac cactggcacg aaggcaacta ctacgaatcc 780

ggctgcaacc cagccaccgg actcggcgcc gcccgacgca tcgcccacct cacctaccgt 840

ggcgaactag aaatcgacga acgcttcggc accaaagccc aaaagaacga aaacccactc 900

ggtccctacc gcaagcccga ccagcgcttc gccgtggaat cctacttgga ctaccaagca 960

gacaagctag tacagcgttt cgacgccggc tcctacgtct tgctcaccga cgccctcaac 1020

cgccacgaca ttggtcgcga ccgcggaggc ctcaacaagg cactcgaatc catcaaagtt 1080
ccagtccttg tcgcaggcgt agataccgat attttgtacc cctaccacca gcaagaacac 1140
ctctccagaa acctgggaaa tctactggca atggcaaaaa tcgtatcccc tgtcggccac 1200
gatgctttcc tcaccgaaag ccgccaaatg gatcgcatcg tgaggaactt cttcagcctc 1260
atctccccag acgaagacaa cccttcgacc tacatcgagt tctacatcta aactagttcg 1320
gacctaggga tatcgtcgac atcgatgctc ttctgcgtta attaacaatt gggatcctct 1380
agacccggga tttaaatcgc tagcgggctg ctaaaggaag cggaacacgt agaaagccag 1440
tccgcagaaa cggtgctgac cccggatgaa tgtcagctac tgggctatct ggacaaggga 1500
aaacgcaagc gcaaagagaa agcaggtagc ttgcagtggg cttacatggc gatagctaga 1560
ctgggcggtt ttatggacag caagcgaacc ggaattgcca gctggggcgc cctctggtaa 1620
ggttgggaag ccctgcaaag taaactggat ggctttcttg ccgccaagga tctgatggcg 1680
caggggatca agatctgatc aagagacagg atgaggatcg tttcgcatga ttgaacaaga 1740
tggattgcac gcaggttctc cggccgcttg ggtggagagg ctattcggct atgactgggc 1800
acaacagaca atcggctgct ctgatgccgc cgtgttccgg ctgtcagcgc aggggcgccc 1860
ggttcttttt gtcaagaccg acctgtccgg tgccctgaat gaactgcagg acgaggcagc 1920
gcggctatcg tggctggcca cgacgggcgt tccttgcgca gctgtgctcg acgttgtcac 1980
tgaagcggga agggactggc tgctattggg cgaagtgccg gggcaggatc tcctgtcatc 2040
tcaccttgct cctgccgaga aagtatccat catggctgat gcaatgcggc ggctgcatac 2100
gcttgatccg gctacctgcc cattcgacca ccaagcgaaa catcgcatcg agcgagcacg 2160
tactcggatg gaagccggtc ttgtcgatca ggatgatctg gacgaagagc atcaggggct 2220
cgcgccagcc gaactgttcg ccaggctcaa ggcgcgcatg cccgacggcg aggatctcgt 2280
cgtgacccat ggcgatgcct gcttgccgaa tatcatggtg gaaaatggcc gcttttctgg 2340
attcatcgac tgtggccggc tgggtgtggc ggaccgctat caggacatag cgttggctac 2400
ccgtgatatt gctgaagagc ttggcggcga atgggctgac cgcttcctcg tgctttacgg 2460
tatcgccgct cccgattcgc agcgcatcgc cttctatcgc cttcttgacg agttcttctg 2520
agcgggactc tggggttcga aatgaccgac caagcgacgc ccaacctgcc atcacgagat 2580
ttcgattcca ccgccgcctt ctatgaaagg ttgggcttcg gaatcgtttt ccgggacgcc 2640
ggctggatga tcctccagcg cggggatctc atgctggagt tcttcgccca cgctagcggc 2700
gcgccggccg gcccggtgtg aaataccgca cagatgcgta aggagaaaat accgcatcag 2760
gcgctcttcc gcttcctcgc tcactgactc gctgcgctcg gtcgttcggc tgcggcgagc 2820
ggtatcagct cactcaaagg cggtaatacg gttatccaca gaatcagggg ataacgcagg 2880
aaagaacatg tgagcaaaag gccagcaaaa ggccaggaac cgtaaaaagg ccgcgttgct 2940
ggcgtttttc cataggctcc gcccccctga cgagcatcac aaaaatcgac gctcaagtca 3000
gaggtggcga aacccgacag gactataaag ataccaggcg tttccccctg gaagctccct 3060
cgtgcgctct cctgttccga ccctgccgct taccggatac ctgtccgcct ttctcccttc 3120
gggaagcgtg gcgctttctc atagctcacg ctgtaggtat ctcagttcgg tgtaggtcgt 3180
tcgctccaag ctgggctgtg tgcacgaacc ccccgttcag cccgaccgct gcgccttatc 3240
cggtaactat cgtcttgagt ccaacccggt aagacacgac ttatcgccac tggcagcagc 3300
cactggtaac aggattagca gagcgaggta tgtaggcggt gctacagagt tcttgaagtg 3360

gtggcctaac tacggctaca ctagaaggac agtatttggt atctgcgctc tgctgaagcc 3420
agttaccttc ggaaaaagag ttggtagctc ttgatccggc aaacaaacca ccgctggtag 3480
cggtggtttt tttgtttgca agcagcagat tacgcgcaga aaaaaaggat ctcaagaaga 3540
tcctttgatc ttttctacgg ggtctgacgc tcagtggaac gaaaactcac gttaagggat 3600
tttggtcatg agattatcaa aaaggatctt cacctagatc cttttaaagg ccggccgcgg 3660
ccgcgcaaag tcccgcttcg tgaaaatttt cgtgccgcgt gattttccgc caaaaacttt 3720
aacgaacgtt cgttataatg gtgtcatgac cttcacgacg aagtactaaa attggcccga 3780
atcatcagct atggatctct ctgatgtcgc gctggagtcc gacgcgctcg atgctgccgt 3840
cgatttaaaa acggtgatcg gatttttccg agctctcgat acgacggacg cgccagcatc 3900
acgagactgg gccagtgccg cgagcgacct agaaactctc gtggcggatc ttgaggagct 3960
ggctgacgag ctgcgtgctc ggccagcgcc aggaggacgc acagtagtgg aggatgcaat 4020
cagttgcgcc tactgcggtg gcctgattcc tccccggcct gacccgcgag gacggcgcgc 4080
aaaatattgc tcagatgcgt gtcgtgccgc agccagccgc gagcgcgcca acaaacgcca 4140
cgccgaggag ctggaggcgg ctaggtcgca aatggcgctg gaagtgcgtc ccccgagcga 4200
aattttggcc atggtcgtca cagagctgga agcggcagcg agaattatcg cgatcgtggc 4260
ggtgcccgca ggcatgacaa acatcgtaaa tgccgcgttt cgtgtgccgt ggccgcccag 4320
gacgtgtcag cgccgccacc acctgcaccg aatcggcagc agcgtcgcgc gtcgaaaaag 4380
cgcacaggcg gcaagaagcg ataagctgca cgaatacctg aaaaatgttg aacgccccgt 4440
gagcggtaac tcacagggcg tcggctaacc cccagtccaa acctgggaga aagcgctcaa 4500
aaatgactct agcggattca cgagacattg acacaccggc ctggaaattt tccgctgatc 4560
tgttcgacac ccatcccgag ctcgcgctgc gatcacgtgg ctggacgagc gaagaccgcc 4620
gcgaattcct cgctcacctg ggcagagaaa atttccaggg cagcaagacc cgcgacttcg 4680
ccagcgcttg gatcaaagac ccggacacgg agaaacacag ccgaagttat accgagttgg 4740
ttcaaaatcg cttgcccggt gccagtatgt tgctctgacg cacgcgcagc acgcagccgt 4800
gcttgtcctg gacattgatg tgccgagcca ccaggccggc gggaaaatcg agcacgtaaa 4860
ccccgaggtc tacgcgattt tggagcgctg ggcacgcctg gaaaaagcgc cagcttggat 4920
cggcgtgaat ccactgagcg ggaaatgcca gctcatctgg ctcattgatc cggtgtatgc 4980
cgcagcaggc atgagcagcc cgaatatgcg cctgctggct gcaacgaccg aggaaatgac 5040
ccgcgttttc ggcgctgacc aggctttttc acataggctg agccgtggcc actgcactct 5100
ccgacgatcc cagccgtacc gctggcatgc ccagcacaat cgcgtggatc gcctagctga 5160
tcttatggag gttgctcgca tgatctcagg cacagaaaaa cctaaaaaac gctatgagca 5220
ggagttttct agcggacggg cacgtatcga agcggcaaga aaagccactg cggaagcaaa 5280
agcacttgcc acgcttgaag caagcctgcc gagcgccgct gaagcgtctg gagagctgat 5340
cgacggcgtc cgtgtcctct ggactgctcc agggcgtgcc gcccgtgatg agacggcttt 5400
tcgccacgct ttgactgtgg gataccagtt aaaagcggct ggtgagcgcc taaaagacac 5460
caagggtcat cgagcctacg agcgtgccta caccgtcgct caggcggtcg gaggaggccg 5520
tgagcctgat ctgccgccgg actgtgaccg ccagacggat tggccgcgac gtgtgcgcgg 5580
ctacgtcgct aaaggccagc cagtcgtccc tgctcgtcag acagagacgc agagccagcc 5640

gaggcgaaaa gctctggcca ctatgggaag acgtggcggt aaaaaggccg cagaacgctg 5700

gaaagaccca aacagtgagt acgcccgagc acagcgagaa aaactagcta agtccagtca 5760

acgacaagct aggaaagcta aaggaaatcg cttgaccatt gcaggttggt ttatgactgt 5820

tgagggagag actggctcgt ggccgacaat caatgaagct atgtctgaat ttagcgtgtc 5880

acgtcagacc gtgaatagag cacttaaggt ctgcgggcat tgaacttcca cgaggacgcc 5940

gaaagcttcc cagtaaatgt gccatctcgt aggcagaaaa cggttccccc gtagggtctc 6000

tctcttggcc tcctttctag gtcgggctga ttgctcttga agctctctag gggggctcac 6060

accataggca gataacgttc cccaccggct cgcctcgtaa gcgcacaagg actgctccca 6120

aagatcttca aagccactgc cgcgactgcc ttcgcgaagc cttgccccgc ggaaatttcc 6180

tccaccgagt tcgtgcacac ccctatgcca agcttctttc accctaaatt cgagagattg 6240

gattcttacc gtggaaattc ttcgcaaaaa tcgtcccctg atcgcccttg cgacgttggc 6300

gtcggtgccg ctggttgcgc ttggcttgac cgacttgatc agcggccgc 6349

<210> 35
<211> 29
<212> DNA
<213> Corynebacterium glutamicum

<220>
<223> SEQ_ID_35

<400> 35
gagactcgag agctgccaat tattccggg 29

<210> 36
<211> 41
<212> DNA
<213> Corynebacterium glutamicum

<220>
<223> SEQ_ID_36

<400> 36
cctgaaggcg cgagggtggg catgggtaaa aaatcctttc g 41

<210> 37
<211> 19
<212> DNA
<213> Corynebacterium glutamicum

<220>
<223> SEQ_ID_37

<400> 37
cccaccctcg cgccttcag 19

<210> 38
<211> 18
<212> DNA
<213> Corynebacterium glutamicum

<220>
<223> SEQ_ID_38

<400> 38
ctgggtacat tgcggccc 18

<210> 39
<211> 6386
<212> DNA
<213> Corynebacterium glutamicum

<220>
<223> PCLIK5MCS

<220>
<221> misc_feature
<222> (6 )..(196 )
<223> Psod

<220>
<221> misc_feature
<222> (197 )..(1330 )
<223> metA

<220>
<221> misc_feature
<222> (1752 )..(2543 )
<223> KanR

<220>
<221> misc_feature
<222> (2810 )..(3670 )
<223> Ori-Ec (pMB) complement

<220>
<221> misc_feature
<222> (3816 )..(4490 )
<223> Orf1

<220>
<221> misc_feature
<222> (4824 )..(5945 )
<223> Rep Protein

<400> 39
tcgagagctg ccaattattc cgggcttgtg acccgctacc cgataaatag gtcggctgaa 60

aaatttcgtt gcaatatcaa caaaaaggcc tatcattggg aggtgtcgca ccaagtactt 120

ttgcgaagcg ccatctgacg gattttcaaa agatgtatat gctcggtgcg gaaacctacg 180

aaaggatttt ttacccatgc ccaccctcgc gccttcaggt caacttgaaa tccaagcgat 240

cggtgatgtc tccaccgaag ccggagcaat cattacaaac gctgaaatcg cctatcaccg 300

ctggggtgaa taccgcgtag ataaagaagg acgcagcaat gtcgttctca tcgaacacgc 360

cctcactgga gattccaacg cagccgattg gtgggctgac ttgctcggtc ccggcaaagc 420

catcaacact gatatttact gcgtgatctg taccaacgtc atcggtggtt gcaacggttc 480

caccggacct ggctccatgc atccagatgg aaatttctgg ggtaatcgct tccccgccac 540

gtccattcgt gatcaggtaa acgccgaaaa acaattcctc gacgcactcg gcatcaccac 600

ggtcgccgca gtacttggtg gttccatggg tggtgcccgc accctagagt gggccgcaat 660

gtacccagaa actgttggcg cagctgctgt tcttgcagtt tctgcacgcg ccagcgcctg 720

gcaaatcggc attcaatccg cccaaattaa ggcgattgaa aacgaccacc actggcacga 780

aggcaactac tacgaatccg gctgcaaccc agccaccgga ctcggcgccg cccgacgcat 840

cgcccacctc acctaccgtg gcgaactaga aatcgacgaa cgcttcggca ccaaagccca 900

aaagaacgaa aacccactcg gtccctaccg caagcccgac cagcgcttcg ccgtggaatc 960

ctacttggac taccaagcag acaagctagt acagcgtttc gacgccggct cctacgtctt 1020

gctcaccgac gccctcaacc gccacgacat tggtcgcgac cgcggaggcc tcaacaaggc 1080

actcgaatcc atcaaagttc cagtccttgt cgcaggcgta gataccgata ttttgtaccc 1140

ctaccaccag caagaacacc tctccagaaa cctgggaaat ctactggcaa tggcaaaaat 1200

cgtatcccct gtcggccacg atgctttcct caccgaaagc cgccaaatgg atcgcatcgt 1260

gaggaacttc ttcagcctca tctccccaga cgaagacaac ccttcgacct acatcgagtt 1320
ctacatctaa catatgacta gttcggacct agggatatcg tcgacatcga tgctcttctg 1380
cgttaattaa caattgggat cctctagacc cgggatttaa atcgctagcg ggctgctaaa 1440
ggaagcggaa cacgtagaaa gccagtccgc agaaacggtg ctgaccccgg atgaatgtca 1500
gctactgggc tatctggaca agggaaaacg caagcgcaaa gagaaagcag gtagcttgca 1560
gtgggcttac atggcgatag ctagactggg cggttttatg gacagcaagc gaaccggaat 1620
tgccagctgg ggcgccctct ggtaaggttg ggaagccctg caaagtaaac tggatggctt 1680
tcttgccgcc aaggatctga tggcgcaggg gatcaagatc tgatcaagag acaggatgag 1740
gatcgtttcg catgattgaa caagatggat tgcacgcagg ttctccggcc gcttgggtgg 1800
agaggctatt cggctatgac tgggcacaac agacaatcgg ctgctctgat gccgccgtgt 1860
tccggctgtc agcgcagggg cgcccggttc tttttgtcaa gaccgacctg tccggtgccc 1920
tgaatgaact gcaggacgag gcagcgcggc tatcgtggct ggccacgacg ggcgttcctt 1980
gcgcagctgt gctcgacgtt gtcactgaag cgggaaggga ctggctgcta ttgggcgaag 2040
tgccggggca ggatctcctg tcatctcacc ttgctcctgc cgagaaagta tccatcatgg 2100
ctgatgcaat gcggcggctg catacgcttg atccggctac ctgcccattc gaccaccaag 2160
cgaaacatcg catcgagcga gcacgtactc ggatggaagc cggtcttgtc gatcaggatg 2220
atctggacga agagcatcag gggctcgcgc cagccgaact gttcgccagg ctcaaggcgc 2280
gcatgcccga cggcgaggat ctcgtcgtga cccatggcga tgcctgcttg ccgaatatca 2340
tggtggaaaa tggccgcttt tctggattca tcgactgtgg ccggctgggt gtggcggacc 2400
gctatcagga catagcgttg gctacccgtg atattgctga agagcttggc ggcgaatggg 2460
ctgaccgctt cctcgtgctt tacggtatcg ccgctcccga ttcgcagcgc atcgccttct 2520
atcgccttct tgacgagttc ttctgagcgg gactctgggg ttcgaaatga ccgaccaagc 2580
gacgcccaac ctgccatcac gagatttcga ttccaccgcc gccttctatg aaaggttggg 2640
cttcggaatc gttttccggg acgccggctg gatgatcctc cagcgcgggg atctcatgct 2700
ggagttcttc gcccacgcta gcggcgcgcc ggccggcccg gtgtgaaata ccgcacagat 2760
gcgtaaggag aaaataccgc atcaggcgct cttccgcttc ctcgctcact gactcgctgc 2820
gctcggtcgt tcggctgcgg cgagcggtat cagctcactc aaaggcggta atacggttat 2880
ccacagaatc aggggataac gcaggaaaga acatgtgagc aaaaggccag caaaaggcca 2940
ggaaccgtaa aaaggccgcg ttgctggcgt ttttccatag gctccgcccc cctgacgagc 3000
atcacaaaaa tcgacgctca agtcagaggt ggcgaaaccc gacaggacta taaagatacc 3060
aggcgtttcc ccctggaagc tccctcgtgc gctctcctgt tccgaccctg ccgcttaccg 3120
gatacctgtc cgcctttctc ccttcgggaa gcgtggcgct ttctcatagc tcacgctgta 3180
ggtatctcag ttcggtgtag gtcgttcgct ccaagctggg ctgtgtgcac gaaccccccg 3240
ttcagcccga ccgctgcgcc ttatccggta actatcgtct tgagtccaac ccggtaagac 3300
acgacttatc gccactggca gcagccactg gtaacaggat tagcagagcg aggtatgtag 3360
gcggtgctac agagttcttg aagtggtggc ctaactacgg ctacactaga aggacagtat 3420
ttggtatctg cgctctgctg aagccagtta ccttcggaaa aagagttggt agctcttgat 3480
ccggcaaaca aaccaccgct ggtagcggtg gtttttttgt ttgcaagcag cagattacgc 3540
gcagaaaaaa aggatctcaa gaagatcctt tgatcttttc tacggggtct gacgctcagt 3600

ggaacgaaaa ctcacgttaa gggattttgg tcatgagatt atcaaaaagg atcttcacct 3660
agatcctttt aaaggccggc cgcggccgcg caaagtcccg cttcgtgaaa attttcgtgc 3720
cgcgtgattt tccgccaaaa actttaacga acgttcgtta taatggtgtc atgaccttca 3780
cgacgaagta ctaaaattgg cccgaatcat cagctatgga tctctctgat gtcgcgctgg 3840
agtccgacgc gctcgatgct gccgtcgatt taaaaacggt gatcggattt ttccgagctc 3900
tcgatacgac ggacgcgcca gcatcacgag actgggccag tgccgcgagc gacctagaaa 3960
ctctcgtggc ggatcttgag gagctggctg acgagctgcg tgctcggcca gcgccaggag 4020
gacgcacagt agtggaggat gcaatcagtt gcgcctactg cggtggcctg attcctcccc 4080
ggcctgaccc gcgaggacgg cgcgcaaaat attgctcaga tgcgtgtcgt gccgcagcca 4140
gccgcgagcg cgccaacaaa cgccacgccg aggagctgga ggcggctagg tcgcaaatgg 4200
cgctggaagt gcgtcccccg agcgaaattt tggccatggt cgtcacagag ctggaagcgg 4260
cagcgagaat tatcgcgatc gtggcggtgc ccgcaggcat gacaaacatc gtaaatgccg 4320
cgtttcgtgt gccgtggccg cccaggacgt gtcagcgccg ccaccacctg caccgaatcg 4380
gcagcagcgt cgcgcgtcga aaaagcgcac aggcggcaag aagcgataag ctgcacgaat 4440
acctgaaaaa tgttgaacgc cccgtgagcg gtaactcaca gggcgtcggc taacccccag 4500
tccaaacctg ggagaaagcg ctcaaaaatg actctagcgg attcacgaga cattgacaca 4560
ccggcctgga aattttccgc tgatctgttc gacacccatc ccgagctcgc gctgcgatca 4620
cgtggctgga cgagcgaaga ccgccgcgaa ttcctcgctc acctgggcag agaaaatttc 4680
cagggcagca agacccgcga cttcgccagc gcttggatca aagacccgga cacggagaaa 4740
cacagccgaa gttataccga gttggttcaa aatcgcttgc ccggtgccag tatgttgctc 4800
tgacgcacgc gcagcacgca gccgtgcttg tcctggacat tgatgtgccg agccaccagg 4860
ccggcgggaa aatcgagcac gtaaaccccg aggtctacgc gattttggag cgctgggcac 4920
gcctggaaaa agcgccagct tggatcggcg tgaatccact gagcgggaaa tgccagctca 4980
tctggctcat tgatccggtg tatgccgcag caggcatgag cagcccgaat atgcgcctgc 5040
tggctgcaac gaccgaggaa atgacccgcg ttttcggcgc tgaccaggct ttttcacata 5100
ggctgagccg tggccactgc actctccgac gatcccagcc gtaccgctgg catgcccagc 5160
acaatcgcgt ggatcgccta gctgatctta tggaggttgc tcgcatgatc tcaggcacag 5220
aaaaacctaa aaaacgctat gagcaggagt tttctagcgg acgggcacgt atcgaagcgg 5280
caagaaaagc cactgcggaa gcaaaagcac ttgccacgct tgaagcaagc ctgccgagcg 5340
ccgctgaagc gtctggagag ctgatcgacg gcgtccgtgt cctctggact gctccagggc 5400
gtgccgcccg tgatgagacg gcttttcgcc acgctttgac tgtgggatac cagttaaaag 5460
cggctggtga gcgcctaaaa gacaccaagg gtcatcgagc ctacgagcgt gcctacaccg 5520
tcgctcaggc ggtcggagga ggccgtgagc ctgatctgcc gccggactgt gaccgccaga 5580
cggattggcc gcgacgtgtg cgcggctacg tcgctaaagg ccagccagtc gtccctgctc 5640
gtcagacaga gacgcagagc cagccgaggc gaaaagctct ggccactatg ggaagacgtg 5700
gcggtaaaaa ggccgcagaa cgctggaaag acccaaacag tgagtacgcc cgagcacagc 5760
gagaaaaact agctaagtcc agtcaacgac aagctaggaa agctaaagga aatcgcttga 5820
ccattgcagg ttggtttatg actgttgagg gagagactgg ctcgtggccg acaatcaatg 5880

aagctatgtc tgaatttagc gtgtcacgtc agaccgtgaa tagagcactt aaggtctgcg 5940

ggcattgaac ttccacgagg acgccgaaag cttcccagta aatgtgccat ctcgtaggca 6000

gaaaacggtt cccccgtagg gtctctctct tggcctcctt tctaggtcgg gctgattgct 6060

cttgaagctc tctagggggg ctcacaccat aggcagataa cgttccccac cggctcgcct 6120

cgtaagcgca caaggactgc tcccaaagat cttcaaagcc actgccgcga ctgccttcgc 6180

gaagccttgc cccgcggaaa tttcctccac cgagttcgtg cacaccccta tgccaagctt 6240

ctttcaccct aaattcgaga gattggattc ttaccgtgga aattcttcgc aaaaatcgtc 6300

ccctgatcgc ccttgcgacg ttggcgtcgg tgccgctggt tgcgcttggc ttgaccgact 6360

tgatcagcgg ccgctcgatt taaatc 6386

<210> 40
<211> 1005
<212> DNA
<213> Corynebacterium glutamicum

<220>
<223> FRUCTOSE-1,6-BISPHOSPHATASE

<400> 40
atgaacctaa agaaccccga aacgccagac cgtaaccttg ctatggagct ggtgcgagtt 60

acggaagcag ctgcactggc ttctggacgt tgggttggac gtggcatgaa gaatgaaggc 120

gacggtgccg ctgttgacgc catgcgccag ctcatcaact cagtgaccat gaagggcgtc 180

gttgttatcg gcgagggcga aaaagacgaa gctccaatgc tgtacaacgg cgaagaggtc 240

ggaaccggct ttggacctga ggttgatatc gcagttgacc cagttgacgg caccaccctg 300

atggctgagg gtcgccccaa cgcaatttcc attctcgcag ctgcagagcg tggcaccatg 360

tacgatccat cctccgtctt ctacatgaag aagatcgccg tgggacctga ggccgcaggc 420

aagatcgaca tcgaagctcc agttgcccac aacatcaacg cggtggcaaa gtccaaggga 480

atcaaccctt ccgacgtcac cgttgtcgtg cttgaccgtc ctcgccacat cgaactgatc 540

gcagacattc gtcgtgcagg cgcaaaggtt cgtctcatct ccgacggcga cgttgcaggt 600

gcagttgcag cagctcagga ttccaactcc gtggacatca tgatgggcac cggcggaacc 660

ccagaaggca tcatcactgc gtgcgccatg aagtgcatgg gtggcgaaat ccagggcatc 720

ctggccccaa tgaacgattt cgagcgccag aaggcacacg acgctggtct ggttcttgat 780

caggttctgc acaccaacga tctggtgagc tccgacaact gctacttcgt ggcaaccggt 840

gtgaccaacg gtgacatgct ccgtggcgtt tcctaccgcg caaacggcgc aaccacccgt 900

tccctggtta tgcgcgcaaa gtcaggcacc atccgccaca tcgagtctgt ccaccagctg 960

tccaagctgc aggaatactc cgtggttgac tacaccaccg cgacc 1005

<210> 41
<211> 335
<212> PRT
<213> Corynebacterium glutamicum

<400> 41
Met Asn Leu Lys Asn Pro Glu Thr Pro Asp Arg Asn Leu Ala Met Glu
1               5                   10                  15

Leu Val Arg Val Thr Glu Ala Ala Ala Leu Ala Ser Gly Arg Trp Val
            20                  25                  30

Gly Arg Gly Met Lys Asn Glu Gly Asp Gly Ala Ala Val Asp Ala Met

35                  40                  45

Arg Gln Leu Ile Asn Ser Val Thr Met Lys Gly Val Val Val Ile Gly
    50                  55                  60

Glu Gly Glu Lys Asp Glu Ala Pro Met Leu Tyr Asn Gly Glu Glu Val
65                  70                  75                  80

Gly Thr Gly Phe Gly Pro Glu Val Asp Ile Ala Val Asp Pro Val Asp
                85                  90                  95

Gly Thr Thr Leu Met Ala Glu Gly Arg Pro Asn Ala Ile Ser Ile Leu
            100                 105                 110

Ala Ala Ala Glu Arg Gly Thr Met Tyr Asp Pro Ser Ser Val Phe Tyr
        115                 120                 125

Met Lys Lys Ile Ala Val Gly Pro Glu Ala Ala Gly Lys Ile Asp Ile
    130                 135                 140

Glu Ala Pro Val Ala His Asn Ile Asn Ala Val Ala Lys Ser Lys Gly
145                 150                 155                 160

Ile Asn Pro Ser Asp Val Thr Val Val Val Leu Asp Arg Pro Arg His
                165                 170                 175

Ile Glu Leu Ile Ala Asp Ile Arg Arg Ala Gly Ala Lys Val Arg Leu
            180                 185                 190

Ile Ser Asp Gly Asp Val Ala Gly Ala Val Ala Ala Ala Gln Asp Ser
        195                 200                 205

Asn Ser Val Asp Ile Met Met Gly Thr Gly Gly Thr Pro Glu Gly Ile
    210                 215                 220

Ile Thr Ala Cys Ala Met Lys Cys Met Gly Gly Glu Ile Gln Gly Ile
225                 230                 235                 240

Leu Ala Pro Met Asn Asp Phe Glu Arg Gln Lys Ala His Asp Ala Gly
                245                 250                 255

Leu Val Leu Asp Gln Val Leu His Thr Asn Asp Leu Val Ser Ser Asp
            260                 265                 270

Asn Cys Tyr Phe Val Ala Thr Gly Val Thr Asn Gly Asp Met Leu Arg
        275                 280                 285

Gly Val Ser Tyr Arg Ala Asn Gly Ala Thr Thr Arg Ser Leu Val Met
    290                 295                 300

Arg Ala Lys Ser Gly Thr Ile Arg His Ile Glu Ser Val His Gln Leu
305                 310                 315                 320

Ser Lys Leu Gln Glu Tyr Ser Val Val Asp Tyr Thr Thr Ala Thr
                325                 330                 335


<210> 42
<211> 6
<212> DNA
<213> Corynebacterium glutamicum

<220>
<223> POTENTIELLE_-10-REGION_1

<400> 42
tgcaat                                                                  6


<210> 43
<211> 7
<212> DNA
<213> Corynebacterium glutamicum

<220>
<223> POTENTIELLE_-10-REGION_2

<400> 43
tatcatt                                                                  7


<210> 44
<211> 7
<212> DNA
<213> Corynebacterium glutamicum

<220>
<223> RIBOSOMALE_BINDUNGSSTELLE\SHINE-DALGARNO

<400> 44
gaaagga                                                                  7

## Beschreibung

Die vorliegende Erfindung betrifft die Verwendung von Nukleinsäuresequenzen zur Regulation der Transkription und Expression von Genen, die neuen Promotoren und Expressionseinheiten selbst, Verfahren zur Veränderung oder Verursachung der Transkriptionsrate und/oder Expressionsrate von Genen, Expressionskasetten, enthaltend die Expressionseinheiten, genetisch veränderte Mikroorganismen mit veränderter oder verursachter Trankriptionsrate und/oder Expressionsrate sowie Verfahren zur Herstellung von biosynthetischen Produkten durch Kultivierung der genetisch veränderten Mikroorganismen.

Verschiedene biosynthetische Produkte, wie beispielsweise Feinchemikalien, wie unter anderem Aminosäuren, Vitamine aber auch Proteine werden über natürliche Stoffwechselprozesse in Zellen hergestellt und werden in vielen Industriezweigen verwendet, einschließlich der Nahrungsmittel-, Futtermittel-, Kosmetik-, Feed-, Food- und pharmazeutischen Industrie. Diese Substanzen, die zusammen als Feinchemikalien/Proteine bezeichnet werden, umfassen unter anderem organische Säuren, sowohl proteinogene als auch nicht-proteinogene Aminosäuren, Nukleotide und Nukleoside, Lipide und Fettsäuren, Diole, Kohlenhydrate, aromatische Verbindungen, Vitamine und Cofaktoren, sowie Proteine und Enzyme. Ihre Produktion erfolgt am zweckmäßigsten im Großmaßstab mittels Anzucht von Bakterien, die entwickelt wurden, um große Mengen der jeweils gewünschten Substanz zu produzieren und sezernieren. Für diesen Zweck besonders geeignete Organismen sind coryneforme Bakterien, grampositive nicht-pathogene Bakterien.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen, wie zum Beispiel Rührung und Versorgung mit Sauerstoff, oder die Zusammensetzung der Nährmedien, wie zum Beispiel die Zuckerkonzentration während der Fermentation, oder die Aufarbeitung zum Produkt, beispielsweise durch lonenaustauschchromatographie aber auch Sprühtrocknung, oder die intrinsischen Leistungseigenschaften des Mikroorganismus selbst betreffen.

Seit einigen Jahren werden ebenfalls Methoden der rekombinanten DNA-Technik zur Stammverbesserung von Feinchemikalien/Proteine produzierender Stämme von Corynebacterium eingesetzt, indem man einzelne Gene amplifiziert und die Auswirkung auf die Produktion von Feinchemikalien/Proteine untersucht.

Andere Wege, um ein Verfahren für die Herstellung Feinchemikalien, Aminosäuren oder Proteine zu entwickeln, oder die Produktivität eines bereits existierenden Verfahrens für die Herstellung Feinchemikalien, Aminosäuren oder Proteine zu erhöhen bzw. zu verbessern, sind die Expression eines oder mehrerer Gene zu erhöhen bzw. zu verändern und oder die Translation einer mRNA durch geeignete Polynukleotidsequenzen zu beeinflussen. Beeinflussung kann in diesem Zusammenhang die Erhöhung, Verringerung, oder auch andere Parameter der Expression von Genen wie zeitliche Expressionsmuster umfassen.

Dem Fachmann sind unterschiedliche Bestandteile von bakteriellen Regulationssequenzen bekannt. Man unterscheidet die Bindungstellen von Regulatoren, auch Operatoren genannt, die Bindungstellen von RNA-Polymerase-Holoenzymen, auch -35 und - 10 Regionen genannt, und die Bindungsstelle von Ribosomaler 16S-RNA, auch Ribosomale Bindungsstelle oder auch Shine-Dalgarno-Sequenz genannt.

Als Sequenz einer Ribosomalen Bindungsstelle, auch Shine-Dalgarno-Sequenz genannt, im Sinne dieser Erfindung werden Polynukleotidsequenzen verstanden, die sich bis zu 20 Basen stromauf des Initiationskodon der Translation befinden.

In der Literatur (E. coli und S. typhimurium, Neidhardt F.C. 1995 ASM Press) wird beschrieben, dass sowohl die Zusammensetzung der Polynukletidsequenz der Shine-Delgarno-Sequenz, die Sequenzabfolge der Basen, aber auch der Abstand einer in der Shine-Delgarno-Sequenz enthaltenen Polynukletidsequenz zum einen wesentlichen Einfluss auf die Initiationstionsrate der Translation hat.

Nukleinsäuresequenzen mit Promotoraktivität können die Bildung von mRNA auf unterschiedliche Weise beeinflussen. Promotoren, deren Aktivität unabhängig von der physiologischen Wachstumsphase des Organismus sind, nennt man konstitutiv. Wiederum andere Promotoren reagieren auf externe chemische, wie physikalische Stimuli wie Sauerstoff, Metabolite, Hitze, pH, etc.. Wiederum andere zeigen in unterschiedlichen Wachstumsphasen eine starke Abhängigkeit ihrer Aktivität. Beispielsweise sind in der Literatur Promotoren beschrieben, die während der exponentiellen Wachstumsphase von Mikroorganismen eine besonders ausgeprägte Aktivität zeigen, oder aber auch genau in der stationären Phase des mikrobiellen Wachstums. Beide Charakteristika von Promotoren können für eine Produktion von Feinchemikalien und Proteine je nach Stoffwechselweg einen günstigen Einfluss auf die Produktivität haben.

Zum Beispiel kann man Promotoren die während des Wachstums die Expresssion eines Gens ausschalten, diese aber nach einem optimalen Wachstum anschalten dazu nutzen, ein Gen zu regulieren, das die Produktion eines Metaboliten kontrolliert. Der veränderte Stamm weist dann die gleichen Wachstumsparameter wie der Ausgangsstamm auf, produziert aber mehr Produkt pro Zelle. Diese Art der Modifizierung kann sowohl den Titer (g Produkt/Liter) als auch die C-Ausbeute (g Produkt/g-C-Quelle) erhöhen.

In Corynebacterium Spezies konnten bereits solche Nukleotidsequenzen isoliert werden, die für eine Erhöhung bzw. eine Abschwächung der Genexpression genutzt werden können. Diese regulierten Promotoren können die Rate, mit der ein Gen transkribiert wird, abhängig von den internen und/oder externen Bedingungen der Zelle erhöhen oder erniedrigen. Zum Teil kann die Anwesenheit eines bestimmten Faktors, bekannt als Inducer, die Rate der Transkrition vom Promotor stimulieren. Inducer können direkt oder aber indirekt die Transkription vom Promotor beeinflussen. Eine andere Klasse von Faktoren, bekannt als Suppressoren ist in der Lage, die Transkription vom Promotor zu reduzieren oder aber zu inhibieren. Wie auch die Inducer, können auch die Suppressoren direkt oder indirekt wirken. Es sind jedoch auch Promotoren bekannt, die über die Temperatur reguliert werden. So kann der Level der Transkription solcher Promotoren zum Beispiel durch eine Erhöhung der Wachstumstemperatur über die normale Wachstumstemperatur der Zelle erhöht oder aber abgeschwächt werden.

Eine geringe Anzahl von Promotoren aus C. glutamicum wurden bis zum heutigen Tag beschrieben. Der Promotor des Malatsynthase-Gens aus C. glutamicum wurde im DE 4440118 beschrieben. Dieser Promotor wurde einem für ein Protein kodierendes Strukturgen vorgeschaltet. Nach Transformation eines solchen Konstrukts in ein coryneformes Bakterium wird die Expression des dem Promotor nachgeschalteten Strukturgen reguliert. Die Expression des Strukturgens wird induziert sobald dem Medium ein ensprechender Induktor zugesetzt wird.

Reinscheid et al., Microbiology 145:503 (1999) haben eine trankriptionelle Fusion zwischen dem pta-ack Promotor aus C. glutamicum und einem Reportergen (Chloramphenicol Acetyltransferase) beschrieben. Zellen von C. glutamicum, die eine solche transkriptionelle Fusion enthalten, wiesen eine erhöhte Expression des Reportergenes bei Wachstum auf Acetat haltigem Medium auf. Im Vergleich dazu zeigten transformierte Zellen, die auf Glucose wuchsen, keine erhöhte Expression dieses Reportergens.

In Pa'tek et al., Microbiology 142:1297 (1996) wurden einige DNA Sequenzen aus C. glutamicum beschrieben, die die Expression eines Reportergens in C. glutamicum Zellen verstärken können, beschrieben. Diese Sequenzen wurden miteinander verglichen, um Consensus-Sequenzen für C. glutamicum Promotoren zu definieren.

Weitere DNA-Sequenzen aus C. glutamicum, die zur Regulation der Genexpression genutzt werden können, sind im Patent WO 02/40679 beschrieben worden. Diese isolierten Polynukleotide stellen Expressionseinheiten aus Corynebakterium glutamicum dar, die entweder zur Erhöhung oder aber zur Verringerung einer Genexpression genutzt werden können. Weiterhin sind in diesem Patent rekombinante Plasmide beschrieben, auf denen die Expressionseinheiten aus Corynebakterium glutamicum mit heterologen Genen assoziiert sind. Die hier beschrieben Methode , Fusion von einem Promotor aus Corynebakterium glutamicum mit einem heterlogen Gen, kann unter anderem zur Regulation der Gene der Aminosäurebiosynthese eingesetzt werden.

Der Erfindung lag die Aufgabe zugrunde weitere Promotoren und/oder Expressionseinheiten mit vorteilhaften Eigenschaften zur Verfügung zustellen.

Demgemäß wurde gefunden, dass man Nukleinsäuren mit Promotoraktivität, enthaltend
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist
   oder
C) eine Nukleinsäuresequenz, die mit der Nukleinsäuresequenz SEQ. ID. NO. 1 unter stringenten Bedingungen hybridisiert oder
D) funktionell äquivalente Fragmente der Sequenzen unter A), B) oder C)
zur Transkription von Genen verwenden kann.

Unter "Transkription" wird erfindungsgemäß der Prozess verstanden, durch den ausgehend von einer DNA-Matrize ein komplementäres RNA-Molekül hergestellt wird. An diesem Prozess sind Proteine wie die RNA-Polymerase sogenannte Sigma-Faktoren und transkriptionelle Regulatorproteine beteiligt. Die synthetisierte RNA dient dann als Matrize im Prozess der Translation, der dann zum biosynthetisch aktiven Protein führt.

Die Bildungsrate, mit der ein biosynthetsich aktives Protein hergestellt wird, ist ein Produkt aus der Rate der Transkription und der Translation. Beide Raten können erfindungsgemäß beeinflusst werden und damit die Rate der Bildung von Produkten in einem Mikroorganismus beeinflussen.

Unter einem "Promotor" oder einer "Nukleinsäure mit Promotoraktivität" wird erfindungsgemäß eine Nukleinsäure verstanden, die in funktioneller Verknüpfung mit einer zu trankripierenden Nukleinsäure, die Transkription dieser Nukleinsäure reguliert.

Unter einer "funktionellen Verknüpfung" versteht man in diesem Zusammenhang beispielsweise die sequentielle Anordnung einer der erfindungsgemäßen Nukleinsäuren mit Promotoraktivität und einer zu transkribierenden Nukleinsäuresequenz und ggf. weiterer regulativer Elemente wie zum Beispiel Nukleinsäureseuqenzen, die die Transkription von Nukleinsäuren gewährleisten, sowie zum Beipsiel einen Terminator derart, dass jedes der regulativen Elemente seine Funktion bei der Transkription der der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu trankribierende Nukleinsäuresequenz hinter (d.h. am 3'-Ende) der erfindungsgemäßen Promotorsequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Unter "Promotoraktivität" wird erfindungsgemäß die in einer bestimmten Zeit durch den Promotor gebildete Menge RNA, also die Trankriptionsrate verstanden.

Unter "spezifischer Promotoraktivität" wird erfindungsgemäß die in einer bestimmten Zeit durch den Promotor gebildete Menge RNA pro Promotor verstanden.

Unter dem Begriff "Wildtyp" wird erfindungsgemäß der entsprechende Ausgangsmikroorganismus verstanden.

Je nach Zusammenhang kann unter dem Begriff "Mikroorganismus" der Ausgangsmikroorganismus (Wildtyp) oder ein erfindungsgemäßer, genetisch veränderter Mikroorganismus oder beides verstanden werden.

Vorzugsweise und insbesondere in Fällen, in denen der Mikroorganismus oder der Wildtyp nicht eindeutig zugeordnet werden kann, wird unter "Wildtyp" für die Veränderung oder Verursachung der Promotoraktivität oder Trankriptionsrate, für die Veränderung oder Verursachung der Expressionsaktivität oder Expressionsrate und für die Erhöhung des Gehalts an biosynthetischen Produkten jeweils ein Referenzorganismus verstanden.

In einer bevorzugten Ausführungsform ist dieser Referenzorganismus Corynebakterium glutamicum ATCC 13032.

In einer bevorzugten Ausführungsform werden Ausgangsmikroorganismen verwendet die bereits in der Lage sind, die gewünschte Feinchemikalie herzustellen. Besonders bevorzugt sind dabei unter den besonders bevorzugten Mikroorganismen der Bakterien der Gattung Corynebacterien und den besonders bevorzugten Feinchemikalien L-Lysin, L-Methionin und L-Threonin, diejenigen Ausgangsmikroorganismen die bereits in der Lage sind, L-Lysin, L-Methionin und/oder L-Threonin herzustellen. Dies sind besonderes bevorzugt Corynebakterien bei denen beispielsweise das Gen kodierend für eine Aspartokinase (ask-Gen) dereguliert ist oder die feed-back-Inhibierung aufgehoben oder reduziert ist. Beispielsweise weisen solche Bakterien im ask-Gen eine Mutation auf, die zu einer Reduzierung oder Aufhebung der feed-back-Inhibierung führen, wie beispielsweise die Mutation T311l.

Bei einer "verursachten Promotoraktivität" oder Transkriptionsrate im Bezug auf ein Gen im Vergleich zum Wildtyp wird somit im Vergleich zum Wildtyp die Bildung einer RNA verursacht, die im Wildtyp so nicht vorhanden war.

Bei einer veränderten Promotoraktivität oder Transkriptionsrate im Bezug auf ein Gen im Vergleich zum Wildtyp wird somit im Vergleich zum Wildtyp in einer bestimmten Zeit die gebildete Menge der RNA verändert.

Unter "verändert" wird in diesem Zusammenhang bevorzugt erhöht oder erniedrigt verstanden.

Dies kann beispielsweise durch Erhöhung oder Reduzierung der spezifischen Promotoraktivität des endogenen erfindungsgemäßen Promotors, beispielsweise durch Mutation des Promotors oder durch Stimmulierung oder Hemmung des Promotors erfolgen.

Weiterhin kann die erhöhte Promotoraktivität oder Transkriptionsrate beispielsweise durch Regulation der Transkription von Genen im Mikroorganismus durch erfindungsgemäße Nukleinsäuren mit Promotoraktivität oder durch Nukleinsäuren mit erhöhter spezifischer Promotoraktivität erreicht werden, wobei die Gene in Bezug auf die Nukleinsäuren mit Promotoraktivität heterolog sind.

Vorzusgweise wird die Regulation der Transkription von Genen im Mikroorganismus durch erfindungsgemäße Nukleinsäuren mit Promotoraktivität oder durch Nukleinsäuren mit erhöhter spezifischer Promotoraktivität dadurch erreicht, dass man
eine oder mehrere erfindungsgemäße Nukleinsäuren mit Promotoraktivität, gegebenenfalls mit veränderter spezifischer Promotoraktivität, in das Genom des Mikroorganismus einbringt, so dass die Transkription eines oder mehrerer endogenen Gene unter der Kontrolle der eingebrachten erfindungsgemäßen Nukleinsäure mit Promotoraktivität gegebenenfalls mit veränderter spezifischer Promotoraktivität, erfolgt oder
ein oder mehrere Gene in das Genom des Mikrorganismus einbringt, so dass die Transkription eines oder mehrerer der eingebrachten Gene unter der Kontrolle der endogenen erfindungsgemäßen Nukleinsäuren mit Promotoraktivität, gegebenenfalls mit veränderter spezifischer Promotoraktivität, erfolgt oder
ein oder mehrere Nukleinsäurekonstrukte, enthaltend eine erfindungsgemäße Nukleinsäure mit Promotoraktivität, gegebenenfalls mit veränderter spezifischer Promotoraktivität, und funktionell verknüpft eine oder mehrere, zu transkribierende Nukleinsäuren, in den Mikroorganismus einbringt.

Die erfindungsgemäßen Nukleinsäuren mit Promotoraktivität enthalten
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist
   oder
C) eine Nukleinsäuresequenz, die mit der Nukleinsäuresequenz SEQ. ID. NO. 1 unter stringenten Bedingungen hybridisiert oder
D) funktionell äquivalente Fragmente der Sequenzen unter A), B) oder C)

Die Nukleinsäuresequenz SEQ. ID. NO. 1 stellt die Promotorsequenz der Superoxid-Dismutase (Psod) aus Corynebakterium glutamicum dar. SEQ. ID.NO. 1 entspricht der Promotorsequenz des Wildtyps.

Die Erfindung betrifft weiterhin Nukleinsäuren mit Promotoraktivität enthaltend eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist.

Weitere natürliche erfindungsgemäße Beispiele für erfindungsgemäße Promotoren lassen sich beispielsweise aus verschiedenen Organismen, deren genomische Sequenz bekannt ist, durch Identitätsvergleiche der Nukleinsäuresequenzen aus Datenbanken mit der vorstehend beschriebenen Sequenzen SEQ ID NO: 1 leicht auffinden.

Künstliche erfindungsgemäße Promotor-Sequenzen lassen sich ausgehend von der Sequenz SEQ ID NO: 1 durch künstliche Variation und Mutation, beispielsweise durch Substitution, Insertion oder Deletion von Nukleotiden leicht auffinden.

Unter dem Begriff "Substitution" ist in der Beschreibung der Austausch einer oder mehrerer Nukleotide durch ein oder mehrere Nukleotide zu verstehen. "Deletion" ist das Ersetzen eines Nukleotides durch eine direkte Bindung. Insertionen sind Einfügungen von Nukleotiden in die Nukleinsäuresequenz, wobei formal eine direkte Bindung durch ein oder mehrere Nukleotide ersetzt wird.

Unter Identität zwischen zwei Nukleinsäuren wird die Identität der Nukleotide über die jeweils gesamte Nukleinsäurelänge verstanden, insbesondere die Identität die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:
Multiple alignment parameter:
   Gap opening penalty 10
   Gap extension penalty 10
   Gap separation penalty range 8
   Gap separation penalty off
   % identity for alignment delay 40
   Residue specific gaps off
   Hydrophilic residue gap off
   Transition weighing 0
Pairwise alignment parameter:
   FAST algorithm on
   K-tuplesize 1
   Gap penalty 3
   Window size 5
   Number of best diagonals 5

Unter einer Nukleinsäuresequenz, die eine Identität von mindestens 90 % mit der Sequenz SEQ ID NO: 1 aufweist, wird dementsprechend eine Nukleinsäuresequenz verstanden, die bei einem Vergleich seiner Sequenz mit der Sequenz SEQ ID NO: 1, insbesondere nach obigen Programmlogarithmus mit obigem Parametersatz eine Identität von mindestens 90 % aufweist.

Besonders bevorzugte Promotoren weisen mit der Nukleinsäuresequenz SEQ. ID. NO. 1 eine Identität von 91 %, bevorzugter 92%, 93%, 94%, 95%, 96%, 97%, 98%, besonders bevorzugt 99% auf.

Weitere natürliche Beispiele für Promotoren lassen sich weiterhin ausgehend von den vorstehend beschriebenen Nukleinsäuresequenzen, insbesondere ausgehend von der Sequenz SEQ ID NO: 1 aus verschiedenen Organismen, deren genomische Sequenz nicht bekannt ist, durch Hybridisierungstechniken in an sich bekannter Weise leicht auffinden.

Ein weiterer Gegenstand der Erfindung betrifft daher Nukleinsäuren mit Promotoraktivität, enthaltend eine Nukleinsäuresequenz, die mit der Nukleinsäuresequenz SEQ. ID. No. 1 unter stringenten Bedingungen hybridisiert. Diese Nukleinsäuresequenz umfasst mindestens 10, bevorzugter mehr als 12,15,30,50 oder besonders bevorzugt mehr als 150 Nukleotide.

Die Hybridisierung erfolgt erfinungsgemäß unter stringenten Bedingungen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben:

Unter stringenten Hybridisierungs-Bedingungen werden insbesondere verstanden: Die über Nacht Inkubation bei 42°C in einer Lösung bestehend aus 50 % Formamid, 5 x SSC (750 mM NaCl, 75 mM Tri-Natrium Citrat), 50 mM Natrium Phosphat (ph7,6), 5x Denhardt Lösung, 10% Dextransulfat und 20 g/ml denaturierte, gescheerte Lachsspermien-DNA, gefolgt von einem Waschen der Filter mit 0,1x SSC bei 65°C.

Unter einem "funktionell äquivalenten Fragment" werden für Nukleinsäuresequenzen mit Promotoraktivität, Fragmente verstanden die im wesentlichen die gleiche oder eine höhere spezifische Promotoraktivität aufweisen wie die Ausgangssequenz.

Unter "im wesentlichen gleich" wird eine spezifische Promotoraktivität verstanden die mindestens 50%, vorzugsweise 60%, bevorzugter 70%, bevorzugter 80%, bevorzugter 90%, besonders bevorzugt 95% der spezifischen Promotoraktivität der Ausgangssequenz aufweist.

Unter "Fragmente" werden Teilsequenzen der durch Ausführungsform A), B) oder C) beschriebenen Nukleinsäuren mit Promotoraktivität verstanden. Vorzusgweise weisen diese Fragmente mehr als 10, bevorzugter aber mehr als 12,15, 30, 50 oder besonders bevorzugts mehr als 150 zusammenhängende Nukleotide der Nukleinsäuresequenz SEQ. ID. NO. 1 auf.

Besonders bevorzugt ist die Verwendung der Nukleinsäuresequenz SEQ. ID. NO. 1 als Promotor, d.h. zur Transkriptiion von Genen.

Die SEQ. ID. NO. 1 ist ohne Funktionszuordnung im Genbank-Eintrag AP005283 beschrieben worden. Daher betrifft die Erfindung ferner die neuen, erfindungsgemäßen Nukleinsäuresequenzen mit Promotoraktivität.

Insbesondere betrifft die Erfindung eine Nukleinsäure mit Promotoraktivität, enthaltend
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist
   oder
C) eine Nukleinsäuresequenz, die mit der Nukleinsäuresequenz SEQ. ID. NO. 1 unter stringenten Bedingungen hybridisiert oder
D) funktionell äquivalente Fragmente der Sequenzen unter A), B) oder C),
mit der Maßgabe, dass die Nukleinsäure mit der Sequenz SEQ. ID. NO. 1 ausgenommen ist.

Alle vorstehend erwähnten Nukleinsäuren mit Promotoraktivität sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Die Erfindung betrifft ferner die Verwendung einer Expressionseinheit, enthaltend eine der erfindungsgemäßen Nukleinsäuren mit Promotoraktivität und zusätzlich funktionell verknüpft eine Nukleinsäuresequenz, die die Translation von Ribonukleinsäuren gewährleistet, zur Expression von Genen.

Unter einer Expressioneinheit wird erfindungsgemäß eine Nukleinsäure mit Expressionsaktivität verstanden, also eine Nukleinsäure verstanden, die in funktioneller Verknüpfung mit einer zu exprimierenden Nukleinsäure oder Gens, die Expression, also die Transkription und die Translation dieser Nukleinsäure oder dieses Gens reguliert.

Unter einer "funktionellen Verknüpfung" versteht man in diesem Zusammenhang beispielsweise die sequentielle Anordnung einer der erfindungsgemäßen Expressionseinheit und einer transgen zu exprimierenden Nukleinsäuresequenz und ggf. weiterer regulativer Elemente wie zum Beispiel einem Terminator derart, dass jedes der regulativen Elemente seine Funktion bei der transgenen Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierende Nukleinsäuresequenz hinter (d.h. am 3'-Ende) der erfindungsgemäßen Expressionseinheitssequenz positioniert wird, so dass beide Sequenzen kovalent miteinander verbunden sind. Bevorzugt ist dabei der Abstand zwischen der Expressionseinheitssequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Unter "Expressionsaktivität" wird erfindungsgemäß die in einer bestimmten Zeit durch die Expressionseinheit gebildete Menge Protein, also die Expressionsrate verstanden.

Unter "spezifischer Expressionsaktivität" wird erfindungsgemäß die in einer bestimmten Zeit durch die Expressionseinheit gebildete Menge Protein pro Expressionseinheit verstanden.

Bei einer "verursachten Expressionsaktivität" oder Expressionsrate im Bezug auf ein Gen im Vergleich zum Wildtyp wird somit im Vergleich zum Wildtyp die Bildung eines Proteins verursacht, das im Wildtyp so nicht vorhanden war.

Bei einer "veränderten Expressionsaktivität" oder Expressionsrate im Bezug auf ein Gen im Vergleich zum Wildtyp wird somit im Vergleich zum Wildtyp in einer bestimmten Zeit die gebildete Menge des Proteins verändert.

Unter "verändert" wird in diesem Zusammenhang bevorzugt erhöht oder erniedrigt verstanden.

Dies kann beispielsweise durch Erhöhung oder Reduzierung der spezifischen Aktivität der endogenen Expressionseinheit, beispielsweise durch Mutation der Expressionseinheit oder durch Stimmulierung oder Hemmung der Expressionseinheit erfolgen.

Weiterhin kann die erhöhte Expressionsaktivität oder Expressionsrate beispielsweise durch Regulation der Expression von Genen im Mikroorganismus durch erfindungsgemäße Expressionseinheiten oder durch Expressionseinheiten mit Erhöhter spezifischer Expressionsaktivität erreicht werden, wobei die Gene im Bezug auf die Expressionseinheiten heterolog sind.

Vorzugsweise wird die Regulation der Expression von Genen im Mikroorganismus durch erfindungsgemäße Expressionseinheiten oder durch erfindungsgemäße Expressionseinheiten mit Erhöhter spezifischer Expressionsaktivitätdadurch erreicht, dass man
eine oder mehrere erfindungsgemäße Expressionseinheiten, gegebenenfalls mit veränderter spezifischer Expressionsaktivität, in das Genom des Mikroorganismus einbringt, so dass die Expression eines oder mehrerer endogenen Gene unter der Kontrolle der eingebrachten erfindungsgemäßen Expressionseinheiten, gegebenenfalls mit veränderter spezifischer Expressionsaktivität, erfolgt oder
ein oder mehrere Gene in das Genom des Mikrorganismus einbringt, so dass die Expression eines oder mehrerer der eingebrachten Gene unter der Kontrolle der endogenen, erfindungsgemäßen Expressionseinheiten, gegebenenfalls mit veränderter spezifischer Expressionsaktivität, erfolgt oder
ein oder mehrere Nukleinsäurekonstrukte, enthaltend eine erfindungsgemäße Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Die erfindungsgemäßen Expressionseinheiten, enthalten eine erfindungsgemäße, vorstehend bechriebene Nukleinsäure mit Promotoraktivität und zusätzlich funktionell verknüpft eine Nukleinsäuresequenz, die die Translation von Ribonukleinsäuren gewährleistet.

Vorzugsweise enthält diese Nukleinsäuresequenz, die die Translation von Ribonukleinsäuren gewährleistet, die Nukleinsäuresequenz SEQ. ID. NO. 42 als ribosomale Bindungsstelle.

In einer bevorzugten Ausführungsform enthält die erfindungsgemäße Expressionseinheit:
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist oder
G) eine Nukleinsäuresequenz, die mit der Nukleinsäuresequenz SEQ. ID. NO. 2 unter stringenten Bedingungen hybridisiert oder
H) funktionell äquivalente Fragmente der Sequenzen unter E), F) oder G).

Die Nukleinsäuresequenz SEQ. ID. NO. 2 stellt die Nukleinsäuresequenz der Expressionseinheit der Superoxid-Dismutase (Psod) aus Corynebakterium glutamicum dar. SEQ. ID.NO. 2 entspricht der Sequenz der Expressionseinheit des Wildtyps.

Die Erfindung betrifft weiterhin Expressionseinheiten, enthaltend eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist.

Weitere natürliche erfindungsgemäße Beispiele für erfindungsgemäße Expressionseinheiten lassen sich beispielsweise aus verschiedenen Organismen, deren genomische Sequenz bekannt ist, durch Identitätsvergleiche der Nukleinsäuresequenzen aus Datenbanken mit der vorstehend beschriebenen Sequenzen SEQ ID NO: 2 leicht auffinden.

Künstliche erfindungsgemäße Sequenzen der Expressionseinheiten lassen sich ausgehend von der Sequenz SEQ ID NO: 2 durch künstliche Variation und Mutation, beispielsweise durch Substitution, Insertion oder Deletion von Nukleotiden leicht auffinden.

Unter einer Nukleinsäuresequenz, die eine Identität von mindestens 90 % mit der Sequenz SEQ ID NO: 2 aufweist, wird dementsprechend eine Nukleinsäuresequenz verstanden, die bei einem Vergleich seiner Sequenz mit der Sequenz SEQ ID NO: 2, insbesondere nach obigen Programmlogarithmus mit obigem Parametersatz eine Identität von mindestens 90 % aufweist.

Besonders bevorzugte Expressionseinheiten weisen mit der Nukleinsäuresequenz SEQ. ID. NO. 2 eine Identität von 91 %, bevorzugter 92%, 93%, 94%, 95%, 96%, 97%, 98%, besonders bevorzugt 99% auf.

Weitere natürliche Beispiele für Expressionseinheiten lassen sich weiterhin ausgehend von den vorstehend beschriebenen Nukleinsäuresequenzen, insbesondere ausgehend von der Sequenz SEQ ID NO: 2 aus verschiedenen Organismen, deren genomische Sequenz nicht bekannt ist, durch Hybridisierungstechniken in an sich bekannter Weise leicht auffinden.

Ein weiterer Gegenstand der Erfindung betrifft daher Expressionseinheiten, enthaltend eine Nukleinsäuresequenz, die mit der Nukleinsäuresequenz SEQ. ID. No. 2 unter stringenten Bedingungen hybridisiert. Diese Nukleinsäuresequenz umfasst mindestens 10, bevorzugter mehr als 12,15,30,50 oder besonders bevorzugt mehr als 150 Nukleotide.

Unter "hybridisieren" versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Die Hybridisierung erfolgt erfinungsgemäß unter stringenten Bedingungen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben:

Unter stringenten Hybridisierungs-Bedingungen werden insbesondere verstanden: Die über Nacht Inkubation bei 42°C in einer Lösung bestehend aus 50 % Formamid, 5 x SSC (750 mM NaCl, 75 mM Tri-Natrium Citrat), 50 mM Natrium Phosphat (ph7,6), 5x Denhardt Lösung, 10% Dextransulfat und 20 g/ml denaturierte, gescheerte Lachsspermien-DNA, gefolgt von einem Waschen der Filter mit 0,1x SSC bei 65°C.

Die erfindungsgemäß Nukleotidsequenzen ermöglichen ferner die Erzeugung von Sonden und Primern, die zur Identifizierung und/oder Klonierung von homologer Sequenzen in anderen Zelltypen und Mikroorganismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Unter einem "funktionell äquivalenten Fragment" werden für Expressionseinheiten, Fragmente verstanden die im wesentlichen die gleiche oder eine höhere spezifische Expressionsaktivität aufweisen wie die Ausgangssequenz.

Unter "im wesentlichen gleich" wird eine spezifische Expressionsaktivität verstanden die mindestens 50%, vorzugsweise 60%, bevorzugter 70%, bevorzugter 80%, bevorzugter 90%, besonders bevorzugt 95% der spezifischen Expressionsaktivität der Ausgangssequenz aufweist.

Unter "Fragmente" werden Teilsequenzen der durch Ausführungsform E), F) oder G) beschriebenen Expressionseinheiten verstanden. Vorzusgweise weisen diese Fragmente mehr als 10, bevorzugter aber mehr als 12,15, 30, 50 oder besonders bevorzugts mehr als 150 zusammenhängende Nukleotide der Nukleinsäuresequenz SEQ. ID. NO. 1 auf.

Besonders bevorzugt ist die Verwendung der Nukleinsäuresequenz SEQ. ID. NO. 2 als Expressionseinheit, d.h. zur Expression von Genen.

Die SEQ. ID. NO. 2 ist ohne Funktionszuordnung im Genbank-Eintrag AP005283 beschrieben worden. Daher betrifft die Erfindung ferner die neuen, erfindungsgemäßen Expressionseinheiten.

Insbesondere betrifft die Erfindung eine Expressionseinheit, enthaltend eine erfindungsgemäße Nukleinsäure mit Promotoraktivität zusätzlich funktionell verknüpft eine Nukleinsäuresequenz, die die Translation von Ribonukleinsäuren gewährleistet.

Besonders bevorzugt betrifft die Erfindung eine Expressionseinheit, enthaltend
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist oder
G) eine Nukleinsäuresequenz, die mit der Nukleinsäuresequenz SEQ. ID. NO. 2 unter stringenten Bedingungen hybridisiert oder
H) funktionell äquivalente Fragmente der Sequenzen unter E), F) oder G),
mit der Maßgabe, dass die Nukleinsäure mit der Sequenz SEQ. ID. NO. 2 ausgenommen ist.

Die erfindungsgemäßen Expressionseinheiten umfassen ein oder mehrere der folgenden genetischen Elemente: eine Minus 10 ("-10") Sequenz; eine Minus 35 ("-35") Sequenz; einen Transkriptionsstart, eine Enhancer Region; und eine Operator Region.

Vorzugsweise sind diese genetischen Elemente spezifisch für die Spezies Corynebakterien, speziell für Corynbacterium glutamicum.

Alle vorstehend erwähnten Expressionseinheiten sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Für die Erfindungen in diesem Patent wurden Methoden und Techniken genutzt, die dem Fachmann, der in mikrobiologischen und rekombinanten DNA-Techniken geübt ist, bekannt sind. Methoden und Techniken für das Wachstum von Bakterienzellen, das Einschleusen von isolierten DNA-Molekülen in die Wirtszelle, und die Isolierung, Klonierung und Sequenzierung von isolierten Nukleinsäuremolekülen usw. sind Beispiele für solche Techniken und Methoden. Diese Methoden sind in vielen Standardliteraturstellen beschrieben: Davis et al., Basic Methods In Molecular Biology (1986); J. H. Miller, Experiments in Molecular Genetics, Cold Spring Harbor Laboratory Press, Cold Spring Harbor, New York (1972); J.H. Miller, A Short Course in Bacterial Genetics, Cold Spring Harbor Laboratory Press, Cold Spring Harbor, New York (1992); M. Singer and P. Berg, Genes & Genomes, University Science Books, Mill Valley, Carlifornia (1991); J. Sambrook, E.F. Fritsch and T. Maniatis, Molecular Cloning: A Laboratory Manual, 2nd ed., Cold Spring Harbor Laboratory Press, Cold Spring Harbor, New York (1989); P.B. Kaufmann et al., Handbook of Molcular and Cellular Methods in Biology and Medicine, CRC Press, Boca Raton, Florida (1995); Methods in Plant Molecular Biology and Biotechnology, B.R. Glick and J.E. Thompson, eds., CRC Press, Boca Raton, Florida (1993); and P.F. Smith-Keary, Molecular Genetics of Escherichia coli, The Guilford Press, New York, NY (1989).

Alle Nukleinsäuremoleküle der vorliegenden Erfindung liegen bevorzugt in Form eines isolierten Nukleinsäuremoleküls vor. Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Die Erfindung umfasst weiterhin die zu den konkret beschriebenen Nukleotidsequenzen komplementären Nukleinsäuremoleküle oder einen Abschnitt davon.

Die erfindungsgemäßen Promotoren und/oder Expressionseinheiten lassen sich beispielsweise besonders vorteilhaft in verbesserten Verfahren zur fermentativen Herstellung von biosynthetischen Produkten wie nachstehend beschrieben verwenden.

Die erfindungsgemäßen Promotoren und/oder Expressionseinheiten weisen insbesondere den Vorteil auf, dass sie in Mikroorganismen durch Stress induziert werden. Durch geeignete Steuerung des Fermentationsprozesses lässt sich diese Stress-Induktion gezieit für eine Erhöhung der Trankritptions/Expressionsrate gewünschter Gene steuern. Insbesondere bei der Produktion von L-Lysin wird diese Stressphase sehr früh erreicht, so das hier sehr früh eine erhöhte Transkriptions/Expressionsrate von gewünschten Genen erreicht werden kann.

Die erfindungesgemäßen Nukleinsäuren mit Promotoraktivität können zur Veränderung, also zur Erhöhung oder Reduzierung, oder zur Verursachung der Transkriptionsrate von Genen in Mikroorganismen im Vergleich zum Wildtyp verwendet werden.

Die erfindungsgemäßen Expressionseinheiten können zur Veränderung, also zur Erhöhung oder Reduzierung, oder zur Verursachung der Expressionsrate von Genen in Mikroorganismen im Vergleich zum Wildtyp verwendet werden.

Ferner können die erfindungsgemäßen Nukleinsäuren mit Promotoraktivität und die erfindungesgemäßen Expressionseinheiten zur Regulation und Verstärkung der Bildung von verschiedenen biosynthetischen Produkten, wie beispielsweise Feinchemikalien, Proteinen, inbesondere Aminosäuren, in Mikroorganismen, insbsondere in Corynebacterium species dienen.

Die Erfindung betrifft daher ein Verfahren zur Veränderung oder Verursachung der Transkriptionsrate von Genen in Mikroorganismen im Vergleich zum Wildtyp durch
a) Veränderung der spezifischen Promotoraktivität im Mikroorganismus von endogenen, erfindungsgemäßen Nukleinsäuren mit Promotoraktivität, die die Transkription von endogenen Genen regulieren, im Vergleich zum Wildtyp oder
b) Regulation der Transkription von Genen im Mikroorganismus durch erfindungsgemäße Nukleinsäuren mit Promotoraktivität oder durch Nukleinsäuren mit veränderter spezifischer Promotoraktivität gemäß Ausführungsform a), wobei die Gene in Bezug auf die Nukleinsäuren mit Promotoraktivität heterolog sind.

Gemäß Ausführungsform a) kann die Veränderung oder Verursachung der Transkriptionsrate von Genen in Mikroorganismen im Vergleich zum Wildtyp dadurch erfolgen, dass im Mikroorganismus die spezifische Promotoraktivität verändert, also erhöht oder erniedrigt wird. Dies kann beispielsweise durch gezielte Mutation der erfindungsgemäßen Nukleinsäuresequenz mit Promotoraktivität, also durch gezielte Substitution, Deletion oder Insertion von Nukleotiden erfolgen. Eine erhöhte bzw. erniedrigte Promotoraktivität kann dadurch erreicht werden,dass Nukleotide in der Bindungsstelle des RNA-Polymerase-Holoenzym-Bindungsstellen (dem Fachmann auch als -10-Region und - 35 Region bekannt) ausgetauscht werden. Weiterhin dadurch dass der Abstand der beschriebenen RNA-Polymerase-Holoenzym-Bindungsstellen zueinander durch Deletionen von Nukleotiden oder Insertionen von Nukleotiden verkleinert oder vergrößert werden. Weiterhin dadurch dass Bindungsstellen (dem Fachmann auch als - Operatoren bekannt) für Regulatiosproteine (dem Fachmann bekannt als Repressoren und Aktiviatoren) in räumliche Nähe an die Bindungsstellen des RNA-Polymerase-Holoenzyms gebracht werden, dass diese Regulatoren nach Bindung an eine Promotor-Sequenz die Bindung des und Transkriptionsaktivität des RNA-Polymerase-Holoenzyms abschwächen oder verstärken, oder auch unter einen neuen regulatorischen Einfluss stellen.

Die Nukleinsäuresequenz SEQ. ID. NO. 44 stellt vorzugsweise die ribosomale Bindungsstelle der erfindungsgemäßen Expressionseinheiten, die Sequenzen SEQ. ID. NOs. 42 oder 43 die -10-Region der erfindungsgemäßen Expressionseinheiten dar. Veränderungen der Nukleinsäureaequenz in diesen Regionen führen zu einer Veränderung der spezifischen Expressionsaktivität.

Die Erfindung betrifft daher die Verwendung der Nukleinsäuresequenz SEQ. ID. NO. 44 als ribosomale Bindungsstelle in Expressionseinheiten, die die Expression von Genen in Bakterien der Gattung Corynebacterium oder Brevibacterium ermöglichen.

Weiterhin betrifft die Erfindung die Verwendung der Nukleinsäuresequenzen SEQ. ID. NOs. 42 oder 43 als -10-Region in Expressionseinheiten, die die Expression von Genen in Bakterien der Gattung Corynebacterium oder Brevibacterium ermöglichen.

Insbesondere betrifft die Erfindung eine Expressionseinheit, die die Expression von Genen in Bakterien der Gattung Corynebacterium oder Brevibacterium ermöglicht, enthaltend die Nukleinsäuresequenz SEQ. ID. NO. 44. Vorzugsweise wird dabei die Nukleinsäuresequenz SEQ. ID. NO. 44. als ribosomale Bindungsstelle verwendet.

Weiterhin betrifft die Erfindung eine Expressionseinheit, die die Expression von Genen in Bakterien der Gattung Corynebacterium oder Brevibacterium ermöglicht, enthaltend mindestens eine der Nukleinsäuresequenzen SEQ. ID. NOs. 42 oder 43. Vorzugsweise wird dabei eine der Nukleinsäuresequenzen SEQ. ID. NOs. 42 oder 43 als -10-Region verwendet.

In Bezug auf die "spezifische Promotoraktivität" wird unter Erhöhung oder Reduzierung im Vergleich zum Wildtyp eine Erhöhung oder Reduzierung der spezifischen Aktivität gegenüber der erfindungsgemäßen Nukleinsäure mit Promotoraktivität des Wildtyps, also beispielsweise gegenüber der SEQ. ID. NO. 1 verstanden.

Gemäß Ausführungsform b) kann die Veränderung oder Verursachung der Transkriptionsrate von Genen in Mikroorganismen im Vergleich zum Wildtyp dadurch erfolgen, dass man die Transkription von Genen im Mikroorganismus durch erfindungsgemäße Nukleinsäuren mit Promotoraktivität oder durch Nukleinsäuren mit veränderter spezifischer Promotoraktivität gemäß Ausführungsform a) reguliert, wobei die Gene in Bezug auf die Nukleinsäuren mit Promotoraktivität heterolog sind.

Dies wird bevorzugt dadurch erreicht, dass man
b1) eine oder mehrere erfindungsgemäße Nukleinsäuren mit Promotoraktivität, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, gegebenenfalls mit veränderter spezifischer Promotoraktivität, erfolgt oder
b2) ein oder mehrere Gene in das Genom des Mikrorganismus einbringt, so dass die Transkription eines oder mehrerer der eingebrachten Gene unter der Kontrolle der endogenen, erfindungsgemäßen Nukleinsäuren mit Promotoraktivität, gegebenenfalls mit veränderter spezifischer Promotoraktivität, erfolgt oder
b3) ein oder mehrere Nukleinsäurekonstrukte, enthaltend eine erfindungsgemäße Nukleinsäure mit Promotoraktivität, gegebenenfalls mit veränderter spezifischer Promotoraktivität, und funktionell verknüpft eine oder mehrere, zu transkribierende Nukleinsäuren, in den Mikroorganismus einbringt.

Es ist somit möglich, die Transkriptionsrate eines endogenen Gens des Wildtyps zu verändern, also zu erhöhen oder zu erniedrigen indem man
gemäß Ausführungsform b1) eine oder mehrere erfindungsgemäße Nukleinsäuren mit Promotoraktivität, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, gegebenenfalls mit veränderter spezifischer Promotoraktivität, erfolgt oder
gemäß Ausführungsform b2) ein oder mehrere endogene Gene in das Genom des Mikrorganismus einbringt, so dass die Transkription eines oder mehrerer der eingebrachten, endogenen Gene unter der Kontrolle der endogenen, erfindungsgemäßen Nukleinsäuren mit Promotoraktivität, gegebenenfalls mit veränderter spezifischer Promotoraktivität, erfolgt oder

Gemäß Ausführungsform b3) ein oder mehrere Nukleinsäurekonstrukte, enthaltend eine erfindungsgemäße Nukleinsäure mit Promotoraktivität, gegebenenfalls mit veränderter spezifischer Promotoraktivität, und funktionell verknüpft eine oder mehrere, zu transkribierende endogene Nukleinsäuren, in den Mikroorganismus einbringt.

Ferner ist es somit möglich, die Transkriptionsrate eines exogenen Gens im Vergleich zum Wildtyps zu verursachen, indem man
gemäß Ausführungsform b2) ein oder mehrere exogene Gene in das Genom des Mikrorganismus einbringt, so dass die Transkription eines oder mehrerer der eingebrachten, exogenen Gene unter der Kontrolle der endogenen, erfindungsgemäßen Nukleinsäuren mit Promotoraktivität, gegebenenfalls mit veränderter spezifischer Promotoraktivität, erfolgt oder

Gemäß Ausführungsform b3) ein oder mehrere Nukleinsäurekonstrukte, enthaltend eine erfindungsgemäße Nukleinsäure mit Promotoraktivität, gegebenenfalls mit veränderter spezifischer Promotoraktivität, und funktionell verknüpft eine oder mehrere, zu transkribierende exogene Nukleinsäuren, in den Mikroorganismus einbringt.

Die Insertion von Genen gemäß Ausführungsform b2) kann dabei so erfolgen, dass das Gen in kodierende Bereiche oder nicht-kodierende Bereiche integriert wird. Vorzugsweise erfolgt die Insertion in nicht-kodierende Bereiche.

Die Insertion von Nukleinsäurekonstrukten gemäß Ausführungsform b3) kann dabei chromosomal oder extrachromosomal erfolgen. Vorzugsweise erfolgt die Insertion der Nukleinsäurekonstrukte chromosomal. Eine "chromosomale" Integration ist die Insertion eines exogenen DNA-Fragmentes in das Chromosom einer Wirtszelle. Dieser Begriff wird auch für die homologe Rekombination zwischen einem exogenen DNA-Fragment und der entsprechenden Region auf dem Chromosom der Wirtszelle genutzt.

In Ausführungsform b) werden bevorzugt auch erfindungsgemäße Nukleinsäuren mit veränderter spezifischer Promotoraktivität gemäß Ausführungsform a) eingesetzt. Diese können in Ausführungsform b), wie in Ausführungsform a) beschrieben im Mikroorganismus vorliegen und hergestellt werden oder in isolierter Form in den Mikroorganismus eingebracht werden.

Unter "endogen" werden genetische Informationen, wie beispielsweise Gene, verstanden, die bereits im Wildtypgenom enthalten sind.

Unter "exogen" werden genetische Informationen, wie beispielsweise Gene, verstanden, die im Wildtypgenom nicht enthalten sind.

Unter dem Begriff "Gene" in Bezug auf Regulation der Transkription durch die erfindungsgemäßen Nukleinsäuren mit Promotoraktivität werden vorzugsweise Nukleinsäuren verstanden, die einen zu transkripierenden Bereich, also beispielsweise einen Bereich der die Translation reguliert, einen kodierenden Bereich, sowie gegebenenfalls weitere Regulationselemente, wie beispielsweise einen Terminator, enthalten.

Unter dem Begriff "Gene" in Bezug auf die nachstehend beschriebene Regulation der Expression durch die erfindungsgemäßen Expressionseinheiten werden vorzugsweise Nukleinsäuren verstanden, die einen kodierenden Bereich, sowie gegebenenfalls weitere Regulationselemente, wie beispielsweise einen Terminator, enthalten.

Unter einem "kodierenden Bereich" wird eine Nukleinsäuresequenz verstanden, die ein Protein kodiert.

Unter "heterolog" in Bezug auf Nukleinsären mit Promotoraktivität und Gene wird verstanden, dass die verwendeten Gene im Wildtyp nicht unter Regulation der erfindungsgemäßen Nukleinsäuren mit Promotoraktivität transkribiert werden, sondern das eine neue, im Wildtyp nicht vorkommende funktionelle Verknüpfung entsteht und die funktionelle Kombination aus erfindungsgemäßer Nukleinsäure mit Promotoraktivität und spezifisches Gen im Wildtyp nicht vorkommt.

Unter "heterolog" in Bezug auf Expressionseinheiten und Gene wird verstanden, dass die verwendeten Gene im Wildtyp nicht unter Regulation der erfindungsgemäßen Expressionseinheiten exprimiert werden, sondern das eine neue, im Wildtyp nicht vorkommende funktionelle Verknüpfung entsteht und die funktionelle Kombination aus erfindungsgemäßer Expressionseinheit und spezifisches Gen im Wildtyp nicht vorkommt.

Die Erfindung betrifft ferner in einer bevorzugten Ausführungsform ein Verfahren zur Erhöhung oder Verursachung der Transkriptionsrate von Genen in Mikroorganismen im Vergleich zum Wildtyp indem man
ah) die spezifische Promotoraktivität im Mikroorganismus von erfindungsgemäßen endogenen Nukleinsäuren mit Promotoraktivität , die die Transkription von endogenen Genen regulieren, im Vergleich zum Wildtyp erhöht oder
bh) die Transkription von Genen im Mikroorganismus durch erfindungsgemäße Nukleinsäuren mit Promotoraktivität oder durch Nukleinsäuren mit erhöhter spezifischer Promotoraktivität gemäß Ausführungsform a) reguliert, wobei die Gene in Bezug auf die Nukleinsäuren mit Promotoraktivität heterolog sind.

Vorzugsweise wird die Regulation der Transkription von Genen im Mikroorganismus durch erfindungsgemäße Nukleinsäuren mit Promotoraktivität oder durch erfindungsgemäße Nukleinsäuren mit erhöhter spezifischer Promotoraktivität gemäß Ausführungsform ah) dadurch erreicht wird, dass man
bh1) eine oder mehrere erfindungsgemäße Nukleinsäuren mit Promotoraktivität, gegebenenfalls mit erhöhter spezifischer Promotoraktivität, in das Genom des Mikroorganismus einbringt, so dass die Transkription eines oder mehrerer endogenen Gene unter der Kontrolle der eingebrachten erfindungsgemäßen Nukleinsäure mit Promotoraktivität, gegebenenfalls mit erhöhter spezifischer Promotoraktivität, erfolgt oder
bh2) ein oder mehrere Gene in das Genom des Mikrorganismus einbringt, so dass die Transkription eines oder mehrerer der eingebrachten Gene unter der Kontrolle der erfindungsgemäßen, endogenen Nukleinsäuren mit Promotoraktivität, gegebenenfalls mit erhöhter spezifischer Promotoraktivität, erfolgt oder
bh3) ein oder mehrere Nukleinsäurekonstrukte, enthaltend eine erfindungsgemäße Nukleinsäure mit Promotoraktivität, gegebenenfalls mit erhöhter spezifischer Promotoraktivität, und funktionell verknüpft eine oder mehrere, zu transkribierende Nukleinsäuren, in den Mikroorganismus einbringt.

Die Erfindung betrifft ferner in einer bevorzugten Ausführungsform ein Verfahren zur Reduzierung der Transkriptionsrate von Genen in Mikroorganismen im Vergleich zum Wildtyp, indem man
ar) die spezifische Promotoraktivität im Mikroorganismus von endogenen erfindungsgemäßen Nukleinsäuren mit Promotoraktivität, die die Transkription der endogenen Gene regulieren, im Vergleich zum Wildtyp reduziert oder
br) Nukleinsäuren mit reduzierter spezifischer Promotoraktivität gemäß Ausführungsform a) in das Genom des Mikroorganismus einbringt, so dass die Transkription endogene Gene unter der Kontrolle der eingebrachten Nukleinsäure mit reduzierter Promotoraktivität erfolgt.

Die Erfindung betrifft ferner ein Verfahren zur Veränderung oder Verursachung der Expressionsrate eines Gens in Mikroorganismen im Vergleich zum Wildtyp durch
c) Veränderung der spezifischen Expressionsaktivität im Mikroorganismus von erfindungsgemäßen, endogenen Expressionseinheiten, die die Expression der endogenen Gene regulieren, im Vergleich zum Wildtyp oder
d) Regulation der Expression von Genen im Mikroorganismus durch erfindungsgemäße Expressionseinheiten oder durch erfindungsgemäße Expressionseinheiten mit veränderter spezifischer Expressionsaktivität gemäß Ausführungsform c), wobei die Gene im Bezug auf die Expressionseinheiten heterolog sind.

Gemäß Ausführungsform c) kann die Veränderung oder Verursachung der Expressionsrate von Genen in Mikroorganismen im Vergleich zum Wildtyp dadurch erfolgen, dass im Mikroorganismus die spezifische Expressionsaktivität verändert, also erhöht oder erniedrigt wird. Dies kann beispielsweise durch gezielte Mutation der erfindungsgemäßen Nukleinsäuresequenz mit Promotoraktivität, also durch gezielte Substitution, Deletion oder Insertion von Nukleotiden erfolgen. Beispielsweise führt die Verlängerung des Abstandes zwischen Shine-Dalgarno-Sequenz und dem translationellen Startcodon in der Regel zu einer Änderung, einer Verkleinerung oder aber auch einer Verstärkung der spezifischen Expressionsaktivität. Eine Veränderung der der spezifischen Expressionsaktivität kann auch dadurch erreicht werden, dass die Sequenz der Shine-Dalgarno-Region (Ribosomale Bindungsstelle) in seinem Abstand zum translationellen Startcodon durch Deletionen oder Insertionen von Nukleotiden entweder verkürzt oder verlängert wird. Aber auch dadurch dass die Sequenz der Shine-Dalgarno-Region so verändert wird, dass die Homologie zu komplementären 3' Seite 16S rRNA entweder verstärkt oder aber auch verringert wird.

In Bezug auf die "spezifische Expressionsaktivität" wird unter Erhöhung oder Reduzierung im Vergleich zum Wildtyp eine Erhöhung oder Reduzierung der spezifischen Aktivität gegenüber der erfindungsgemäßen Expressionseinheit des Wildtyps, also beispielsweise gegenüber der SEQ. ID. NO. 2 verstanden.

Gemäß Ausführungsform d) kann die Veränderung oder Verursachung der Expressionsrate von Genen in Mikroorganismen im Vergleich zum Wildtyp dadurch erfolgen, dass man die Expression von Genen im Mikroorganismus durch erfindungsgemäße Expressionseinheiten oder durch erfindungsgemäße Expressionseinheiten mit veränderter spezifischer Expressionsaktivität gemäß Ausführungsform c) reguliert, wobei die Gene in Bezug auf die Expressionseinheiten heterolog sind.

Dies wird bevorzugt dadurch erreicht, dass man
d1) eine oder mehrere erfindungsgemäße Expressionseinheiten, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten erfolgt oder
d2) ein oder mehrere Gene in das Genom des Mikrorganismus einbringt, so dass die Expression eines oder mehrerer der eingebrachten Gene unter der Kontrolle der erfindungsgemäßen, endogenen Expressionseinheiten, gegebenenfalls mit veränderter spezifischer Expressionsaktivität, erfolgt oder
d3) ein oder mehrere Nukleinsäurekonstrukte, enthaltend eine erfindungsgemäße Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Es ist somit möglich, die Expressionsrate eines endogenen Gens des Wildtyps zu verändern, also zu erhöhen oder zu erniedrigen indem man
gemäß Ausführungsform d1) eine oder mehrere erfindungsgemäße Expressionseinheiten, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten erfolgt oder
gemäß Ausführungsform d2) ein oder mehrere Gene in das Genom des Mikrorganismus einbringt, so dass die Expression eines oder mehrerer der eingebrachten Gene unter der Kontrolle der erfindungsgemäßen, endogenen Expressionseinheiten, gegebenenfalls mit veränderter spezifischer Expressionsaktivität, erfolgt oder
gemäß Ausführungsform d3) ein oder mehrere Nukleinsäurekonstrukte, enthaltend eine erfindungsgemäße Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Ferner ist es somit möglich, die Expressionssrate eines exogenen Gens im Vergleich zum Wildtyps zu verursachen, indem man
gemäß Ausführungsform d2) ein oder mehrere exogene Gene in das Genom des Mikrorganismus einbringt, so dass die Expression eines oder mehrerer der eingebrachten Gene unter der Kontrolle der erfindungsgemäßen, endogenen Expressionseinheiten, gegebenenfalls mit veränderter spezifischer Expressionsaktivität, erfolgt oder
gemäß Ausführungsform d3) ein oder mehrere Nukleinsäurekonstrukte, enthaltend eine erfindungsgemäße Expressionseinheit, gegebenenfalls mit veränderter spezifischer Expressionsaktivität, und funktionell verknüpft eine oder mehrere, zu exprimierende, exogene Nukleinsäuren, in den Mikroorganismus einbringt.

Die Insertion von Genen gemäß Ausführungsform d2) kann dabei so erfolgen, dass das Gen in kodierende Bereiche oder nicht-kodierende Bereiche integriert wird. Vorzugsweise erfolgt die Insertion in nicht-kodierende Bereiche.

Die Insertion von Nukleinsäurekonstrukten gemäß Ausführungsform d3) kann dabei chromosomal oder extrachromosomal erfolgen. Vorzugsweise erfolgt die Insertion der Nukleinsäurekonstrukte chromosomal.

Die Nukleinsäurekonstrukte werden im folgenden auch als Expressionskasetten bezeichnet.

In Ausführungsform d) werden bevorzugt auch erfindungsgemäße Expressionseinheiten mit veränderter spezifischer Expressionsaktivität gemäß Ausführungsform c) eingesetzt. Diese können in Ausführungsform d), wie in Ausführungsform d) beschrieben im Mikroorganismus vorliegen und hergestellt werden oder in isolierter Form in den Mikroorganismus eingebracht werden.

Die Erfindung betrifft ferner in einer bevorzugten Ausführungsform ein Verfahren zur Erhöhung oder Verursachung der Expressionsrate eines Gens in Mikroorganismen im Vergleich zum Wildtyp indem man
ch) die spezifische Expressionsaktivität im Mikroorganismus von erfindungsgemäßen, endogenen Expressionseinheiten, die die Expression der endogenen Gene regulieren, im Vergleich zum Wildtyp erhöht oder
dh) die Expression von Genen im Mikroorganismus durch erfindungsgemäße Expressionseinheiten oder durch Expressionseinheiten mit erhöhter spezifischer Expressionsaktivität gemäß Ausführungsform c) reguliert, wobei die Gene im Bezug auf die Expressionseinheiten heterolog sind.

Vorzugsweise wird die Regulation der Expression von Genen im Mikroorganismus durch erfindungsgemäße Expressionseinheiten oder durch Expressionseinheiten mit erhöhter spezifischer Expressionsaktivität gemäß Ausführungsform c) dadurch erreicht, dass man
dh1) eine oder mehrere erfindungsgemäße Expressionseinheiten, gegebenenfalls mit erhöhter spezifischer Expressionsaktivität, in das Genom des Mikroorganismus einbringt, so dass die Expression eines oder mehrerer endogenen Gene unter der Kontrolle der eingebrachten Expressionseinheiten, gegebenenfalls mit erhöhter spezifischer Expressionsaktivität, erfolgt oder
dh2) ein oder mehrere Gene in das Genom des Mikrorganismus einbringt, so dass die Expression eines oder mehrerer der eingebrachten Gene unter der Kontrolle der erfindungsgemäßen, endogenen Expressionseinheiten, gegebenenfalls mit erhöhter spezifischer Expressionsaktivität, erfolgt oder
dh3) ein oder mehrere Nukleinsäurekonstrukte, enthaltend eine erfindungsgemäße Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

Die Erfindung betrifft ferner ein Verfahren zur Reduzierung der Expressionsrate von Genen in Mikroorganismen im Vergleich zum Wildtyp, indem man
cr) die spezifische Expressionsaktivität im Mikroorganismus von endogenen, erfindungsgemäßen Expressionseinheiten, die die Expression der endogenen Gene regulieren, im Vergleich zum Wildtyp reduziert oder
dr) Expressionseinheiten mit reduzierter spezifischer Expressionsaktivität gemäß Ausführungsform cr) in das Genom des Mikroorganismus einbringt, so dass die Expression endogener Gene unter der Kontrolle der eingebrachten Expressionseinheiten mit reduzierter Expressionsaktivität erfolgt.

In einer bevorzugten Ausführungsform der vorstehend beschriebenen erfindungsgemäßen Verfahren zur Veränderung oder Verursachung der Transkriptionsrate und/oder Expressionsrate von Genen in Mikroorganismen sind die Gene ausgewählt aus der Gruppe Nukleinsäuren kodierend ein Protein aus den Biosyntheseweg von Feinchemikalien, wobei die Gene gegebenenfalls weitere Regulationselemente enthalten können.

In einer besonders bevorzugten Ausführungsform der vorstehend beschriebenen erfindungsgemäßen Verfahren zur Veränderung oder Verursachung der Transkriptionsrate und/oder Expressionsrate von Genen in Mikroorganismen sind die Gene ausgewählt aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

In einer besondere bevorzugten Ausführungsform sind die Proteine aus dem Biosyntheseweg von Aminosäuren ausgewählt aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

Bevorzugte Proteine und Nukleinsäuren kodierend diese Proteine der vorstehend beschriebenen Proteine aus dem Biosyntheseweg von Aminosäuren sind Proteinsequenzen bzw. Nukleinsäuresequenzen mikrobiellen Ursprungs, vorzusgweise aus Bakterien der Gattung Corynebacterium oder Brevibacterium, bevorzugt aus coryneformen Bakterien, besonders bevorzugt aus Corynebakterium glutamicum.

Beispiele für besonders bevorzugte Proteinsequenzen und die entsprechenden Nukleinsäuresequenzen kodierend diese Proteine aus dem Biosyntheseweg von Aminosäuren, deren Bezugsdokument, sowie deren Bezeichnung im Bezugsdokument sind in Tabelle 1 aufgelistet:

**Tabelle 1**

| Protein | Nukleinsäure kodierend Protein | Bezugsdokument | SEQ. ID. NO. im Bezugsdokument |
|---|---|---|---|
| Aspartatkinase | ask oder lysC | EP1108790 | DNA: 281 |
| | | | Protein: 3781 |
| Aspartat-Semialdehyd- | asd | EP1108790 | DNA: 331 |
| Dehydrogenase | | | Protein: 3831 |
| Dihydrodipicolinate- | dapA | WO 0100843 | DNA: 55 |
| Synthetase | | | Protein: 56 |
| Dihydrodipicolinate- | dapB | WO 0100843 | DNA: 35 |
| Reduktase | | | Protein: 36 |
| Meso-Diaminopimelat- | ddh | EP1108790 | DNA : 3494 |
| D-Dehydrogenase | | | Protein : 6944 |
| Diaminopicolinat- | IysA | EP1108790 | DNA: 3451 |
| Decarboxylase | | | Prot.:6951 |
| Lysin-Exporter | lysE | EP1108790 | DNA: 3455 |
| | | | Prot.: 6955 |
| Arginyl-t-RNA Synthetase | argS | EP1108790 | DNA: 3450 |
| | | | Prot.: 6950 |
| Glucose-6-Phosphat- | zwf | WO 0100844 | DNA: 243 |
| Dehydrognease | | | Prot.: 244 |
| Glycerinaldehyd-3- | gap | WO 0100844 | DNA: 187 |
| Phosphat-Dehydrogenase | | | Prot.: 188 |
| 3-Phosphoglycerat- | pgk | WO 0100844 | DNA: 69 |
| kinase | | | Prot.: 70 |
| Pyruvat-Carboxylase | pycA | EP1108790 | DNA:765 |
| | | | Prot.: 4265 |
| Triosephosphat- | tpi | WO 0100844 | DNA: 61 |
| Isomerase | | | Prot.: 62 |
| Biotin-Ligase | birA | EP1108790 | DNA: 786 |
| | | | Prot.: 4286 |
| PEP-Carboxylase | pck | EP1108790 | DNA: 3470 |
| | | | Prot.: 6970 |
| Homoserin Kinase | thrB | WO 0100843 | DNA: 173 |
| | | | Prot.: 174 |
| Threonin Synthase | thrC | WO 0100843 | DNA: 175 |
| | | | Prot.: 176 |
| Threonin Export Carrier | thrE | WO 0251231 | DNA: 41 |
| | | | Prot.: 42 |
| Threonin Efflux Protein | RXA2390 | WO 0100843 | DNA: 7 |
| | | | Prot.: 8 |
| Threonin Dehydratase | ilvA | EP 1108790 | DNA: 2328 |
| | | | Prot.: 5828 |
| Homoserin-O- | metA | EP 1108790 | DNA:727 |
| Acetyltransferase | | | Prot: 4227 |
| Cystathionin-gamma- | metB | EP 1108790 | DNA:3491 |
| synthase | | | Prot: 6991 |
| Cystathionin-beta- | metC | EP 1108790 | DNA:2535 |
| Lyase | | | Prot: 6035 |
| Coenzym B12- | metH | EP 1108790 | DNA:1663 |
| abhängige Methionin-Synthase, - | | | Prot: 5163 |
| O-Acetylhomoserin- | metY | EP 1108790 | DNA:726 |
| Sulfhydrylase | | | Prot: 4226 |
| Methylentetrahydro- | metF | EP 1108790 | DNA:2379 |
| folat-Reduktase | | | Prot: 5879 |
| D-3-Phosphoglycerat-Dehydrogenase | serA | EP 1108790 | DNA:1415 |
| | | | Prot: 4915 |
| Phosphoserin- | serB | WO 0100843 | DNA: 153 |
| Phosphatase 1 | | | Prot.:154 |
| Phosphoserin- | serB | EP 1108790 | DNA: 467 |
| Phosphatase 2 | | | Prot: 3967 |
| Phosphoserin- | serB | EP 1108790 | DNA: 334 |
| Phosphatase 3 | | | Prot.: 3834 |
| Phosphoserin- | serC | WO 0100843 | DNA: 151 |
| Aminotransferase | | | Prot.: 152 |
| Serin Acetyl- | cysE | WO 0100843 | DNA: 243 |
| Transferase | | | Prot.: 244 |
| Cystein-Synthase I | cysK | EP 1108790 | DNA: 2817 |
| | | | Prot.: 6317 |
| Cystein Synthase II | CysM | EP 1108790 | DNA: 2338 |
| | | | Prot.: 5838 |
| Homoserin- | hom | EP 1108790 | DNA: 3452 |
| Dehydrogenase | | | Prot.: 6952 |
| Coenzym B12- | metE | WO 0100843 | DNA:755 |
| unabhängige Me- thionin-Synthase | | | Prot.: 756 |
| Serin- | glyA | WO 0100843 | DNA: 143 |
| Hydroxymethyltransferase | | | Prot.: 144 |
| Protein in Sulfat- | RXA247 | EP 1108790 | DNA: 3089 |
| Reduktion | | | Prot.: 6589 |
| Protein in Sulfat- | RXA248 | EP 1108790 | DNA: 3090 |
| Reduktion | | | Prot.: 6590 |
| Sulfatadenyltransferase | CysN | EP 1108790 | DNA: 3092 |
| Untereinheit 1 | | | Prot.: 6592 |
| Sulfatadenyltransferase | CysD | EP 1108790 | DNA: 3093 |
| Untereinheit 2 | | | Prot.: 6593 |
| Phosphoadenosin | CysH | WO 02729029 | DNA: 7 |
| Phosphosulfat Reduktase | | | Prot.: 8 |
| Ferredoxin-Sulfit- | RXA073 | WO 0100842 | DNA: 329 |
| Reduktase | | | Prot.: 330 |
| Ferredoxin NADP Re- | RXA076 | WO 0100843 | DNA: 79 |
| duktase | | | Prot.: 80 |
| Transkriptioneller Re- | IuxR | WO 0100842 | DNA: 297 |
| gulator LuxR | | | Protein: 298 |
| Transkriptioneller Re- | IysR1 | EP 1108790 | DNA: 676 |
| gulator LysR1 | | | Protein: 4176 |
| Transkriptioneller Re- | lysR2 | EP 1108790 | DNA: 3228 |
| gulator LysR2 | | | Protein: 6728 |
| Transkriptioneller Re- | lysR3 | EP 1108790 | DNA: 2200 |
| gulator LysR3 | | | Protein: 5700 |
| Malat-Quinon- | mqo | WO 0100844 | DNA: 569 |
| Oxodoreduktase | | | Protein: 570 |
| Transketolase | RXA2739 | EP 1108790 | DNA: 1740 |
| | | | Prot: 5240 |
| Transaldolase | RXA2738 | WO 0100844 | DNA: 245 |
| | | | Prot: 246 |
| OPCA | opcA | WO 0100804 | DNA: 79 |
| | | | Prot: 80 |
| 1-Phosphofructokinase | pfk1 | WO0100844 | DNA: 55 |
| 1 | | | Protein: 56 |
| 1-Phosphofructokinase | pfk2 | WO0100844 | DNA: 57 |
| 2 | | | Protein: 58 |
| 6-Phosphofructokinase | 6-pfk1 | EP 1108790 | DNA: 1383 |
| 1 | | | Protein: 4883 |
| 6-Phosphofructokinase | 6-pfk2 | DE 10112992 | DNA: 1 |
| 2 | | | Protein: 2 |
| Fructose-1,6- | fbr1 | EP1108790 | DNA:1136 |
| bisphosphatase 1 | | | Protein: 4636 |
| Pyruvat Oxidase | poxB | WO 0100844 | DNA : 85 |
| | | | Protein: 86 |
| RXA00655-Regulator | RXA655 | US2003162267 | DNA: 1 |
| | | .2 | Prot.: 2 |
| RXN02910-Regulator | RXN2910 | US2003162267 | DNA: 5 |
| | | .2 | Prot.: 6 |
| 6- | RXA2735 | WO 0100844 | DNA: 1 |
| phosphogluconolactonase | | | Prot.: 2 |

Ein weiteres Beispiel für eine besonders bevorzugte Proteinsequenz und die entsprechenden Nukleinsäuresequenz kodierend dieses Protein aus dem Biosyntheseweg von Aminosäuren, ist die Sequenz der Fructose-1,6-bisphosphatase 2, oder auch fbr2 genannt, (SEQ. ID. NO. 41) und die entsprechenden Nukleinsäuresequenz kodierend eine Fructose-1,6-bisphosphatase 2 (SEQ. ID. NO. 40).

Ein weiteres Beispiel für eine besonders bevorzugte Proteinsequenz und die entsprechenden Nukleinsäuresequenz kodierend dieses Protein aus dem Biosyntheseweg von Aminosäuren, ist die Sequenz des Proteins in Sulfat-Reduktion, oder auch RXA077 genannt, (SEQ. ID. NO. 4) und die entsprechenden Nukleinsäuresequenz kodierend ein Protein in Sulfat-Reduktion (SEQ. ID. NO. 3)

Weitere besonders bevorzugte Proteinsequenzen aus dem Biosyntheseweg von Aminosäuren, weisen jeweils die in Tabelle 1 für dieses Protein angegebene Aminosäuresequenz auf, wobei das jeweilige Protein jeweils an mindestens einer der in Tabelle 2/Spalte2 für diese Aminosäuresequenz angegebenen Aminosäurepositionen eine andere proteinogene Aminosäure aufweist als die jeweilige in Tabelle2/Spalte3 in der gleichen Zeile angegebene Aminosäure. In einer weiter bevorzugten Ausführungsform, weisen die Proteine an mindestens einer der in Tabelle 2/Spalte 2 für die Aminosäuresequenz angegebenenen Aminosäureposition die in Tabelle2/Spalte4 in der gleichen Zeile angegebene Aminosäure auf. Es handelt sich bei den in Tabelle 2 angegebenen Proteine um mutierte Proteine des Biosyntheseweges von Aminosäuren, die besonders vorteilhafte Eigenschaften aufweisen und sich deshalb insbesondere zur Expression der entsprechenden Nukleinsäuren durch den erfinungsgemäßen Promotor und zur Herstellung von Aminosäuren eignen. Beispielsweise führt die Mutation T311 I zu einem Ausschalten derfeedback-Inhibierung von ask.

Die entsprechenden Nukleinsäuren, die ein vorstehend beschriebenes mutiertes Protein aus Tabelle 2 kodieren, lassen sich durch übliche verfahren herstellen.

Als Ausgangspunkt zur Herstellung der Nukleinsäuresequenzen kodierend ein mutiertes Protein eignet sich beispielsweise das Genom eines Corynebacterium glutamicum-Stammes, der von der American Type Culture Collection unter der Bezeichnung ATCC 13032 erhältlich ist oder die in Tabelle 1 in Bezug genommenen Nukleinsäuresequenzen. Für die Rückübersetzung der Aminosäuresequenz der mutierten Proteine in die Nukleinsäuresequenzen kodierend diese Proteine ist es vorteilhaft, die codon usage desjenigen Organismus zu verwenden, in den die Nukleinsäuresequenz eingebracht werden soll oder in der die Nukleinsäuresequenz vorliegt. Beispielsweise ist es vorteihaft für Corynebakterium glutamicum die codon usage von Corynebakterium glutamicum zu verwenden. Die codon usage des jeweiligen Organismus lässt sich in an sich bekannter Weise aus Datenbanken oder Patentanmeldungen ermitteln, die zumindest ein Protein und ein Gen, das dieses Protein kodiert, aus dem gewünschten Organismus beschreiben.

Die Angaben in Tabelle 2 sind folgendermassen zu verstehen:
In Spalte 1"Identifikation" wird eine eindeutige Bezeichnung für jede Sequenz in Bezug auf Tabelle 1 aufgeführt.
In Spalte 2 "AS-POS" bezieht sich die jeweilige Zahl auf die Aminosäureposition der entsprechenden Polypeptidsequenz aus Tabelle 1. Eine "26" in der Spalte "AS-POS" bedeutet demzufolge die Aminosäureposition 26 der entsprechend angegebenen Polypeptidsequenz. Die Zählung der Position beginnt N-Terminal bei +1.
In Spalte 3 "AS-Wildtyp" bezeichnet der jeweilige Buchstabe die Aminosäure - dargestellt im Ein-Buchstaben-Code- an der in Spalte 2 angegebenen Position beim entsprechenden Wildtyp-Stamm der Sequenz aus Tabelle 1.
In Spalte 4 "AS-Mutante" bezeichnet der jeweilige Buchstabe die Aminosäure - dargestellt im Ein-Buchstaben-Code- an der in Spalte 2 angegebenen Position beim entsprechenden Mutanten-Stamm.
In Spalte 5 "Funktion" wird die physiologische Funktion der entsprechenden Polypeptidsequenz aufgeführt.

Für ein mutiertes Protein mit einer bestimmten Funktion (Spalte 5) und einer bestimmten Ausgangsaminosäuresequenz (Tabelle 1) werden in den Spalten 2,3 und 4 mindestens eine Mutation, bei einigen Sequenzen auch mehrere Mutationen beschrieben. Diese mehreren Mutationen beziehen sich immer auf die jeweils obenstehende, nächstliegendste Ausgangsaminosäuresequenz (Tabelle 1). Unter dem Begriff "mindestens eine der Aminsäurepositionen" einer bestimmten Aminosäuresequenz wird vorzugsweise mindestens eine der für diese Aminosäuresequenz in Spalte 2, 3 und 4 beschriebenen Mutationen verstanden.

### Ein-Buchstaben-Code der proteinogenen Aminosäuren:

A Alanin
C Cystein
D Aspartat
E Glutamat
FPhenylalanin
G Glycin
H His
I Isoleucin
K Lysin
L Leucin
M Methionin
N Asparagin
P Prolin
Q Glutamin
R Arginin
S Serin
TThreonin
V Valin
W Tryptophan
Y Tyrosin

**Tabelle 2**

| Spalte 1 | Spalte 2 | Spalte 3 | Spalte 4 | Spalte 5 |
|---|---|---|---|---|
| Identifikation | AS Position | AS Wildtyp | AS Mutante | Funktion |
| ask | 317 | S | A | Aspartatkinase |
| | 311 | T | I | |
| | 279 | A | T | |
| asd | 66 | D | G | Aspartat-Semialdehyd-Dehydrogenase |
| | 234 | R | H | |
| | 272 | D | E | |
| | 285 | K | E | |
| | 20 | L | F | |
| dapA | 2 | S | A | Dihydrodipicolinat Synthetase |
| | 84 | K | N | |
| | 85 | L | V | |
| dapB | 91 | D | A | Dihydrodipicolinat-Reduktase |
| | 83 | D | N | |
| ddh | 174 | D | E | Meso-Diaminopimelat-D-Dehydrogenase |
| | 235 | F | L | |
| | 237 | S | A | |
| lysA | 265 | A | D | Diaminopicolinat-Decarboxylase |
| | 320 | D | N | |
| | 332 | I | V | |
| argS | 355 | G | D | Arginyl-t-RNA-Synthetase |
| | 156 | A | S | |
| | 513 | V | A | |
| | 540 | H | R | |
| zwf | 8 | S | T | Glucose-6-Phosphat-Dehydrogenase |
| | 150 | T | A | |
| | 321 | G | S | |
| gap | 264 | G | S | Glycerinaldehyd-3-Phosphat-Dehydrogenase |
| pycA | 7 | S | L | Pyruvat-Carboxylase |
| | 153 | E | D | |
| | 182 | A | S | |
| | 206 | A | S | |
| | 227 | H | R | |
| | 455 | A | G | |
| | 458 | P | S | |
| | 639 | S | T | |
| | 1008 | R | H | |
| | 1059 | S | P | |
| | 1120 | D | E | |
| pck | 162 | H | Y | PEP-Carboxylase |
| | 241 | G | D | |
| | 829 | T | R | |
| thrB | 103 | S | A | Homoserin Kinase |
| | 190 | T | A | |
| | 133 | A | V | |
| | 138 | P | S | |
| thrC | 69 | G | R | Threonin Synthase |
| | 478 | T | I | |
| RXA330 | 85 | I | M | Threonin-Efflux Protein |
| | 161 | F | I | |
| | 195 | G | D | |
| hom | 104 | V | I | Homoserin-Deydrogenase |
| | 116 | T | I | |
| | 148 | G | A | |
| | 59 | V | A | |
| | 270 | T | S | |
| | 345 | R | P | |
| | 268 | K | N | |
| | 61 | D | H | |
| | 72 | E | Q | |
| lysR1 | 80 | R | H | transkriptioneller Regulator LysR1 |
| IysR3 | 142 | R | W | transkriptioneller Regulator LysR3 |
| | 179 | A | T | |
| RXA2739 | 75 | N | D | Transketolase |
| | 329 | A | T | |
| | 332 | A | T | |
| | 556 | V | I | |
| RXA2738 | 242 | K | M | Transaldolase |
| opcA | 107 | Y | H | OpcA |
| | 219 | K | N | |
| | 233 | P | S | |
| | 261 | Y | H | |
| | 312 | S | F | |
| | 65 | G | R | Aspartat-1-Decarboxylase |
| | 33 | G | S | 6- Phosphogluconolactonase |

In den vorstehend beschriebenen erfindungsgemäßen Verfahren zur Veränderung oder Verursachung der Transkriptionsrate und/oder Expressionsrate von Genen in Mikroorganismen sowie den nachstehend beschriebenen Verfahren zur Herstellung von genetisch veränderten Mikroorganismen, den nachstehend beschriebenen genetisch veränderten Mikroorganoismen und den nachstehend beschriebenen Verfahren zur Herstellung von biosynthetischen Produkten erfolgt das Einbringen der erfindungsgemäßen Nukleinsäuren mit Promotoraktivität, der erfindungsgemäßen Expressionseinheiten, der vorstehend beschriebenen Gene und der vorstehend beschriebnen Nukleinsäurekonstrukte oder Expressionskasetten in den Mikroorganismus, insbeondere in corynefome Bakterien, vorzugsweise durch die SacB-Methode.

Die SacB-Methode ist dem Fachmann bekannt und beispielsweise in Schäfer A, Tauch A, Jäger W, Kalinowski J, Thierbach G, Pühler A.; Small mobilizable multi-purpose cloning vectors derived from the Escherichia coli plasmids pK18 and pK19: selection of defined deletions in the chromosome of Corynebacterium glutamicum, Gene. 1994 Jul 22;145(1):69-73 und Blomfield IC, Vaughn V, Rest RF, Eisenstein BI.; Allelic exchange in Escherichia coli using the Bacillus subtilis sacB gene and a temperature-sensitive pSC101 replicon; Mol Microbiol. 1991 Jun;S(6):1447-57 beschrieben.

In einer bevorzugten Ausführungsform der vorstehend beschriebenen erfindungsgemäßen Verfahren erfolgt die Veränderung oder Verursachung der Transkriptionsrate und/oder Expressionsrate von Genen in Mikroorganismen durch Einbringen von erfindungsgemäßen Nukleinsäuren mit Promotoraktivität bzw. erfindungsgemäßen Expressionseinheiten in den Mikroorganismus.

In einer weiteren bevorzugten Ausführungsform der vorstehend beschriebenen erfindungsgemäßen Verfahren erfolgt die Veränderung oder Verursachung der Transkriptionsrate und/oder Expressionsrate von Genen in Mikroorganismen durch Einbringen der vorstehend beschriebenen Nukleinsäurekonstrukte oder Expressionskasetten in den Mikroorganismus.

Die Erfindung betrifft daher ferner eine Expressionskassette, umfassend
mindestens eine erfindungsgemäße Expressionseinheit
mindestens eine weitere, zu exprimierende Nukleinsäuresequenz, also ein zu exprimierendes Gen und
gegebenenfalls weitere genetische Kontrollelemente, wie beispielsweise einen Terminator,
wobei mindestens eine Expressionseinheit und eine weitere, zu exprimierende, Nukleinsäuresequenz funktionell miteinander verknüpft sind und die weitere, zu exprimierende, Nukleinsäuresequenz in Bezug auf die Expressionseinheit heterolog ist.

Vorzugsweise ist die zu exprimierende Nukleinsäuresequenz mindestens eine Nukleinsäuren kodierend ein Protein aus den Biosyntheseweg von Feinchemikalien.

Besonders bevorzugt ist die zu exprimierende Nukleinsäuresequenz ausgewählt aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen.

Bevorzugte Proteine aus dem Biosyntheseweg von Aminosäuren sind vorstehend und deren Beispiele in Tabelle 1 und 2 beschrieben.

In den erfindungsgemäßen Expressionskassetten ist die physikalische Lage der Expressionseinheit relativ zum zu exprimierenden Gen so gewählt, daß die Expressioneinheit die Transkription und vorzugsweise auch die Translation des zu exprimierenden Gens reguliert und damit die Bildung eines oder mehrerer Proteine ermöglicht. Die "Bildung ermöglichen" beinhaltet dabei die konstitutive Steigerung der Bildung, Abschwächung bzw. Blockierung der Bildung unter spezifischen Bedingungen und oder die Steigerung der Bildung unter spezifischen Bedingungen. Die "Bedingungen" umfassen dabei: (1) Zugabe einer Komponente zum Kulturmedium, (2) Entfernen einer Komponente vom Kulturmedium, (3) Austausch einer Komponente im Kulturmedium durch eine zweite Komponente, (4) Erhöhung der Temperatur des Kulturmediums, (5) Erniedrigung der Temperatur des Kulturmediums, und (6) Regulierung der atmosphärischen Bedingungen, wie z.B. die Sauerstoff- oder Stickstoffkonzentration, in der das Kulturmedium gehalten wird.

Die Erfindung betrifft ferner einen Expressionsvektor enthaltend eine vorstehend beschriebene, erfindungsgemäße Expressionskassette.

Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Plasmide eignen sich solche besonders bevorzugt, die in coryneformen Bakterien repliziert werden. Zahlreiche bekannte Plasmidvektoren, wie z. B. pZ1 (Menkel et al., Applied and Environmental Microbiology (1989) 64: 549-554), pEKEx1 (Eikmanns et al., Gene 102: 93-98 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie z. B. pCLiK5MCS, oder solche, die auf pCG4 (US-A 4,489,160) oder pNG2 (Serwold-Davis et al., FEMS Microbiology Letters 66, 119-124 (1990)) oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Remscheid et al. (Applied and Environmental Microbiology 60,126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Sirnon et al., Bio/Technology 1,784-791 (1983)), pK18mob oder pK19mob (Schäfer et al., Gene 145,69-73 (1994)), Bernard et al., Journal of Molecular Biology, 234: 534-541 (1993)), pEM1 (Schrumpf et al. 1991, Journal of Bacteriology 173: 4510-4516) oder pBGS8 (Spratt et al.,1986, Gene 41: 337-342) in Frage. Der Plasmidvektor, der das zu amplifizierende Gen enthält, wird anschließend durch Transformation in den gewünschten Stamm von C. glutamicum überführt. Methoden zur Transformation sind beispielsweise bei Thierbach et al. (Applied Microbiology and Biotechnology 29, 356-362 (1988)), Dunican und Shivnan (Biotechnology 7, 1067-1070 (1989)) und Tauch et al. (FEMS Microbiological Letters 123,343-347 (1994)) beschrieben.

Die Erfindung betrifft ferner einen genetisch verändertern Mikroorganismus, wobei die genetische Veränderung zu einer Veränderung oder Verursachung der Transkriptionsrate von mindestens einem Gen im Vergleich zum Wildtyp führt und bedingt ist durch
a) Veränderung der spezifischen Promotoraktivität im Mikroorganismus von mindestens einer endogenen Nukleinsäure mit Promotoraktivität gemäß Anspruch 1, die die Transkription mindestens eines endogenen Gens reguliert oder
b) Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a), wobei die Gene in Bezug auf die Nukleinsäuren mit Promotoraktivität heterolog sind.

Wie vorstehend bei den Verfahren beschrieben, wird die Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a) dadurch erreicht, dass man
b1) eine oder mehrere Nukleinsäuren mit Promotoraktivität gemäß Anspruch 1, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität gemäß Anspruch 1, gegebenenfalls mit veränderter spezifischer Promotoraktivität, erfolgt oder
b2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 1, gegebenenfalls mit veränderter spezifischer Promotoraktivität, erfolgt oder
b3) ein oder mehrere Nukleinsäurekonstrukte, enthaltend eine Nukleinsäure mit Promotoraktivität gemäß Anspruch 1, gegebenenfalls mit veränderter spezifischer Promotoraktivität, und funktionell verknüpft eine oder mehrere, zu transkribierende Nukleinsäuren, in den Mikroorganismus einbringt.

Die Erfindung betrifft ferner eine genetisch verändertern Mikroorganismus mit erhöhter oder verursachter Transkriptionsrate von mindestens einem Gen im Vergleich zum Wildtyp, wobei
ah) die spezifische Promotoraktivität im Mikroorganismus von endogenen Nukleinsäuren mit Promotoraktivität gemäß Anspruch 1, die die Transkription von endogenen Genen regulieren, im Vergleich zum Wildtyp erhöht ist oder
bh) die Transkription von Genen im Mikroorganismus durch Nukleinsäuren mit Promotoraktivität gemäß Anspruch 1 oder durch Nukleinsäuren mit erhöhter spezifischer Promotoraktivität gemäß Ausführungsform ah) reguliert wird, wobei die Gene in Bezug auf die Nukleinsäuren mit Promotoraktivität heterolog sind.

Wie vorstehend bei den Verfahren beschrieben, wird die Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a) dadurch erreicht, dass man
bh1) eine oder mehrere Nukleinsäuren mit Promotoraktivität gemäß Anspruch 1, gegebenenfalls mit erhöhter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, gegebenenfalls mit erhöhter spezifischer Promotoraktivität, erfolgt oder
bh2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 1, gegebenenfalls mit erhöhter spezifischer Promotoraktivität, erfolgt oder
bh3) ein oder mehrere Nukleinsäurekonstrukte, enthaltend eine Nukleinsäure mit Promotoraktivität gemäß Anspruch 1, gegebenenfalls mit erhöhter spezifischer Promotoraktivität, und funktionell verknüpft eine oder mehrere, zu transkribierende Nukleinsäuren, in den Mikroorganismus einbringt.

Die Erfindung betrifft ferner einen genetisch verändertern Mikroorganismus mit reduzierter Transkriptionsrate von mindetstens einem Gen im Vergleich zum Wildtyp, wobei
ar) die spezifische Promotoraktivität im Mikroorganismus von mindestens einer endogenen Nukleinsäure mit Promotoraktivität gemäß Anspruch 1, die die Transkription von mindestens einem, endogenen Gen reguliert, im Vergleich zum Wildtyp reduziert ist oder
br) eine oder mehrere Nukleinsäuren mit reduzierter Promotoraktivität gemäßAusführungsform a) in das Genom des Mikrorganismus eingebracht wurden, so dass die Transkription mindestens eines endogenen Gens unter der Kontrolle der eingebrachten Nukleinsäure mit reduzierter Promotoraktivität erfolgt.

Die Erfindung betrifft ferner einen genetisch verändertern Mikroorganismus, wobei die genetische Veränderung zu einer Veränderung oder Verursachung der Expressionsrate mindestens eines Gens im Vergleich zum Wildtyp führt und bedingt ist durch
c) Veränderung der spezifischen Expressionsaktivität im Mikroorganismus von mindestens einer endogenen Expressionseinheiten gemäß Anspruch 2 oder 3, die die Expression mindestens eines endogenen Gens reguliert, im Vergleich zum Wildtyp oder
d) Regulation der Expression von Genen im Mikroorganismus durch Expressionseinheiten gemäß Anspruch 2 oder 3 oder durch Expressionseinheiten gemäß Anspruch 2 oder 3 mit veränderter spezifischer Expressionsaktivität gemäß Ausführungsform a), wobei die Gene im Bezug auf die Expressionseinheiten heterolog sind.

Wie vorstehend bei den Verfahren beschrieben, wird die Regulation der Expression von Genen im Mikroorganismus durch Expressionseinheiten gemäß Anspruch 2 oder 3 oder durch Expressionseinheiten gemäß Anspruch 2 oder 3 mit veränderter spezifischer Expressionsaktivität gemäß Ausführungsform a) dadurch erreicht, dass man
d1) eine oder mehrere Expressionseinheiten gemäß Anspruch 2 oder 3, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten gemäß Anspruch 2 oder 3, gegebenenfalls mit veränderter spezifischer Expressionsaktivität, erfolgt oder
d2) ein oder mehrere Gene in das Genom des Mikrorganismus einbringt, so dass die Expression eines oder mehrerer der eingebrachten Gene unter der Kontrolle der endogenen Expressionseinheiten gemäß Anspruch 2 oder 3, gegebenenfalls mit veränderter spezifischer Expressionsaktivität, erfolgt oder
d3) ein oder mehrere Nukleinsäurekonstrukte, enthaltend eine Expressionseinheit gemäß Anspruch 2 oder 3, gegebenenfalls mit veränderter spezifischer Expressionsaktivität, und funktionell verknüpft eine oder mehrere, zu exprimierende Nukleinsäuren, in den Mikroorganismus einbringt.

Die Erfindung betrifft ferner einen genetisch verändertern Mikroorganismus mit erhöhter oder verursachter Expressionsrate mindestens eines Gens im Vergleich zum Wildtyp, wobei man
ch) die spezifische Expressionsaktivität im Mikroorganismus von mindestens einer endogenen Expressionseinheiten gemäß Anspruch 2 oder 3, die die Expression der endogenen Gene reguliert, im Vergleich zum Wildtyp erhöht oder
dh) die Expression von Genen im Mikroorganismus durch Expressionseinheiten gemäß Anspruch 2 oder 3 oder durch Expressionseinheiten gemäß Anspruch 2 oder 3 mit erhöhter spezifischer Expressionsaktivität gemäß Ausführungsform a) reguliert, wobei die Gene im Bezug auf die Expressionseinheiten heterolog sind.

Wie vorstehend bei den Verfahren beschrieben, wird die Regulation der Expression von Genen im Mikroorganismus durch Expressionseinheiten gemäß Anspruch 2 oder 3 oder durch Expressionseinheiten gemäß Anspruch 2 oder 3 mit erhöhter spezifischer Expressionsaktivität gemäß Ausführungsform a) dadurch erreicht, dass man
dh1) eine oder mehrere Expressionseinheiten gemäß Anspruch 2 oder 3, gegebenenfalls mit erhöhter spezifischer Expressionsaktivität, in das Genom des Mikroorganismus einbringt, so dass die Expression eines oder mehrerer endogenen Gene unter der Kontrolle der eingebrachten Expressionseinheiten gemäß Anspruch 2 oder 3, gegebenenfalls mit erhöhter spezifischer Expressionsaktivität, erfolgt oder
dh2) ein oder mehrere Gene in das Genom des Mikrorganismus einbringt, so dass die Expression eines oder mehrerer der eingebrachten Gene unter der Kontrolle der endogenen Expressionseinheiten gemäß Anspruch 2 oder 3, gegebenenfalls mit erhöhter spezifischer Expressionsaktivität, erfolgt oder
dh3) ein oder mehrere Nukleinsäurekonstrukte, enthaltend eine Expressionseinheit gemäß Anspruch 2 oder 3, gegebenenfalls mit erhöhter spezifischer Expressionsaktivität, und funktionell verknüpft eine oder mehrere, zu exprimierende Nukleinsäuren, in den Mikroorganismus einbringt.

Die Erfidnung betrifft ferner einen genetisch verändertern Mikroorganismus mit reduzierter Expressionsrate von mindetstens einem Gen im Vergleich zum Wildtyp, wobei,
cr) die spezifische Expressionsaktivität im Mikroorganismus von mindestens einer endogenen Expressionseinheit gemäß Anspruch 2 oder 3, die die Expression von mindestens einem edogenen Gen reguliert, im Vergleich zum Wildtyp reduziert ist oder
dr) eine oder mehrere Expressionseinheiten gemäß Anspruch 2 oder 3 mit reduzierter Expressionsaktivität in das Genom des Mikrorganismus eingebracht wurden, so dass die Expression mindestens eines Gens unter der Kontrolle der eingebrachten Expressionseinheit gemäß Anspruch 2 oder 3 mit reduzierter Expressionsaktivität erfolgt.

Ferner betrifft die Erfindung einen genetisch veränderter Mikroorganismus, enthaltend eine Expressionseinheit gemäß Anspruch 2 oder 3 und funktionell verknüpft ein zu eprimierendes Gen, wobei das Gen im Bezug auf die Expressionseinheit heterolog ist.

Besonders bevorzugt enthält dieser genetisch veränderte Mikroorganismus eine erfindungsgemäße Expressionskasette.

Besonders bevorzugt betrifft die vorliegende Erfindung gentisch veränderte Mikroorgansismen, insbesondere coryneforme Bakterien, die einen Vektor, insbesondere Pendelvektor oder Plasmidvektor, der wenigstens ein rekombinantes Nukleinsäurekonstrukt erfindungsgemäßer Definition trägt, enthalten.

In einer bevorzugten Ausführungsform der genetisch veränderten Mikroorganismen sind die vorstehend beschriebenen Gene mindestens eine Nukleinsäuren kodierend ein Protein aus den Biosyntheseweg von Feinchemikalien.

In einer besonders bevorzugten Ausführungsform der genetisch veränderten Mikroorganismen sind die vorstehend beschriebenen Gene ausgewählt sind aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

Bevorzugte Proteine aus dem Biosyntheseweg von Aminosäuren sind ausgewählt aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase

Besonders bevorzugte Beispiele der Proteine und Gene aus dem Biosyntheseweg von Aminosäuren sind vorstehend in Tabelle 1 und Tabelle 2 beschrieben.

Bevorzugte Mikroorganismen oder genetisch veränderte Mikroorganismen sind Bakterien, Algen, Pilze oder Hefen.

Besonders bevorzugte Mikroorgansimen sind insbeondere coryneforme Bakterien.

Bevorzugte coryneforme Bakterien sind Bakterien der Gattung Corynebacterium, insbesondere der Arten Corynebacterium glutamicum, Corynebacterium acetoglutamicum, Corynebacterium acetoacidophilum, Corynebacterium thermoaminogenes, Corynebacterium melassecola und Corynebacterium efficiens oder der Gattung Brevibacterium, insbesondere der Arten Brevibacterium flavum, Brevibacterium lactofermentum und Brevibacterium divaricatum.

Besonders bevorzugte Bakterien der Gattungen Corynebacterium und Brevibacterium sind ausgewählt aus der Gruppe Corynebacterium glutamicum ATCC 13032, Corynebacterium acetoglutamicum ATCC 15806, Corynebacterium acetoacidophilum ATCC 13870, Corynebacterium thermoaminogenes FERM BP-1539, Corynebacterium melassecola ATCC 17965, Corynebacterium efficiens DSM 44547, Corynebacterium efficiens DSM 44548. Corynebacterium efficiens DSM 44549, Brevibacterium flavum ATCC 14067, Brevibacterium lactofermentum ATCC 13869, Brevibacterium divaricatum ATCC 14020, Corynebacterium glutamicum KFCC10065 und Corynebacterium glutamicum ATCC21608.

Mit der Abkürzung KFCC ist die Korean Federation of Culture Collection gemeint, mit der Abkürzung ATCC die American type strain culture collection, mit der Abkürzung DSM die Deutsche Sammlung von Mikroorganismen.

Weitere besonders bevorzugte Bakterien der Gattungen Corynebacterium und Brevibacterium sind in Tabelle 3 aufgelistet:

| Bakterium | | Hinterlegungsnummer | | | | | | | |
|---|---|---|---|---|---|---|---|---|---|
| Genus | species | ATCC | FERM | NRRL | CECT | NCIMB | CBS | NCTC | DSMZ |
| Brevibacterium | ammoniagenes | 21054 | | | | | | | |
| Brevibacterium | ammoniagenes | 19350 | | | | | | | |
| Brevibacterium | ammoniagenes | 19351 | | | | | | | |
| Brevibacterium | ammoniagenes | 19352 | | | | | | | |
| Brevibacterium | ammoniagenes | 19353 | | | | | | | |
| Brevibacterium | ammoniagenes | 19354 | | | | | | | |
| Brevibacterium | ammoniagenes | 19355 | | | | | | | |
| Brevibacterium | ammoniagenes | 19356 | | | | | | | |
| Brevibacterium | ammoniagenes | 21055 | | | | | | | |
| Brevibacterium | ammoniagenes | 21077 | | | | | | | |
| Brevibacterium | ammoniagenes | 21553 | | | | | | | |
| Brevibacterium | ammoniagenes | 21580 | | | | | | | |
| Brevibacterium | ammoniagenes | 39101 | | | | | | | |
| Brevibacterium | butanicum | 21196 | | | | | | | |
| Brevibacterium | divaricatum | 21792 | P928 | | | | | | |
| Brevibacterium | flavum | 21474 | | | | | | | |
| Brevibacterium | flavum | 21129 | | | | | | | |
| Brevibacterium | flavum | 21518 | | | | | | | |
| Brevibacterium | flavum | | | B11474 | | | | | |
| Brevibacterium | flavum | | | B11472 | | | | | |
| Brevibacterium | flavum | 21127 | | | | | | | |
| Brevibacterium | flavum | 21128 | | | | | | | |
| Brevibacterium | flavum | 21427 | | | | | | | |
| Brevibacterium | flavum | 21475 | | | | | | | |
| Brevibacterium | flavum | 21517 | | | | | | | |
| Brevibacterium | flavum | 21528 | | | | | | | |
| Brevibacterium | flavum | 21529 | | | | | | | |
| Brevibacterium | flavum | | | B11477 | | | | | |
| Brevibacterium | flavum | | | B11478 | | | | | |
| Brevibacterium | flavum | 21127 | | | | | | | |
| Brevibacterium | flavum | | | B11474 | | | | | |
| Brevibacterium | healii | 15527 | | | | | | | |
| Brevibacterium | ketoglutamicum | 21004 | | | | | | | |
| Brevibacterium | ketoglutamicum | 21089 | | | | | | | |
| Brevibacterium | ketosoreductum | 21914 | | | | | | | |
| Brevibacterium | lactofermentum | | | | 70 | | | | |
| Brevibacterium | lactofermentum | | | | 74 | | | | |
| Brevibacterium | lactofermentum | | | | 77 | | | | |
| Brevibacterium | lactofermentum | 21798 | | | | | | | |
| Brevibacterium | lactofermentum | 21799 | | | | | | | |
| Brevibacterium | lactofermentum | 21800 | | | | | | | |
| Brevibacterium | lactofermentum | 21801 | | | | | | | |
| Brevibacterium | lactofermentum | | | B11470 | | | | | |
| Brevibacterium | lactofermentum | | | B11471 | | | | | |
| Brevibacterium | lactofermentum | 21086 | | | | | | | |
| Brevibacterium | lactofermentum | 21420 | | | | | | | |
| Brevibacterium | lactofermentum | 21086 | | | | | | | |
| Brevibacterium | lactofermentum | 31269 | | | | | | | |
| Brevibacterium | linens | 9174 | | | | | | | |
| Brevibacterium | linens | 19391 | | | | | | | |
| Brevibacterium | linens | 8377 | | | | | | | |
| Brevibacterium | paraffinolyticum | | | | | 11160 | | | |
| Brevibacterium | spec. | | | | | | 717.73 | | |
| Brevibacterium | spec. | | | | | | 717.73 | | |
| Brevibacterium | spec. | 14604 | | | | | | | |
| Brevibacterium | spec. | 21860 | | | | | | | |
| Brevibacterium | spec. | 21864 | | | | | | | |
| Brevibacterium | spec. | 21865 | | | | | | | |
| Brevibacterium | spec. | 21866 | | | | | | | |
| Brevibacterium | spec. | 19240 | | | | | | | |
| Corynebacterium | acetoacidophilum | 21476 | | | | | | | |
| Corynebacterium | acetoacidophilum | 13870 | | | | | | | |
| Corynebacterium | acetoglutamicum | | | B11473 | | | | | |
| Corynebacterium | acetoglutamicum | | | B11475 | | | | | |
| Corynebacterium | acetoglutamicum | 15806 | | | | | | | |
| Corynebacterium | acetoglutamicu m | 21491 | | | | | | | |
| Corynebacterium | acetoglutamicum | 31270 | | | | | | | |
| Corynebacterium | acetophilum | | | B3671 | | | | | |
| Corynebacterium | ammoniagenes | 6872 | | | | | | 2399 | |
| Corynebacterium | ammoniagenes | 15511 | | | | | | | |
| Corynebacterium | fujiokense | 21496 | | | | | | | |
| Corynebacterium | glutamicum | 14067 | | | | | | | |
| Corynebacterium | glutamicum | 39137 | | | | | | | |
| Corynebacterium | glutamicum | 21254 | | | | | | | |
| Corynebacterium | glutamicum | 21255 | | | | | | | |
| Corynebacterium | glutamicum | 31830 | | | | | | | |
| Corynebacterium | glutamicum | 13032 | | | | | | | |
| Corynebacterium | glutamicum | 14305 | | | | | | | |
| Corynebacterium | glutamicum | 15455 | | | | | | | |
| Corynebacterium | glutamicum | 13058 | | | | | | | |
| Corynebacterium | glutamicum | 13059 | | | | | | | |
| Corynebacterium | glutamicum | 13060 | | | | | | | |
| Corynebacterium | glutamicum | 21492 | | | | | | | |
| Corynebacterium | glutamicum | 21513 | | | | | | | |
| Corynebacterium | glutamicum | 21526 | | | | | | | |
| Corynebacterium | glutamicum | 21543 | | | | | | | |
| Corynebacterium | glutamicum | 13287 | | | | | | | |
| Corynebacterium | glutamicum | 21851 | | | | | | | |
| Corynebacterium | glutamicum | 21253 | | | | | | | |
| Corynebacterium | glutamicum | 21514 | | | | | | | |
| Corynebacterium | glutamicum | 21516 | | | | | | | |
| Corynebacterium | glutamicum | 21299 | | | | | | | |
| Corynebacterium | glutamicum | 21300 | | | | | | | |
| Corynebacterium | glutamicum | 39684 | | | | | | | |
| Corynebacterium | glutamicum | 21488 | | | | | | | |
| Corynebacterium | glutamicum | 21649 | | | | | | | |
| Corynebacterium | glutamicum | 21650 | | | | | | | |
| Corynebacterium | glutamicum | 19223 | | | | | | | |
| Corynebacterium | glutamicum | 13869 | | | | | | | |
| Corynebacterium | glutamicum | 21157 | | | | | | | |
| Corynebacterium | glutamicum | 21158 | | | | | | | |
| Corynebacterium | glutamicum | 21159 | | | | | | | |
| Corynebacterium | glutamicum | 21355 | | | | | | | |
| Corynebacterium | glutamicum | 31808 | | | | | | | |
| Corynebacterium | glutamicum | 21674 | | | | | | | |
| Corynebacterium | glutamicum | 21562 | | | | | | | |
| Corynebacterium | glutamicum | 21563 | | | | | | | |
| Corynebacterium | glutamicum | 21564 | | | | | | | |
| Corynebacterium | glutamicum | 21565 | | | | | | | |
| Corynebacterium | glutamicum | 21566 | | | | | | | |
| Corynebacterium | glutamicum | 21567 | | | | | | | |
| Corynebacterium | glutamicum | 21568 | | | | | | | |
| Corynebacterium | glutamicum | 21569 | | | | | | | |
| Corynebacterium | glutamicum | 21570 | | | | | | | |
| Corynebacterium | glutamicum | 21571 | | | | | | | |
| Corynebacterium | glutamicum | 21572 | | | | | | | |
| Corynebacterium | glutamicum | 21573 | | | | | | | |
| Corynebacterium | glutamicum | 21579 | | | | | | | |
| Corynebacterium | glutamicum | 19049 | | | | | | | |
| Corynebacterium | glutamicum | 19050 | | | | | | | |
| Corynebacterium | glutamicum | 19051 | | | | | | | |
| Corynebacterium | glutamicum | 19052 | | | | | | | |
| Corynebacterium | glutamicum | 19053 | | | | | | | |
| Corynebacterium | glutamicum | 19054 | | | | | | | |
| Corynebacterium | glutamicum | 19055 | | | | | | | |
| Corynebacterium | glutamicum | 19056 | | | | | | | |
| Corynebacterium | glutamicum | 19057 | | | | | | | |
| Corynebacterium | glutamicum | 19058 | | | | | | | |
| Corynebacterium | glutamicum | 19059 | | | | | | | |
| Corynebacterium | glutamicum | 19060 | | | | | | | |
| Corynebacterium | glutamicum | 19185 | | | | | | | |
| Corynebacterium | glutamicum | 13286 | | | | | | | |
| Corynebacterium | glutamicum | 21515 | | | | | | | |
| Corynebacterium | glutamicum | 21527 | | | | | | | |
| Corynebacterium | glutamicum | 21544 | | | | | | | |
| Corynebacterium | glutamicum | 21492 | | | | | | | |
| Corynebacterium | glutamicum | | | B8183 | | | | | |
| Corynebacterium | glutamicum | | | B8182 | | | | | |
| Corynebacterium | glutamicum | | | B12416 | | | | | |
| Corynebacterium | glutamicum | | | B12417 | | | | | |
| Corynebacterium | glutamicum | | | B12418 | | | | | |
| Corynebacterium | glutamicum | | | B11476 | | | | | |
| Corynebacterium | glutamicum | 21608 | | | | | | | |
| Corynebacterium | lilium | | P973 | | | | | | |
| Corynebacterium | nitrilophilus | 21419 | | | | 11594 | | | |
| Corynebacterium | spec. | | P4445 | | | | | | |
| Corynebacterium | spec. | | P4446 | | | | | | |
| Corynebacterium | spec. | 31088 | | | | | | | |
| Corynebacterium | spec. | 31089 | | | | | | | |
| Corynebacterium | spec. | 31090 | | | | | | | |
| Corynebacterium | spec. | 31090 | | | | | | | |
| Corynebacterium | spec. | 31090 | | | | | | | |
| Corynebacterium | spec. | 15954 | | | | | | | 20145 |
| Corynebacterium | spec. | 21857 | | | | | | | |
| Corynebacterium | spec. | 21862 | | | | | | | |
| Corynebacterium | spec. | 21863 | | | | | | | |

Die Abkürzungen haben folgende Bedeutung:
ATCC: American Type Culture Collection, Rockville, MD, USA
FERM: Fermentation Research Institute, Chiba, Japan
NRRL: ARS Culture Collection, Northern Regional Research Laboratory, Peoria, IL, USA
CECT: Coleccion Espanola de Cultivos Tipo, Valencia, Spain
NCIMB: National Collection of Industrial and Marine Bacteria Ltd., Aberdeen, UK
CBS: Centraalbureau voor Schimmelcultures, Baarn, NL
NCTC: National Collection of Type Cultures, London, UK
DSMZ: Deutsche Sammlung von Mikroorganismen und Zellkulturen, Braunschweig, Germany

Durch die erfindungsgemäßen Nukleinsäuren mit Promotoraktivität und den erfindungsgemäßen Expressionseinheiten ist es mit Hilfe der vorstehend beschriebenen, erfindungsgemäßen Verfahren möglich in den vorstehend beschriebenen, erfindungsgemäßen genetisch veränderten Mikroorganismen die Stoffwechselwege zu spezifischen biosynthetischen Produkten zu regulieren.

Dazu werden beispielsweise Stoffwechselwege die zu einem spezifischen biosynthetischen Produkt führen durch Verursachung oder Erhöhung der Transkriptionrate bzw. Expressionsrate von Genen dieses Biosyntheseweges verstärkt in dem die erhöhte Proteinmenge zu einer erhöhten Gesamtaktivität dieser Proteine des gewünschten Biosyntheseweges und damit zu einem verstärkten Stoffwechselfluß zu dem gewünschen biosynthetischen Produkt führt.

Weiterhin können Stoffwechselwege die von einem spezifischen biosynthetischen Produkt wegführen durch Reduzierung der Transkriptionrate bzw. Expressionsrate von Genen dieses wegführenden Biosyntheseweges abgeschwächt werden in dem die reduzierte Proteinmenge zu einer reduzierten Gesamtaktivität dieser Proteine des unerwünschten Biosyntheseweges und damit zusätzlich zu einem verstärkten Stoffwechselfluß zu dem gewünschen biosynthetischen Produkt führt.

Die erfindungsgemäßen genetisch veränderten Mikroorganismen sind beispielsweise in der Lage aus Glucose, Saccharose, Lactose, Fructose, Maltose, Melasse, Stärke, Cellulose oder aus Glycerin und Ethanol biosynthetische Produkte herzustellen.

Die Erfindung betrifft daher ein Verfahren zur Herstellung von biosynthetischen Produkten durch Kultivierung von erfindungsgemäßen, genetisch veränderten Mikroorganismen.

Je nach gewünschtem biosynthetischen Produkt muss die Transkriptionsrate bzw. Expressionsrate verschiedener Gene erhöht bzw. reduziert werden. In der Regel ist es vorteilhaft die Transkrioptionsrate bzw. Expressionsrate mehrere Gene zu verändern, d.h. die Transkrioptionsrate bzw. Expressionsrate einer Kombination von Gene zu Erhöhen und/oder die Transkrioptionsrate bzw. Expressionsrate einer Kombination von Gene zu reduzieren.

In den erfindungsgemäßen genetisch veränderten Mikroorganismen ist mindestens eine veränderte, dass heißt erhöhte oder reduzierte Transkriptionsrate bzw. Expressionsrate eines Gens auf eine erfindungsgemäße Nukleinsäure mit Promotoraktivität bzw. erfindungsgemäße Expressionseinheit zurückzuführen.

Weitere, zusätzliche veränderte, d.h. zusätzlich erhöhte oder zusätzlich reduzierte Transkriptionsraten bzw. Expressionsraten von weiteren Genen im genetisch veränderten Mikroorganismus können, müssen aber nicht auf die erfindungsgemäßen Nukleinsäuren mit Promotoraktivität bzw. die erfindungsgemäßen Expressionseinheiten zurück gehen.

Die Erfindung betrifft deshalb weiterhin ein Verfahren zur Herstellung von biosynthetischen Produkten durch Kultivierung von erfindungsgemäßen, genetisch veränderten Mikroorganismen.

Bevorzugte biosynthetische Produkte sind Feinchemikalien.

Der Begriff "Feinchemikalie" ist im Fachgebiet bekannt und beinhaltet Verbidnungen, die von einem Organismus produziert werden und in verschiedenen Industriezweigen Anwendungen finden, wie bspw., jedoch nicht beschränkt auf die pharmazeutische Industrie, die Landwirtschafts-, Kosmetik , Food und Feed-Industrie. Diese Verbindungen umfassen organische Säuren, wie beispielsweise Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlenhydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme und sämtliche anderen von Gutcho (1983) in Chemicals by Fermentation, Noyes Data Corporation, ISBN: 0818805086 und den darin angegebenen Literaturstellen, beschriebenen Chemikalien. Der Metabolismus und die Verwendungen bestimmter Feinchemikalien sind nachstehend weiter erläutert.

### I. Aminosäure-Metabolismus und Verwendungen

Die Aminosäuren umfassen die grundlegenden Struktureinheiten sämtlicher Proteine und sind somit für die normalen Zellfunktionen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ullmann's Encyclopedia of Industrial Chemistry, Bd. A2, S. 57-97 VCH: Weinheim (1985)). Die Aminosäuren können in der D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L. Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, da sie aufgrund der Komplexität ihrer Biosynthese mit der Ernährung aufgenommen werden müssen, werden durch einfache Biosyntheseswege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen, in Ullmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakteriellen Aminosäure-Biosynthese und ihrer Regulation, s. Umbarger, H.E. (1978) Ann. Rev. Biochem. 47: 533 - 606). Glutamat wird durch reduktive Aminierung von - Ketoglutarat, einem Zwischenprodukt im Citronensäure-Zyklus, synthetisiert. Glutamin, Prolin und Arginin werden jeweils nacheinander aus Glutamat erzeugt. Die Biosynthese von Serin erfolgt in einem Dreischritt-Verfahren und beginnt mit 3-Phosphoglycerat (einem Zwischenprodukt bei der Glykolyse), und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten- -Kohlenstoffatoms auf Tetrahydrofolat, in einer durch Serintranshydroxymethylase katalysierten Reaktion. Phenylalanin und Tyrosin werden aus den Vorstufen des Glycolyse- und Pentosephosphatweges, Erythrose-4-phosphat und Phosphoenolpyruvat in einem 9-Schritt-Biosyntheseweg synthetisiert, der sich nur in den letzten beiden Schritten nach der Synthese von Prephenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf der Zelle übersteigt, können nicht gespeichert werden, und werden stattdessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über den Rückkopplungs-Mechanismus bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### II. Vitamine, Cofaktoren und Nutrazeutika-Metabolismus sowie Verwendungen

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullmann's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nicht-proteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullmann's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B6" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)- -alanin) kann entweder durch chemische Synthese oder durch Fermentation hergestellt werden. Die letzten Schritte bei der Pantothenat-Biosynthese bestehen aus der ATP-getriebenen Kondensation von -Alanin und Pantoinsäure. Die für die Biosyntheseschritte für die Umwandlung in Pantoinsäure, in -Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des -Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den metabolischen Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien ebenfalls durch großangelegte Anzucht von Mikroorganismen produziert worden sind, wie Riboflavin, Vitamin B₆, Pantothenat und Biotin. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### III. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteil der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisation zur Bildung von Nukleinsäuremolekülen ist es möglich, die RNA- und DNA-Synthese zu hemmen; wird diese Aktivität zielgerichtet bei kanzerogenen Zellen gehemmt, läßt sich die Teilungs- und Replikations-Fähigkeit von Tumorzellen hemmen.

Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (bspw. Christopherson, R.I. und Lyons, S.D. (1990) "Potent inhibitors of de novo pyrimidine and purine biosynthesis as chemotherapeutic agents", Med. Res. Reviews 10: 505-548). Untersuchungen an Enzymen, die am Purin- und Pyrimidin-Metabolismus beteiligt sind, haben sich auf die Entwicklung neuer Medikamente konzentriert, die bspw. als Immunsuppressionsmittel oderAntiproliferantien verwendet werden können (Smith, J.L. "Enzymes in Nucleotide Synthesis" Curr. Opin. Struct. Biol. 5 (1995) 752-757; Biochem. Soc. Transact. 23 (1995) 877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, werden gewöhnlich als Geschmacksverstärker verwendet (bspw. IMP oder GMP) oder für viele medizinische Anwendungen (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular biology, Bd. 42, Academic Press, S. 259-287; und Michal, G. (1999) "Nucleotides and Nucleosides"; Kap. 8 in : Biochemical Pathways: An Atlas of Biochemistry and Molecular Biology, Wiley, New York). Der Purin-Metabolismus, das Objekt intesiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden über eine Reihe von Schritten über die Zwischenverbindung Inosin-5'-phosphat (IMP) aus Ribose-5-phosphat synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### IV.Trehalose-Metabolismus und Verwendungen

Trehalose besteht aus zwei Glucosemolekülen, die über , -1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist, S. Trends Biotech. 16 (1998) 460-467; Paiva, C.L.A. und Panek, A.D. Biotech Ann. Rev. 2 (1996) 293-314; und Shiosaka, M. J. Japan 172 (1997) 97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

Besonders bevorzugte biosynthetische Produkte sind ausgewählt aus der Gruppe organische Säuren, Proteine, Nukleotide und Nukleoside, sowohl proteinogene als auch nicht-proteinogene Aminosäuren, Lipide und Fettsäuren, Diole, Kohlehydrate, aromatische Verbindungen, Vitamine und Cofaktoren, Enzyme und Proteine.

Bevorzugte organische Säuren sind Weinsäure, Itaconsäure und Diaminopimelinsäure

Bevorzugte Nukleoside und Nukleotide sind beispielsweise beschrieben in Kuninaka, A. (1996) Nucleotides and related compounds, S. 561-612, in Biotechnology Bd. 6, Rehm et al., Hrsg. VCH: Weinheim und den darin enthaltenen Zitaten.

Bevorzugte biosynthetische Produkte sind weiterhin Lipide, gesättigte und ungesättigte Fettsäuren, wie beispielsweise Arachidonsäure, Diole wie beispielsweise Propandiol und Butandiol, Kohlenhydrate, wie beispielsweise Hyaluronsäure und Trehalose, aromatische Verbindungen, wie beispielsweise aromatische Amine, Vanillin und Indigo, Vitamine und Cofaktoren, wie beispielsweise beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme Polyketide (Cane et al. (1998) Science 282: 63-68), und sämtliche anderen von Gutcho (1983) in Chemicals by Fermentation, Noyes Data Corporation, ISBN: 0818805086 und den darin angegebenen Literaturstellen, beschriebenen Chemikalien.

Besonders bevorzugte biosynthetische Produkte sind Aminosäuren, besonders bevorzugt essentielle Aminosäuren, insbeondere L-Glycin, L-Alanin, L-leucin, L-Methionin, L-Phenylalanin, L-Tryptophan, L-Lysin, L-Glutamin, L-Glutaminsäure, L-Serin, L-Prolin, L-Valin, L-Isoleucin, L-Cystein, L-Tyrosin, L-Histidin, L-Arginin, L-Asparagin, L-Asparaginsäure und L-Threonin, L-Homoserin, insbesondere L-Lysin, L-Methionin und L-Threonin. Im folgenden wird unter unter einer Aminosäure, wie beispielsweise Lysin, Methionin und Threonin, sowohl jeweils die L- und die D-Form der Aminosäure, vorzugsweise die L-Form, also beispielsweise L-Lysin, L-Methionin und L-Threonin verstanden.

Die Erfindung betrifft insbesondere ein Verfahren zur Herstellung von Lysin durch Kultivierung von genetisch veränderten Mikroorganismen mit erhöhter oder verursachter Expressionsrate mindestens eines Gens im Vergleich zum Wildtyp, wobei man
ch) die spezifische Expressionsaktivität im Mikroorganismus von mindestens einer endogenen, erfindungsgemäßen Expressionseinheit, die die Expression der endogenen Gene reguliert, im Vergleich zum Wildtyp erhöht oder
dh) die Expression von Genen im Mikroorganismus durch erfindungsgemäße Expressionseinheiten oder durch erfindungsgemäße Expressionseinheiten mit erhöhter spezifischer Expressionsaktivität gemäß Ausführungsform ch) reguliert, wobei die Gene im Bezug auf die Expressionseinheiten heterolog sind,
und wobei die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Diaminopimelat- Dehydrogenase, Nukleinsäuren kodierend eine Diaminopimelat- Decarboxylase, Nukleinsäuren kodierend eine Dihydrodipicolinate-Synthetase, Nukleinsäuren kodierend eine Dihydridipicolinate-Reduktase, Nukleinsäuren kodierend eine Glycerinaldehyd-3-Phosphat Dehydrogenase, Nukleinsäuren kodierend eine 3-Phosphoglycerat-Kinase, Nukleinsäuren kodierend eine Pyruvat Carboxylase, Nukleinsäuren kodierend eine Triosephosphat-Isomerase, Nukleinsäuren kodierend einen Transkriptionellen Regulator LuxR, Nukleinsäuren kodierend einen Transkriptionellen Regulator LysR1, Nukleinsäuren kodierend einen Transkriptionellen Regulator LysR2, Nukleinsäuren kodierend eine Malat-Quinon-Oxodoreduktase, Nukleinsäuren kodierend eine Glucose-6-Phosphat-Deydrognease, Nukleinsäuren kodierend eine 6-Phosphogluconat-Dehydrognease, Nukleinsäuren kodierend eine Transketolase, Nukleinsäuren kodierend eine Transaldolase, Nukleinsäuren kodierend einen Lysin Exporter, Nukleinsäuren kodierend eine Biotin-Ligase, Nukleinsäuren kodierend eine Arginyl-t-RNA-Synthetase, Nukleinsäuren kodierend eine Phosphoenolpyruvat-Carboxylase, Nukleinsäuren kodierend eine Fruktose-1,6-bisphosphatase, Nukleinsäuren kodierend ein Protein OPCA, Nukleinsäuren kodierend eine 1-Phosphofructokinase und Nukleinsäuren kodierend eine 6-Phosphofructokinase.

Wie vorstehend bei den Verfahren beschrieben, wird die Regulation der Expression dieser Genen im Mikroorganismus durch erfindungsgemäße Expressionseinheiten oder durch erfindungsgemäße Expressionseinheiten mit erhöhter spezifischer Expressionsaktivität gemäß Ausführungsform ch) dadurch erreicht, dass man
dh1) eine oder mehrere erfindungsgemäße Expressionseinheiten, gegebenenfalls mit erhöhter spezifischer Expressionsaktivität, in das Genom des Mikroorganismus einbringt, so dass die Expression eines oder mehrerer dieser endogenen Gene unter der Kontrolle der eingebrachten, erfindungsgemäßen Expressionseinheiten, gegebenenfalls mit erhöhter spezifischer Expressionsaktivität, erfolgt oder
dh2) ein oder mehrere dieser Gene in das Genom des Mikrorganismus einbringt, so dass die Expression eines oder mehrerer der eingebrachten Gene unter der Kontrolle der endogenen, erfindungsgemäßen Expressionseinheiten, gegebenenfalls mit erhöhter spezifischer Expressionsaktivität, erfolgt oder
dh3) ein oder mehrere Nukleinsäurekonstrukte, enthaltend eine erfindungsgemäße Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

Eine weiter bevorzugte Ausführungsform des vorstehend beschriebenen Verfahrens zur Herstellung von Lysin ist dadurch gekennzeichnet, dass die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Diaminopimelat- Dehydrogenase-Aktivität, Diaminopimelat-Decarboxylase-Aktivität, Dihydrodipicolinate-Synthetase-Aktivität, Dihydridipicolinate-Reduktase-Aktivität, Glycerinaldehyd-3-Phosphat Dehydrogenase-Aktivität, 3-Phosphoglycerat-Kinase-Aktivität, Pyruvat Carboxylase-Aktivität, Triosephosphat-Isomerase-Aktivität, Aktivität des Transkriptionellen Regulators LuxR, Aktivität des Transkriptionellen Regulators LysR1, Aktivität des Transkriptionellen Regulators LysR2, Malat-Quinon-Oxodoreduktase-Aktivität, Glucose-6-Phosphat-Deydrognease-Aktivität, 6-Phosphogluconat-Dehydrognease-Aktivität, Transketolase-Aktivität, Transaldolase-Aktivität, Lysin-Exporter-Aktivität, Arginyl-t-RNA-Synthetase-Aktivität, Phosphoenolpyruvat-Carboxylase-Aktivität, Fruktose-1,6-bisphosphatase-Aktivität, Protein OpcA-Aktivität, 1-Phosphofructokinase-Aktivität, 6-Phosphofructokinase-Aktivität und Biotin-Ligase-Aktivität aufweisen.

Eine weiter besonders bevorzugte Ausführungsform des vorstehend beschriebenen Verfahrens zur Herstellung von Lysin ist dadurch gekennzeichnet, dass die genetisch veränderten Mikroorganismen im Vergleich zum Wildtyp zusätzlich eine reduzierte Aktivität, mindestens einer der Aktivitäten, ausgewählt aus der Gruppe Threonin Dehydratase-Aktivität, Homoserin O-Acetyltransferase-Aktivität, O-Acetylhomoserin-Sulfhydrylase-Aktivität, Phosphoenolpyruvat-Carboxykinase-Aktivität, Pyruvat-Oxidase-Aktivität, Homoserine-Kinase-Aktivität, Homoserin-Dehydrogenase-Aktivität, Threonin-Exporter-Aktivität, Threonin-Efflux-Protein-Aktivität, Asparaginase-Aktivität, Aspartat-Decarboxylase-Aktivität und Threonin-Synthase-Aktivität aufweisen.

Diese zusätzlichen, erhöhten oder reduzierten Aktivitäten mindestens einer der vorstehend beschriebenen Aktivitäten können, müssen aber nicht durch eine erfindungsgemäße Nukleinsäure mit Promotoraktivität und/oder eine erfindungsgemäße Expressionseinheit verursacht sein.

Die Erfindung betrifft ferner ein Verfahren zur Herstellung von Methionin durch Kultivierung von genetisch veränderten Mikroorganismen mit erhöhter oder verursachter Expressionsrate mindestens eines Gens im Vergleich zum Wildtyp, wobei man
ch) die spezifische Expressionsaktivität im Mikroorganismus von mindestens einer endogenen, erfindungsgemäßen Expressionseinheit, die die Expression der endogenen Gene reguliert, im Vergleich zum Wildtyp erhöht oder
dh) die Expression von Genen im Mikroorganismus durch erfindungsgemäße Expressionseinheiten oder durch erfindungsgemäße Expressionseinheiten mit erhöhter spezifischer Expressionsaktivität gemäß Ausführungsform ch) reguliert, wobei die Gene im Bezug auf die Expressionseinheiten heterolog sind,
und wobei die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Homoserin Dehydrogenase, Nukleinsäuren kodierend eine Glycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin O-Acetyltransferase, Nukleinsäuren kodierend eine Cystahionin-gamma-Synthase, Nukleinsäuren kodierend eine Cystahionin-beta-Lyase, Nukleinsäuren kodierend eine Serin-Hydroxymethyltransferase, Nukleinsäuren kodierend eine O-Acetylhomoserin-Sulfhydrylase, Nukleinsäuren kodierend eine Methylen-Tetrahydrofolat-Reduktase, Nukleinsäuren kodierend eine Phosphoserin-Aminotransferase, Nukleinsäuren kodierend eine Phosphoserin-Phosphatase, Nukleinsäuren kodierend eine Serine Acetyl-TransferaseNukleinsäuren kodierend eine Cystein-Synthase Aktivität I, Nukleinsäuren kodierend eine Cystein-Synthase Aktivität II , Nukleinsäuren kodierend eine Coenzym B12-abhängige Methionin-Synthase-Aktivität, Nukleinsäuren kodierend eine Coenzym B12-unabhängige Methionin-Synthase-Aktivität, Nukleinsäuren kodierend eine Sulfat-Adenylyltransferase-Aktivität, Nukleinsäuren kodierend eine Phosphoadenosin-Phosphosulfat-Reductase-Aktivität, Nukleinsäuren kodierend eine Ferredoxin-Sulfit-Reduktase-Aktivität, Nukleinsäuren kodierend eine Ferredoxin NADPH-Reduktase Aktivität, Nukleinsäuren kodierend eine Ferredoxin-Aktivität, Nukleinsäuren kodierend ein Protein der Sulfat-Reduktion RXA077, Nukleinsäuren kodierend ein Protein der Sulfat-Reduktion RXA248, Nukleinsäuren kodierend ein Protein der Sulfat-Reduktion RXA247, Nukleinsäuren kodierend eine, RXA0655 Regulator und Nukleinsäuren kodierend einen RXN2910 Regulator.

Wie vorstehend bei den Verfahren beschrieben, wird die Regulation der Expression dieser Genen im Mikroorganismus durch erfindungsgemäße Expressionseinheiten oder durch erfindungsgemäße Expressionseinheiten mit erhöhter spezifischer Expressionsaktivität gemäß Ausführungsform ch) dadurch erreicht, dass man
dh1) eine oder mehrere erfindungsgemäße Expressionseinheiten, gegebenenfalls mit erhöhter spezifischer Expressionsaktivität, in das Genom des Mikroorganismus einbringt, so dass die Expression eines oder mehrerer dieser endogenen Gene unter der Kontrolle der eingebrachten, erfindungsgemäßen Expressionseinheiten, gegebenenfalls mit erhöhter spezifischer Expressionsaktivität, erfolgt oder
dh2) ein oder mehrere dieser Gene in das Genom des Mikrorganismus einbringt, so dass die Expression eines oder mehrerer der eingebrachten Gene unter der Kontrolle der endogenen, erfindungsgemäßen Expressionseinheiten, gegebenenfalls mit erhöhter spezifischer Expressionsaktivität, erfolgt oder
dh3) ein oder mehrere Nukleinsäurekonstrukte, enthaltend eine erfindungsgemäße Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

Eine weiter bevorzugte Ausführungsform des vorstehend beschriebenen Verfahrens zur Herstellung von Methionin ist dadurch gekennzeichnet, dass die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Homoserin Dehydrogenase-Aktivität, Glycerinaldehyd-3-Phosphat Dehydrogenase-Aktivität, 3-Phosphoglycerat Kinase-Aktivität, Pyruvat Carboxylase-Aktivität, Triosephosphat Isomerase-Aktivität, Homoserin O-Acetyltransferase-Aktivität, Cystahionin-gamma-Synthase-Aktivität, Cystahionin-beta-Lyase-Aktivität Serin-Hydroxymethyltransferase-Aktivität, O-Acetylhomoserin-Sulfhydrylase-Aktivität, Methylen-Tetrahydrofolat-Reduktase-Aktivität, Phosphoserin-Aminotransferase-Aktivität, Phosphoserin-Phosphatase-Aktivität, Serine Acetyl-Transferase-Aktivität, Cystein-Synthase-Aktivität, Cystein-Synthase II -Aktivität, Coenzym B12-abhängige Methionin-Synthase-Aktivität, Coenzym B12-unabhängige Methionin-Synthase-Aktivität, Sulfat-Adenylyltransferase-Aktivität, Phosphoadenosin-Phosphosulfat-Reductase-Aktivität, Ferredoxin-Sulfit-Reduktase-Aktivität, Ferredoxin NADPH-Reduktase Aktivität, Ferredoxin-Aktivität Aktivität Proteins der Sulfat-Reduktion RXA077, Aktivität eines Proteins der Sulfat-Reduktion RXA248, Aktivität eines Proteins der Sulfat-Reduktion RXA247, Aktivität eines RXA655-Regulators und Aktivität eines RXN2910-Regulators aufweisen

Eine weiter besonders bevorzugte Ausführungsform des vorstehend beschriebenen Verfahrens zur Herstellung von Methionin ist dadurch gekennzeichnet, dass die genetisch veränderten Mikroorganismen im Vergleich zum Wildtyp zusätzlich eine reduzierte Aktivität, mindestens einer der Aktivitäten, ausgewählt aus der Homoserine-Kinase-Aktivität, Threonin-Dehydratase-Aktivität, Threonin Synthase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthase-Aktivität, Dihydrodipicolinat Reduktase-Aktivität, und Diaminopicolinat Decarboxylase-Aktivität aufweisen.

Diese zusätzlichen, erhöhten oder reduzierten Aktivitäten mindestens einer der vorstehend beschriebenen Aktivitäten können, müssen aber nicht durch eine erfindungsgemäße Nukleinsäure mit Promotoraktivität und/oder eine erfindungsgemäße Expressionseinheit verursacht sein.

Die Erfindung betrifft ferner ein Verfahren zur Herstellung von Threonin durch Kultivierung von genetisch veränderten Mikroorganismen mit erhöhter oder verursachter Expressionsrate mindestens eines Gens im Vergleich zum Wildtyp, wobei man
ch) die spezifische Expressionsaktivität im Mikroorganismus von mindestens einer endogenen, erfindungsgemäßen Expressionseinheit, die die Expression der endogenen Gene reguliert, im Vergleich zum Wildtyp erhöht oder
dh) die Expression von Genen im Mikroorganismus durch erfindungsgemäße Expressionseinheiten oder durch erfindungsgemäße Expressionseinheiten mit erhöhter spezifischer Expressionsaktivität gemäß Ausführungsform ch) reguliert, wobei die Gene im Bezug auf die Expressionseinheiten heterolog sind,
und wobei die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Glycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin-Kinase, Nukleinsäuren kodierend eine Threonin Synthase, Nukleinsäuren kodierend einen Threonin Exporter Carrier, Nukleinsäuren kodierend eine Glucose-6-Phosphat-Dehydrogenase, Nukleinsäuren kodierend eine Transaldolase, Nukleinsäuren kodierend eine Transketolase, Nukleinsäuren kodierend einer Malat-Quinon-Oxidoreductase, Nukleinsäuren kodierend eine 6-Phosphogluconat-Dehydrogenase, Nukleinsäuren kodierend einen Lysin-Exporter, Nukleinsäuren kodierend eine Biotin-Ligase, Nukleinsäuren kodierend eine Phosphoenolpyruvat-Carboxylase, Nukleinsäuren kodierend ein Threonin Efflux-Protein, Nukleinsäuren kodierend eine Fruktose-1,6-bisphosphatase, Nukleinsäuren kodierend ein OpcA Protein, Nukleinsäuren kodierend eine 1-Phosphofructokinase, Nukleinsäuren kodierend eine 6-Phosphofructokinase, und Nukleinsäuren kodierend eine Homoserin-Dehydrogenase

Wie vorstehend bei den Verfahren beschrieben, wird die Regulation der Expression dieser Genen im Mikroorganismus durch erfindungsgemäße Expressionseinheiten oder durch erfindungsgemäße Expressionseinheiten mit erhöhter spezifischer Expressionsaktivität gemäß Ausführungsform ch) dadurch erreicht, dass man
dh1) eine oder mehrere erfindungsgemäße Expressionseinheiten, gegebenenfalls mit erhöhter spezifischer Expressionsaktivität, in das Genom des Mikroorganismus einbringt, so dass die Expression eines oder mehrerer dieser endogenen Gene unter der Kontrolle der eingebrachten, erfindungsgemäßen Expressionseinheiten, gegebenenfalls mit erhöhter spezifischer Expressionsaktivität, erfolgt oder
dh2) ein oder mehrere dieser Gene in das Genom des Mikrorganismus einbringt, so dass die Expression eines oder mehrerer der eingebrachten Gene unter der Kontrolle der endogenen, erfindungsgemäßen Expressionseinheiten, gegebenenfalls mit erhöhter spezifischer Expressionsaktivität, erfolgt oder
dh3) ein oder mehrere Nukleinsäurekonstrukte, enthaltend eine erfindungsgemäße Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

Eine weiter bevorzugte Ausführungsform des vorstehend beschriebenen Verfahrens zur Herstellung von Threonin ist dadurch gekennzeichnet, dass die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Glycerinaldehyd-3-Phosphat Dehydrogenase-Aktivität, 3-Phosphoglycerat Kinase-Aktivität, Pyruvat Carboxylase-Aktivität, Triosephosphat Isomerase-Aktivität, Threonin Synthase-Aktivität, Aktivität eines Threonin Export-Carriers, Transaldolase-Aktivität, Transketolase-Aktivität, Glucose-6-Phosphat-dehydrogenase-Aktivität, Malat-Qinon-Oxidoreductase-Aktivität, Homoserin-Kinase-Aktivität, Biotin-Ligase-Aktivität, Phosphoenolpyruvat-Carboxylase-Aktivität, Threonin-Efflux-Protein-Aktivität, Protein OpcA-Aktivität, 1-Phosphofructokinase-Aktivität, 6-Phosphofructokinase-Aktivität, Fruktose 1,6 bisphosphatase-Aktivität, 6-Phosphogluconat-Dehydrogenase und Homoserin-Dehydrogenase-Aktivität aufweisen.

Eine weiter besonders bevorzugte Ausführungsform des vorstehend beschriebenen Verfahrens zur Herstellung von Threonin ist dadurch gekennzeichnet, dass die genetisch veränderten Mikroorganismen im Vergleich zum Wildtyp zusätzlich eine reduzierte Aktivität, mindestens einer der Aktivitäten, ausgewählt aus der Gruppe Threonin Dehydratase-Aktivität, Homoserin O-Acetyltransferase-Aktivität, Serin-Hydroxymethyltransferase-Aktivität, O-Acetylhomoserin-Sulfhydrylase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthetase-Aktivität, Dihydrodipicolinat Reduktase-Aktivität, Asparaginase-Aktivität, Aspartat-Decarboxylase-Aktivität, Lysin-Exporter-Aktivität, Acetolactat-Synthase-Aktivität , Ketol-Aid-Reductoisomerase-Aktivität, Branched chain aminotransferase- Aktivität , Coenzym B12-abhängige Methionin Synthase- Aktivität , Coenzym B12-unabhängige Methion Synthase- Aktivität, Dihydroxy-acid Dehydratase- Aktivität und Diaminopicolinat Decarboxylase-Aktivität aufweisen.

Diese zusätzlichen, erhöhten oder reduzierten Aktivitäten mindestens einer der vorstehend beschriebenen Aktivitäten können, müssen aber nicht durch eine erfindungsgemäße Nukleinsäure mit Promotoraktivität und/oder eine erfindungsgemäße Expressionseinheit verursacht sein.

Unter dem Begriff der "Aktivität" eines Proteins wird bei Enzymen die Enzymaktivität des entsprechenden Proteins, bei anderen Proteinen, beispielsweise Struktur oder Transport-Proteinen die physiologische Aktivität der Proteine verstanden.

Die Enzyme sind in der Regel in der Lage ein Substrat in ein Produkt umzuwandeln bzw. diesen Umwandlunsgschritt zu katalysieren.

Dementsprechend wird unter der "Aktivität" eines Enzyms die in einer bestimmten Zeit durch das Enzym umgesetzte Menge Substrat bzw. gebildete Menge Produkt verstanden.

Bei einer erhöhten Aktivität im Vergleich zum Wildtyp wird somit im Vergleich zum Wildtyp in einer bestimmten Zeit durch das Enzym die umgesetzte Menge Substrat bzw. die gebildete Menge Produkt erhöht.

Vorzugsweise beträgt diese Erhöhung der "Aktivität" bei allen vorstehend und nachstehend beschriebenen Aktivitäten mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der "Aktivität des Wildtyps".

Bei einer reduzierten Aktivität im Vergleich zum Wildtyp wird somit im Vergleich zum Wildtyp in einer bestimmten Zeit durch das Enzym die umgesetzte Menge Substrat bzw. die gebildete Menge Produkt reduziert.

Unter einer reduzierten Aktivität wird vorzugsweise die teilweise oder im wesentlichen vollständige, auf unterschiedliche zellbiologische Mechanismen beruhende Unterbindung oder Blockierung der Funktionalität diese Enzyms in einem Mikroorganismus verstanden.

Eine Reduzierung der Aktivität umfasst eine mengenmäßige Verringerung eines Enzyms bis hin zu einem im wesentlichen vollständigen Fehlen des Enzyms (d.h. fehlende Nachweisbarkeit der entsprechenden Aktivität oder fehlende immunologische Nachweisbarkeit des Enzyms). Vorzugsweise wird die Aktivität im Mikroorganismus im Vergleich zum Wildtyp um mindestens 5 %, weiter bevorzugt um mindestens 20 %, weiter bevorzugt um mindestens 50 %, weiter bevorzugt um 100 % reduziert. Insbesondere meint "Reduzierung" auch das vollständigen Fehlen der entsprechenden Aktivität.

Die Aktivität bestimmter Enzyme in genetisch veränderten Mikroorganismen sowie im Wildtyp und damit die Erhöhung oder Reduzierung der Enzymaktivität lassen sich nach bekannten Verfahren, wie beispielsweise Enzymassays ermitteln.

Beispielsweise wird unter eine Pyruvatcarboxylase ein Protein verstanden, das die enzymatische Aktivität aufweist, Pyruvat in Oxaloacetat umzuwandeln.

Dementsprechend wird unter einer Pyruvatcarboxylase-Aktivität die in einer bestimmten Zeit durch das Protein Pyruvatcarboxylase umgesetzte Menge Pyruvat bzw. gebildete Menge Oxaloacetat verstanden.

Bei einer erhöhten Pyruvatcarboxylase-Aktivität gegenüber dem Wildtyp wird somit im Vergleich zum Wildtyp in einer bestimmten Zeit durch das Protein Pyruvatcarboxylase die umgesetzte Menge Pyruvat bzw. die gebildete Menge Oxaloacetat erhöht.

Vorzugsweise beträgt diese Erhöhung der Pyruvatcarboxylase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Pyruvatcarboxylase -Aktivität des Wildtyps.

Weiterhin wir beispielsweise unter eine Phosphoenolpyruvat-Carboxykinase-Aktivität die Enzymaktivität einer Phosphoenolpyruvat-Carboxykinase-verstanden.

Unter einer Phosphoenolpyruvat-Carboxykinase wird ein Protein verstanden, das die enzymatische Aktivität aufweist, Oxaloacetat in Phosphoenolpyruvat umzuwandeln.

Dementsprechend wird unter Phosphoenolpyruvat-Carboxykinase-Aktivität die in einer bestimmten Zeit durch das Protein Phosphoenolpyruvat umgesetzte Menge Oxaloacetat bzw. gebildete Menge Phosphoenolpyruvat verstanden.

Bei einer reduzierten Phosphoenolpyruvat-Carboxykinase-Aktivität gegenüber dem Wildtyp wird somit im Vergleich zum Wildtyp in einer bestimmten Zeit durch das Protein Phosphoenolpyruvat-Carboxykinase die umgesetzte Menge Oxaloacetat bzw. die gebildete Menge Phosphoenolpyruvat reduziert.

Eine Reduzierung der Phosphoenolpyruvat-Carboxykinase-Aktivität umfasst eine mengenmäßige Verringerung einer Phosphoenolpyruvat-Carboxykinase bis hin zu einem im wesentlichen vollständigen Fehlen der Phosphoenolpyruvat-Carboxykinase (d.h. fehlende Nachweisbarkeit von Phosphoenolpyruvat-Carboxykinase-Aktivität oder fehlende immunologische Nachweisbarkeit der Phosphoenolpyruvat-Carboxykinase). Vorzugsweise wird die Phosphoenolpyruvat-Carboxykinase-Aktivität im Vergleich zum Wildtyp um mindestens 5 %, weiter bevorzugt um mindestens 20 %, weiter bevorzugt um mindestens 50 %, weiter bevorzugt um 100 % reduziert. Insbesondere meint "Reduzierung" auch das vollständigen Fehlen der Phosphoenolpyruvat-Carboxykinase -Aktivität.

Die zusätzliche Erhöhung von Aktivitäten kann durch verschiedene Wege erfolgen, beispielsweise durch Ausschalten von hemmenden Regulationsmechanismen auf Expressions- und Proteinebene oder durch Erhöhung der Genexpression von Nukleinsäuren kodierend die vorstehend beschriebenen Proteine gegenüber dem Wildtyp.

Die Erhöhung der Genexpression der Nukleinsäuren kodierend die vorstehend beschriebenen Proteine gegenüber dem Wildtyp kann ebenfalls durch verschiedene Wege erfolgen, beispielsweise durch Induzierung des Gens durch Aktivatoren oder wie vorstehend beschrieben durch Erhöhung der Promotoraktivität oder Erhöhung der Expressionsaktivität oder durch Einbringen von einer oder mehrerer Genkopien in den Mikroorganismus.

Unter Erhöhung der Genexpression einer Nukleinsäure codierend ein Protein wird erfindungsgemäß auch die Manipulation der Expression der Mikroorganismus eigenen, endogenen Proteine verstanden.

Dies kann beispielsweise, wie vorstehend beschrieben durch Veränderung der Promotor- und/oder Expressionseinheits-Sequenzen der Gene erreicht werden. Eine solche Veränderung, die eine erhöhte Expressionsrate des Gens zur Folge hat, kann beispielsweise durch Deletion oder Insertion von DNA Sequenzen erfolgen.

Es ist, wie vorstehend beschrieben, möglich, die Expression der endogenen Proteine durch die Applikation exogener Stimuli zu verändern. Dies kann durch besondere physiologische Bedingungen, also durch die Applikation von Fremdsubstanzen erfolgen.

Zur Erzielung einer Erhöhung der Genexpression kann der Fachmann weitere unterschiedliche Maßnahmen einzeln oder in Kombination ergreifen. So kann beispielsweise die Kopienzahl der entsprechenden Gene erhöht werden, oder es kann die Promotor- und Regulationsregion oder die Ribosomenbindungsstelle, die sich stromaufwärts des Strukturgens befindet, mutiert werden. Durch induzierbare Promotoren ist es zusätzlich möglich, die Expression im Verlaufe der fermentativen Produktion zu steigern. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Biontechnology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift 0472869, im US Patent 4,601,893, bei Schwarzer und Pühler (Biotechnology 9, 84-87 (1991), bei Remscheid et al. (Applied and Environmental Microbiology 60,126-132 (1994), bei LaBarre et al. (Journal of Bacteriology 175, 1001-1007 (1993)), in der Patentanmeldung WO 96/15246, bei Malumbres et al. (Gene 134, 15-24 (1993)), in der japanischen Offenlegungsschrift JP-A-10-229891, bei Jensen und Hammer (Biotechnology and Bioengineering 58,.191-195 (1998)), bei Makrides (Microbiological Reviews 60 : 512-538 (1996) und in bekannten Lehrbüchern der Genetik und Molekularbiologie.

Weiterhin kann es für die Produktion von biosynthetischen Produkten, insbesondere L-Lysin, L-Methionin und L-Threonin, vorteilhaft sein, neben der Expression bzw. Verstärkung eines Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

In einer bevorzugten Ausführungsform erfolgt die Erhöhung der Genexpression einer Nukleinsäure kodierend eines der vorstehend beschriebenen Proteine durch Einbringen von mindestens einer Nukleinsäure kodierend ein entsprechendes Protein in den Mikroorganismus. Das Einbringen der Nukleinsäure kann chromsomal oder extrachromosomal erfolgen, also durch Erhöhung der Kopienzahl auf dem Chromosom und oder eine Kopie des Gens auf einem sich replizierenden Plasmid in dem Wirtsmikroorganismus.

Vorzugsweise erfolgt das Einbringen der Nukleinsäure, beispielsweise in Form einer Expressionskassete, enthaltend die Nukleinsäure, chromosomal, insbesondere durch die vorstehend beschriebene SacB-Methode.

Dazu kann prinzipiell jedes Gen, das eines der vorstehend beschriebenen Proteine kodiert verwendet werden.

Bei genomischen Nukleinsäure-Sequenzen aus eukaryontischen Quellen, die Introns enthalten, sind für den Fall das der Wirtsmikroorganismus nicht in der Lage ist oder nicht in die Lage versetzt werden kann, die entsprechenden Proteine zu exprimieren, bevorzugt bereits prozessierte Nukleinsäuresequenzen, wie die entsprechenden cDNAs zu verwenden.

Beispiele für die entsprechenden Gene sind in Tabelle 1 und 2 aufgelistet.

Vorzugsweise erfolgt die Reduzierung der vorstehend beschriebenen Aktivitäten in Mikroorganismen durch mindestens eines der nachfolgenden Verfahren:
- Einbringen mindestens einer sense-Ribonukleinsäuresequenz zur Induktion einer Kosuppression oder einer deren Expression gewährleistenden Expressionskassette
- Einbringen mindestens eines DNA- oder Protein-bindenden Faktors gegen das entsprechede -Gen, -RNA oder -Protein oder einer dessen Expression gewährleistenden Expressionskassette
- Einbringen mindestens einer den RNA-Abbau bewirkenden viralen Nukleinsäuresequenz oder einer deren Expression gewährleistenden Expressionskassette
- Einbringen mindestens eines Konstruktes zur Erzeugung eines Funktionsverlustes, wie beispielsweise die Generierung von Stopp-Kodons oder eine Verschiebungen im Leseraster, an einem Gen beispielsweise durch Erzeugung einer Insertion, Deletion, Inversion oder Mutation in einem Gen. Bevorzugt können Knockout-Mutanten mittels gezielter Insertion in das gewünschte Zielgen durch homologe Rekombination oder Einbringen von sequenzspezifischen Nukleasen gegen das Zielgen generiert werden.
- Einbringen eines Promotors mit reduzierter Promotoraktivität oder einer Expressionseinheit mit reduzierter Expressionsaktivität.

Dem Fachmann ist bekannt, dass auch weitere Verfahren im Rahmen der vorliegenden Erfindung zur Reduzierung seiner Aktivität oder Funktion eingesetzt werden können. Beispielsweise kann auch das Einbringen einer dominant-negativen Variante eines Proteins oder einer deren Expression gewährleistenden Expressionskassette vorteilhaft sein.

Dabei kann jedes einzelne dieser Verfahren eine Verminderung der Proteinmenge, mRNA-Menge und/oder Aktivität eines Proteins bewirken. Auch eine kombinierte Anwendung ist denkbar. Weitere Methoden sind dem Fachmann bekannt und können die Behinderung oder Unterbindung der Prozessierung des Proteins, des Transports des Proteins oder dessen mRNA, Hemmung der Ribosomenanlagerung, Hemmung des RNA-Spleißens, Induktion eines RNA abbauenden Enzyms und/oder Hemmung der Translationselongation oder -termination umfassen.

Im erfindungsgemäßen Verfahren zur Herstellung von biosynthetischen Produkten wird vorzugsweise dem Kultivierungsschritt der genetisch veränderten Mikroorganismen ein Isolieren von biosynthetischen Produkten aus den Mikroorganismen oder/oder aus der Fermentationsbrühe angeschlossen. Diese Schritte können gleichzeitig und/oder vorzugsweisie nach dem Kultivierungsschritt stattfinden.

Die erfindungsgemäßen genetisch veränderten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) zur Produktion von biosynthetischen Produkten, insbesondere L-Lysin, L-Methionin und L-Threonin, kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen

Als Schwefelquelle für die Herstellung von Feinchemikalien, insbesondere von Methionin, können anorganische Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht läßt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff haltige Gasmischungen, wie z. B. Umgebungsluft, in die Kultur eingetragen. Die Temperatur der Kultur liegt normalerweise bei 20°C bis 45°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen Fermentationsbrühen haben üblicherweise eine Trockenmasse von 7,5 bis 25 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf 0 bis 3 g/l gehalten, beziehungsweise abgesenkt wird.

Die Isolierung von biosynthetischen Produkten aus der Fermentationsbrühe und/oder den Mikroorganismen erfolgt in an sich bekannter Weise entsprechend den physikalisch-chemischen Eigenschaften des biosynthetischen Wertprodukts und den biosynthetischen Nebenprodukten.

Die Fermentationsbrühe kann anschließend beispielsweise weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Anschließend kann die Fermentationsbrühe mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann anschließend durch Gefriertrocknung, Sprühtrocknung, Sprühgranulation oder durch anderweitige Verfahren aufgearbeitet werden.

Es ist aber auch möglich die biosynthetischen Produkte, insbesonder L-Lysin, L-Methionin und L-Threonin, weiter aufzureinigen. Hierzu wird die produkthaltige Brühe nach dem Abtrennen der Biomasse einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bei einer Temperatur aufbewahrt werden, bei der die Stabilität des Produktes maximal ist.

Die biosynthetischen Produkte können in unterschiedlichen Formen anfallen, beispielsweise in Form ihrer Salze oder Ester.

Die Identität und Reinheit der isolierten Verbindung(en) kann durch Techniken des Standes der Technik bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

Die Erfindung wird nun anhand der folgenden nicht-limitierenden Beispiele näher beschrieben:

### Beispiel 1

### Konstruktion von Plasmid pClS lysC

Im ersten Schritt der Stammkonstruktion wurde ein allelischer Austausch des lysC Wildtypgens in Corynebakterium glutamicum ATCC13032 durchgeführt. Dabei wurde im lysC Gen ein Nukleotidaustausch durchgeführt, so daß im resultierenden Protein die Aminosäure Thr an der Position 311 durch ein Ile ausgetauscht wurde. Ausgehend von der chromosomalen DNA aus ATCC13032 als Template für eine PCR Reaktion wurde mit den Oligonukleotidprimern SEQ ID NO:5 und SEQ ID NO:6 lysC mit Hilfe des PfuTurbo PCR Systems (Stratagene USA) nach Angaben des Herstellers amplifiziert.
SEQ ID NO:5
5'-GAGAGAGAGACGCGTCCCAGTGGCTGAGACGCATC-3'
SEQ ID NO:6
5'-CTCTCTCTGTCGACGAATTCAATCTTACGGCCTG-3'

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Das amplifizierte Fragment wird an seinem 5'-Ende von einem Sall Restriktionsschnitt und an seinem 3'-Ende von einem Mlul Restriktionsschnitt flankiert. Vor der Klonierung wurde das amplifizierte Fragment durch diese beiden Restriktionsenzyme verdaut und mit GFX^{™}PCR, DNA and Gel Band Purification Kit (Amersham Pharmacia, Freiburg) aufgereinigt.

Das erhaltenen Polynukleotid wurde über die Sall und Mlul Restriktionsschnitte in pCLIK5 MCS integrativ SacB im folgenden pClS genannt (SEQ ID NO: 7) kloniert und in E.coli XL-1 blue transformiert. Eine Selektion auf Plasmid tragende Zellen wurde durch das Ausplattieren auf Kanamycin (20µg/ml) haltigen LB Agar (Lennox, 1955, Virology, 1:190) erreicht. Das Plasmid wurden isoliert und durch Sequenzierung die erwartete Nukleotidsequenz bestätigt. Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet. Das erhaltene Plasmid pClS lysC ist als SEQ ID NO:8 aufgeführt.

### Beispiel 2

### Mutagenese des lysC Gens aus C. glutamicum

Die gerichtete Mutagenese des lysC Gens aus C. glutamicum wurde mit dem QuickChange Kit (Fa. Stratagene/USA) nach Angaben des Herstellers durchgeführt. Die Mutagenese wurde im Plasmid pClS lysC, SEQ ID NO:8 durchgeführt. Für den Austausch von thr 311 nach 311 ile mit Hilfe der Quickchange Methode (Stratagene) wurden folgende Oligonukleotidprimer synthetisiert:
SEQ ID NO:9
5'-CGGCACCACCGACATCATCTTCACCTGCCCTCGTTCCG -3'
SEQ ID NO:10
5'-CGGAACGAGGGCAGGTGAAGATGATGTCGGTGGTGCCG-3'

Der Einsatz dieser Oligonukleotidprimer in der Quickchange Reaktion führt in dem lysC Gen SEQ ID NO:11 zu einem Austausch des Nukleotids in Position 932 (von C nach T). Der resultierende Aminosäureaustausch Thr311 Ile im lysC Gen wurde nach Transformation in E.coli XL1-blue und Plasmidpräparation durch ein Sequenzierungsreaktionen bestätigt. Das Plasmid erhielt die Bezeichnung pClS lysC thr311ile und ist als SEQ ID NO:12 aufgeführt.

Das Plasmid pCIS IysC thr311ile wurde in C. glutamicum ATCC13032 mittels Elektroporation wie bei Liebl, et al. (1989) FEMS Microbiology Letters 53:299-303 beschrieben, transformiert. Modifikationen des Protokolls sind in DE 10046870 beschrieben. Die chromosomale Anordnung des IysC-Lokus einzelner Transformanten wurde mit Standardmethoden durch Southernblot und Hybridisierung wie in Sambrook et al. (1989), Molecular Cloning. A Laboratory Manual, Cold Spring Harbor, beschrieben, überprüft. Dadurch wurde sichergestellt, daß es sich bei den Transformanten um solche handelt, die das transformierte Plasmid durch homologe Rekombination am lysc-Lokus integriert haben. Nach Wachstum solcher Kolonien über Nacht in Medien, die kein Antibiotikum enthalten, werden die Zellen auf ein Saccharose-CM-Agarmedium (10% Saccharose, 10 g/l Glucose; 2,5 g/l NaCl; 2 g/l Harnstoff, 10 g/l Bacto Peptone (Fa. Difco); 10 g/l Hefeextrakt, 22,0 g/L Agar (Difco)) ausplattiert und bei 30°C für 24 Stunden inkubiert.

Da das im Vektor pClS lysC thr311 ile enthaltende sacB Gen Saccharose in ein toxisches Produkt umwandelt, können nur solche Kolonien anwachsen, die das sacB Gen durch einen zweiten homologen Rekombinationsschritt zwischen dem Wildtyp IysC Gen und dem mutierten Gen IysC thr311ile deletiert haben. Während der homologen Rekombination kann entweder das Wildtyp Gen, oder das mutierte Gen zusammen mit dem sacB Gen deletiert werden. Wenn das sacB Gen zusammen mit dem Wildtyp Gen entfernt wird, resultiert eine mutierte Transformante.

Anwachsende Kolonien wurden gepickt, und auf eine Kanamycin-sensitiven Phänotyp hin untersucht. Klone mit deletiertem SacB Gen müssen gleichzeitg Kanamycin sensitives Wachstumsverhalten zeigen. Solche Kan-sensitiven Klone wurde im einem Schüttelkolben auf ihre Lysin-Produktivivät hin untersucht (siehe Beispiel 6). Zum Vergleich wurde der nicht behandelte C. glutamicum ATCC13032 angezogen. Klone mit einer gegenüber der Kontrolle erhöhten Lysin-Produktion wurden selektiert, chromosomale DNA gewonnen und der entsprechende Bereich des IysC Gens durch eine PCR-Reaktion amplifiziert und sequenziert. Ein solcher Klon mit der Eigenschaft erhöhter Lysin-Synthese und nachgewiesener Mutation in lysC an der Stelle 932 wurde mit ATCC130321ysC^{fbr} bezeichnet.

### Beispiel 3

Herstellung eines Integrationsplasmids zur Überexpression des IysC-Gens mit Hilfe der heterologen Expressionseinheit Psod (SEQ. ID. NO. 2)

Zur Amplifizierung der Expressionseinheit des Gens, das für die Superoxiddimutase kodiert, wurden die folgenden Oligonukleotide definiert.
SEQ ID 13:
sod8: 5'-accctggcggaaaccctgagtcg -3'
SEO ID 14:
sod1: 5'-tacgaccagggccacgggtaaaaaatcctttcgtaggtttccgcaccgagcatatacatcttttg-3'

Die Primer wurden in eine PCR-Reaktion mit chromosomaler DNA von C. glutamicum ATCC 13032 eingesetzt. Mit diesem Ansatz konnte ein DNA-Fragment amplifiziert werden, das der erwarteten Größe von 657 bp entsprach.

Zur Amplifizierung des Gens, das für die Aspartokinase kodiert, wurden sie folgenden Oligonukleotide definiert.
SEQ ID 15
IysC2: 5'- cctacgaaaggattttttacccgtggccctggtcgtacag-3'
SEQ ID 16:
IysC6: 5'- gattagtggaacctcgtcgtc-3'

Die Primer wurden in eine PCR-Reaktion mit chromosomaler DNA von C. glutamicum ATCC13032 IysC^{fbr} eingesetzt. Mit diesem Ansatz konnte ein DNA-Fragment amplifiziert werden, das der erwarteten Größe von 1638 bp entsprach.

Die Primer sod1 und ask2 enthalten eine überlappende Sequenz und sind an ihren 5'-Enden homolog zueinander.

Die oben erhaltenen PCR-Produkte wurden als Template für eine weitere PCR einsetzt, in der die folgenden Primer genutzt wurden.
SEQ ID 17:
sod4: 5'-gcggcgcaggattttctaa-3'
SEO ID 18:
lysC4: 5'-tcggttgcctgagtaatgtctt-3'

Mit diesem Ansatz konnte ein DNA-Fragment amplifiziert werden, das der erwarteten Größe von 1811 bp entsprach. Diese Psod/IysC-Fusion wurde dann in den Vektor pCR2.1 (Firma Invitrogen GmbH, Karlsruhe, Deutschland) kloniert. In einem weiteren Schritt wurde die Fusion Psod/IysC aus dem Plasmid pCR2.1 (Firma Invitrogen GmbH, Karlsruhe, Deutschland) als 1773 bp EcoRI-Fragment in den Integrationsvektor pK19 mob sacB SEQ ID NO 19 kloniert, der zuvor mit der Restriktionsendonuklease EcoRI geschnitten worden war. Das resultierende Plasmid wurde mit pk19 mob sacB Psod IysC bezeichnet.
Zur Amplifikation eines 5'-Bereiches des IysC-Gens wurden folgende Oligonukleotide definiert:
SEQ ID 20:
IysC23: 5'- caccgcggctttggacatcactgctac-3'
SEQ ID 21:
IysC24: 5'- cctggggctttagcggatgcgtctca-3'

Die Primer wurden in eine PCR-Reaktion mit chromosomaler DNA von C. glutamicum eingesetzt. Mit diesem Ansatz konnte ein DNA-Fragment amplifiziert werden, das der erwarteten Größe von 674 bp entsprach. Dieses DNA-Fragment wurde in den Vektor pCR2.1 (Firma Invitrogen GmbH, Karlsruhe, Deutschland) kloniert. Ein 787 bp Spel/Xbal-Fragment wurde dann anschließend in den Vektor pK19 mob sacB Psod lysC kloniert, der zuvor mit dem Restriktionsenzym Nhel verdaut worden war. Das resultierende Plasmid wurde mit pK19 mob sacB Psod lysC + US (SEQ ID 22) bezeichnet. Bis zu diesem Schritt wurden alle Klonierungen in Escherichia coli XL-1 Blue (Firma Stratagene, Amsterdam, Nierderlande) durchgeführt.

Mit dem Transformationsplasmid pK19 mobsacB Psod IysC + US wurde dann E. coli Mn522 (Firma Stratagene, Amsterdam, Nierderlande) zusammen mit dem Plasmid pTc15AcglM nach Liebl, et al. (1989) FEMS Microbiology Letters 53:299-303 transformiert. Das Plasmid pTc15AcgIM ermöglicht die Methylierung von DNA nach dem Methylierungsmuster von Corynebacterium glutamicum (DE 10046870). Durch diesen Schritt wird eine anschließende Elektroporation von Corynebacterium glutamicum mit dem Integrationsplasmid pK19 mob sacB Psod IysC + US ermöglicht. Aus dieser Elektroporation und der nachfolgenden Selektion auf CM-Platten mit Kanamycin (25 µg/ml) wurden mehrere Transkonjuganten erhalten. Zur Selektion auf das zweite Rekombinationsereignis, das zur Excision des Vectors samt dem IysC-Promotor und dem IysC-Gen führen soll, wurden diese Transkonjuganten in CM-Medium über Nacht ohne Kanamycin angezogen und anschließend zur Selektion auf CM-Platten mit 10% Saccharose ausplattiert. Das auf dem Vektor pK19 mob sacB vorhandenen sacB-Gen kodiert für das Enzym Laevansucrase und führt bei Wachstum auf Saccharose zur Synthese von Laevan. Da Laevan für C. glutamicum toxisch ist, können nur C. glutamicum Zellen, die das Integrationsplasmid durch den zweiten Rekombinationsschritt verloren haben, auf Saccharose-haltigem Medium wachsen (Jäger et al., Journal of Bacteriology 174 (1992) 5462-5466). 100 Saccharose-resistente Klone wurden auf ihre Kanamycin-Sensitivität hin überprüft. Für 57 der getesteten Klone konnte neben der Resistenz gegenüber Saccharose auch eine Sensitivität gegenüber Kanamycin nachgewiesen werden. Ob auch der gewünschte Austausch der natürlichen Expressionseinheit durch die Psod-Expressionseinheit erfolgt war, wurde mittels Polymerase-Kettenreaktion (PCR) überprüft. Für diese Analyse wurde chromosomale DNA aus dem Ausgangsstamm und 20 Klonen isoliert. Hierzu wurden die jeweiligen Klone mit einem Zahnstocher von der Agarplatte abgenommen und in 100 µl H₂O suspendiert und 10 min bei 95°C aufgekocht. Jeweils 10 µl der erhaltenen Lösung wurden als Template in die PCR eingesetzt. Als Primer wurden Oligonukleotide verwendet, die zur Psod-Expressionseinheit und dem IysC-Gen homolog sind. Die PCR-Bedingungen wurden wie folgt gewählt: Vorabdenaturierung:5 min bei 95°C; Denaturierung 30 sec bei 95°C; Hybridisierung 30 sec bei 55°C; Amplifizierung 2 min bei 72°C; 30 Zyklen,; End-Exrension 5 min bei 72°C. Im Ansatz mit der DNA des Ausgangsstammes konnte durch Wahl der Oligonukleotide kein PCR-Produkt entstehen. Nur bei Klonen, die durch die 2. Rekombination den Austausch des natürlichen Promotors (PlysC) gegen Psod vollzogen haben, wurde ein Bande mit einer Größe von 340 bp erwartet. Insgesamt waren von den getesteten 20 Klonen 2 Klone positiv.

### Beispiel 4

### Aspartokinase (IysC) Assay

C. glutamicum Stämme, die entweder eine chromosomale Copy des lysC^{fbr}-Gens mit dem natürlichen Promotor oder eine chromosomale Copie des Psod lysC^{fbr} Konstruktes enthielten, wurden in MMA-Medium (20 g/l Glucose, 10 g/l (NH₄)₂SO₄, 0,5 g KH₂PO₄, 0,5 g K₂HPO₄, 2,5 g Harnstoff, 5 g NaCl, 400 mg MgSO₄ + 7H₂O, 10 mg FeSO₄* 7H₂O, MnSO₄ * 6 H₂O, 100 mg L-Leucin, 100 mg L-Cystein, 250 mg Pantothensäure, 5 mg Nicotinamid, 100 µg Biotin, 200 µg Thiamin, 15 g MOPS, pH6,8) bei 30°C bis zu einer OD600 von 8 angezogen. Die Zellen wurden bei 4°C abzentrifugiert und das zweimal mit kaltem Tris-HCl-Puffer (0,1 %, pH 8,0) gewaschen. Nach erneuter Zentrifugation wurden die Zellen in kalten Tris-HCl-Puffer (0,1 %, pH 8,0) aufgenommen und eine OD₆₀₀ von 160 eingestellt. Für den Zellaufschluß wurden 1 ml dieser Zellsuspension in 2 ml Ribolyserröhrchen der Fa. Hybaid überführt und in einem Ribolyser der Fa. Hybaid bei einer Rotationseinstellung von 6,0 dreimal für jeweils 30 sec lysiert. Das Lysat wurde durch 30minütige Zentrifugation bei 15.000 rpm bei 4°C in einer Eppendorfzentrifuge geklärt und der Überstand in ein neues Eppendororfcup überführt. Der Proteingehalt wurde nach Bradford, M.M. (1976) Anal. Biochem. 72:248-254 bestimmt.

Die enzymatische Aktivität der Aspartokinase wurde wie folgt durchgeführt. Reaktionsansätze von 1 ml mit 100 mM Tris-HCl (pH8,0), 10 mM MgCl2, 600 mM Hydroxylamin-HCl (pH 73,0 mit 10 N KOH), 4 mM ATP, 200 mM Aspartat (Natriumsalz) und H₂O ad 1 ml wurden für 10 min bei 30°C inkubiert. Der Test wurde durch Zugabe von dem jeweiligen Proteinlysat gestartet und bei 30°C für 30 min inkubiert. Zum Abstoppen der Reaktion wurde 1 ml der Stoplösung (10% Eisenchlorid, 3,3 % Trichloressigsäure, 0,7 N NaCl) zum Reaktionsgemisch hinzugegeben. Nach einem Zentrifugationsschritt wurde OD₅₄₀ des Überstandes gemessen. 1 Unit entspricht dabei 1 nmol Aspartat Hydroxamat, das pro mg Protein pro Minute gebildet wird.

Die Ergebnisse sind in Tabelle 1a gezeigt.

**Tabelle 1a**

| Stamm | spezifische Aktivität |
|---|---|
| | [nmol/mg/min] |
| ATCC 13032 IysC^{fbr} | 19,35 |
| ATCC 13032 Psod IysC^{fbr} | 41,22 |

Die Aktivität der Aspartokinase konnte durch die Integration des Psod IysC Konstruktes in das Chromosom verdoppelt werden.

### Beispiel 5

### Produktion von Lysin

Zur Untersuchung der Auswirkung des Psod lysC Konstruktes auf die Lysin-Produktion wurde der Stämm ATCC13032, ATCC13032 IysC^{fbr} und ATCC13032 Psod IysC^{fbr} auf CM-Platten (10,0 g/L D-glucose, 2,5 g/L NaCl, 2,0 g/L Harnstoff, 10,0 g/L Bacto Pepton (Difco), 5,0 g/L Yeast Extract (Difco), 5,0 g/L Beef Extract (Difco), 22,0 g/L Agar (Difco), autoklaviert (20 min. 121°C)) für 2 Tag bei 30°C angezogen. Anschließend wurden die Zellen von der Platte abgekratzt und in Saline resuspendiert. Für die Hauptkultur wurden 10 ml Medium I und 0,5 g autoklaviertes CaCO₃ (Riedel de Haen) in einem 100 ml Erlenmeyerkolben mit der Zellsuspension bis zu einer OD₆₀₀ von 1,5 beimpft und für 39h auf einem vom Typ Infors AJ118 (Fa. Infors, Bottmingen, Schweiz) bei 220 upm inkubiert. Anschließend wurde die Konzentration des in das Medium ausgeschiedene Lysin bestimmt.

### Medium I:

40g/l Saccharose
60g/l Melasse (auf 100% Zuckergehalt berechnet)
10g/l (NH₄)₂SO₄
0.4g/l MgSO₄*7H₂O
0.6g/l KH₂PO₄
0.3mg/l Thiamin*HCl
1mg/l Biotin(aus einer 1 mg/ml steril flitrierten Stammlösung die mit NH₄OH auf pH 8,0 eingestellt wurde)
2mg/l FeSO₄
2mg/l MnSO₄
mit NH₄OH auf pH 7,8 eingestellt, autoklaviert (121°C, 20 min).
zusäztlich wird Vitamin B12 (Hydroxycobalamin Sigma Chemicals) aus einer Stammlösung (200 µg/ml, steril filtriert) bis zu einer Endkonzentration von 100 µg/l zugegeben

Die Bestimmung der Aminosäurekonzentration erfolgte mittels Hochdruckflüssigkeitschromatographie nach Agilent auf einer Agilent 1100 Series LC System HPLC. Eine Vorsäulenderivatisierung mit Ortho-Pthalaldehyd erlaubt die Quantifizierung der gebildeten Aminosäuren, die Auftrennung des Aminosäuregemisch findet auf einer Hypersil AA-Säule (Agilent) statt.
Das Ergebnis der Untersuchung ist in Tabelle 2a dargestellt

**Tabelle 2a**

| Stamm | L-Lysin (g/l) |
|---|---|
| ATCC13032 | 0 |
| ATCC13032 lysC^{fbr} | 10,15 |
| ATCC13032 Psod lysC^{fbr} | 12,67 |

### Beispiel 6

### Herstellung des Vektors pCLiK5MCS

Zunächst wurden Ampicillinresistenz und Replikationsursprung des Vektors pBR322 mit den Oligonukleotidprimern SEQ ID NO:23 und SEQ ID NO:24mit Hilfe der Polymerase-Kettenreaktion (PCR) amplifiziert.
SEQ ID NO:23
5'-CCCGGGATCCGCTAGCGGCGCGCCGGCCGGCCCGGTGTGAAATACCGCACA G-3'
SEQ ID NO:24
5'-TCTAGACTCGAGCGGCCGCGGCCGGCCTTTAAATTGAAGACGAAAGGGCCTC G-3'

Neben den zu pBR322 komplementären Sequenzen, enthält der Oligonukleotidprimer SEQ ID NO:23 in 5'-3' Richtung die Schnittstellen für die Restriktionsendonukleasen Smal, BamHI, Nhel und Ascl und der Oligonukleotidprimer SEQ ID NO:24 in 5'-3' Richtung die Schnittstellen für die Restriktionsendonukleasen Xbal, Xhol, Notl und Dral. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,1 kb wurde mit dem GFX^{™}PCR, DNA and Gel Band Purification Kit (Amersham Pharmacia, Freiburg) nach Angaben des Herstellers gereinigt. Die stumpfen Enden des DNA-Fragmentes wurden mit dem Rapid DNA Ligation Kit (Roche Diagnostics, Mannheim) nach Angaben des Herstellers miteinander ligiert und der Ligationsansatz nach Standardmethoden wie in Sambrook et al. (Molecular Cloning. A Laboratory Manual, Cold Spring Harbor, beschrieben (1989)), in kompetente E.coli XL-1Blue (Stratagene, La Jolla, USA) transformiert. Eine Selektion auf Plasmid tragende Zellen wurde durch das Ausplattieren auf Ampicillin (50µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK1.

Ausgehend vom Plasmid pWLT1 (Liebl et a1., 1992) als Template für eine PCR Reaktion wurde mit den Oligonukleotidprimern SEQ ID NO:25 und SEQ ID NO:26 eine Kanamycin-Resistenzcassette amplifiziert.
SEQ ID NO:25:
5'-GAGATCTAGACCCGGGGATCCGCTAGCGGGCTGCTAAAGGAAGCGGA-3'
SEQ ID NO:26
5'-GAGAGGCGCGCCGCTAGCGTGGGCGAAGAACTCCAGCA-3'

Neben den zu pWLT1 komplementären Sequenzen, enthält der Oligonukleotidprimer SEQ ID NO: 25 in 5'-3' Richtung die Schnittstellen für die Restriktionsendonukleasen Xbal, Smal, BamHl, Nhel und der Oligonukleotidprimer SEQ ID NO:26 in 5'-3' Richtung die Schnittstellen für die Restriktionsendonukleasen Ascl und Nhel. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 1,3 kb wurde mit dem GFX^{™}PCR, DNA and Gel Band Purification Kit (Amersham Pharmacia, Freiburg) nach Angaben des Herstellers gereinigt. Das DNA-Fragment wurde mit den Restriktionsendonukleasen Xbal und Ascl (New England Biolabs, Beverly, USA) geschnitten und im Anschluß daran erneut mit dem GFX^{™}PCR, DNA and Gel Band Purification Kit (Amersham Pharmacia, Freiburg) nach Angaben des Herstellers gereinigt. Der Vektor pCLiK1 wurde ebenfalls mit den Restriktionsendonukleasen Xbal und Ascl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,1 kb) mit dem GFX^{™}PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Ampicillin (50µg/ml) und Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK2.

Der Vektor pCLiK2 wurde mit der Restriktionsendonuklease Dral (New England Biolabs, Beverly, USA) geschnitten. Nach Elektrophorese in einem 0,8%igen Agarosegel wurde ein ca. 2,3 kb großes Vektorfragment mit dem GFX^{™}PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers religiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK3.

Ausgehend vom Plasmid pWLQ2 (Liebl et al., 1992) als Template für eine PCR Reaktion wurde mit den Oligonukleotidprimern SEQ ID NO:27 und SEQ ID NO:28 der Replikationsursprung pHM1519 amplifiziert.
SEQ ID NO:27:
5'-GAGAGGGCGGCCGCGCAAAGTCCCGCTTCGTGAA-3'
SEQ ID NO:28:
5'-GAGAGGGCGGCCGCTCAAGTCGGTCAAGCCACGC-3'

Neben den zu pWLQ2 komplementären Sequenzen, enthalten die Oligonukleotidprimer SEQ ID NO:27 und SEQ ID NO:28 Schnittstellen für die Restriktionsendonuklease Notl. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,7 kb wurde mit dem GFX^{™}PCR, DNA and Gel Band Purification Kit (Amersham Pharmacia, Freiburg) nach Angaben des Herstellers gereinigt. Das DNA-Fragment wurde mit der Restriktionsendonuklease Notl (New England Biolabs, Beverly, USA) geschnitten und im Anschluß daran erneut mit dem GFX^{™}PCR, DNA and Gel Band Purification Kit (Amersham Pharmacia, Freiburg) nach Angaben des Herstellers gereinigt. Der Vektor pCLiK3 wurde ebenfalls mit der Restriktionsendonuklease Notl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,3kb) mit dem GFX^{™}PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5.

Für die Erweiterung von pCLiK5 um eine "multiple cloning site" (MCS) wurden die beide synthetischen, weitestgehend komplementären Oligonukleotide SEQ ID NO:29 und SEQ ID NO:30, die Schnittstellen für die Restriktionsendonukleasen Swal, Xhol, Aatl, Apal, Asp718, Mlul, Ndel, Spel, EcoRV, Sall, Clal, BamHl, Xbal und Smal enthalten, durch gemeinsames erhitzen auf 95°C und langsames abkühlen zu einem doppelsträngigen DNA-Fragment vereinigt.
SEQ ID NO:29:
5'-TCGAATTTAAATCTCGAGAGGCCTGACGTCGGGCCCGGTACCACGCGTCATAT GACTAGTTCGGACCTAGGGATATCGTCGACATCGATGCTCTTCTGCGTTAATTAAC AATTGGGATCCTCTAGACCCGGGATTTAAAT-3'
SEQ ID NO:30:
5'-GATCATTTAAATCCCGGGTCTAGAGGATCCCAATTGTTAATTAACGCAGAAGAG CATCGATGTCGACGATATCCCTAGGTCCGAACTAGTCATATGACGCGTGGTACCG GGCCCGACGTCAGGCCTCTCGAGATTTAAAT-3'

Der Vektor pCLiK5 wurde mit den Restriktionsendonuklease Xhol und BamHl (New England Biolabs, Beverly, USA) geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 5,0 kb) mit dem GFX^{™}PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem synthetischen Doppelsträngigen DNA-Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5MCS.

Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS ist als SEQ ID NO:31 aufgeführt.

### Beispiel 7

### Herstellung des Plasmids PmetA metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 32 und SEQ ID NO 33, der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein das metA Gen inklusice des nichkodierenden 5'-Bereichs amplifiziert.
SEQ ID NO 32
5'-GCGCGGTACCTAGACTCACCCCAGTGCT -3' und
SEQ ID NO 33
5'-CTCTACTAGTTTAGATGTAGAACTCGATGT -3'

Das erhaltene DNA Fragment von ca. 1,3 kb Größe wurde mit dem GFX^{™}PCR, DNA and Gel Band Purification Kit (Amersham Pharmacia, Freiburg) nach Angaben des Herstellers gereinigt. Im Anschluß daran wurde es mit den Restriktionsenzymen Asp718 und Spel (Roche Diagnostics, Mannheim) gespalten und das DNA Fragment mit GFX^{™}PCR, DNA and Gel Band Purification Kit aufgereinigt.

Der Vektor pClik5MCS SEQ ID NO: 31 wurde mit den Restriktionsenzymen Asp718 und Spel geschnitten und ein 5 kb großes Fragment nach elektrophoretischer Auftrennung mit GFX™PCR, DNA and Gel Band Purification Kit isoliert.

Das Vektorfragment wurde zusammen mit dem PCR-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS PmetA metA ist als SEQ ID NO 34: aufgeführt.

### Beispiel 8

### Herstellung des Plasmids pCLiK5MCS Psod metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 35 und SEQ ID NO 36, der chromosomalen DNA als Template und Pfu Turbo Polymerase (Fa. Stratagene) wurde mit Hilfe der Polymerase-Kettenreaktion (PCR) nach Standardmethoden wie Innis et al. (1990) PCR Protocols. A Guide to Methods and Applications, Academic Press ein DNA Fragment von ca. 200 Basenpaaren aus dem nichtkodierenden 5'-Bereich (Region der Expressionseinheit) der Superoxiddismutase (Psod) amplifiziert.
SEQ ID NO 35
5'-GAGACTCGAGAGCTGCCAATTATTCCGGG-3' und
SEQ ID NO 36
5'-CCTGAAGGCGCGAGGGTGGGCATGGGTAAAAAATCCTTTCG -3'

Das erhaltene DNA Fragment wurde mit dem GFX^{™}PCR, DNA and Gel Band Purification Kit (Amersham Pharmacia, Freiburg) nach Angaben des Herstellers gereinigt.

Ausgehend vom Plasmid PmetA metA SEQ ID 34 als Template für eine PCR Reaktion wurde mit den Oligonukleotidprimern SEQ ID NO 37: und SEQ ID NO 38: ein Teil von metA amplifiziert.
SEQ ID NO 37
5'-CCCACCCTCGCGCCTTCAG -3' und
SEQ ID NO 38
5'-CTGGGTACATTGCGGCCC -3'

Das erhaltene DNA Fragment von ungefähr 470 Basenpaaren wurde mit dem GFX^{™}PCR, DNA and Gel Band Purification Kit nach Angaben des Herstellers gereinigt.

In einer weiteren PCR Reaktion wurden die beiden oben erhaltenen Fragmente gemeinsam als Template eingesetzt. Durch die mit dem Oligonukleotidprimer SEQ ID NO: 36 eingebrachten, zu metA homologen Sequenzen, kommt es im Zuge der PCR-Reaktion zu einer Anlagerung beider Fragmente aneinander und einer Verlängerung zu einem durchgehenden DNA-Strang durch die eingesetzte Polymerase. Die Standardmethode wurde dahingehend modifiziert, dass die verwendeten Oligonukleotidprimer SEQ ID NO: 35 und SEQ ID NO: 38 erst mit Beginn des 2. Zykluses dem Reaktionsansatz zugegeben wurden.

Das amplifizierte DNA Fragment von ungefähr 675 Basenpaaren wurde mit dem GFX^{™}PCR, DNA and Gel Band Purification Kit nach Angaben des Herstellers gereinigt. Im Anschluß daran wurde es mit den Restriktionsenzymen Xhol und Ncol (Roche Diagnostics, Mannheim) gespalten und gelelektrophoretisch aufgetrennt. Anschließend wurde das ca. 620 Basenpaar große DNA Fragment mit GFX^{™}PCR, DNA and Gel Band Purification Kit (Amersham Pharmacia, Freiburg) aus der Agarose aufgereinigt. Das Plasmid PmetA metA SEQ ID NO 34: wurde mit den Restriktionsenzymen Ncol und Spel (Roche Diagnostics, Mannheim) gespalten. Nach gelelektrophoretischer Auftrennung wurde ein ca. 0,7 kb großes metA Fragment mit GFX^{™}PCR, DNA and Gel Band Purification Kit aus der Agarose aufgereinigt.

Der Vektor pClik5MCS SEQ ID NO: 31 wurde mit den Restriktionsenzymen Xhol und Spel (Roche Diagnostics, Mannheim) geschnitten und ein 5 kb großes Fragment nach elektrophoretischer Auftrennung mit GFX^{™}PCR, DNA and Gel Band Purification Kit isoliert.

Das Vektorfragment wurde zusammen mit dem PCR-Fragment und dem metA-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS PSODmetA ist als SEQ ID NO 39: aufgeführt.

### Beispiel 9

### MetA-Aktivitäten

Der Stamm Corynebacterium glutamicum ATCC13032 wurde jeweils mit den Plasmiden pClik5 MCS, pClik MCS PmetA metA, pCLiK5MCS Psod metA, nach der beschriebenen Methode (Liebl, et al. (1989) FEMS Microbiology Letters 53:299-303) transformiert. Die Transformationsmischung wurde auf CM-Platten plattiert, die zusätzlich 20mg/l Kanamycin enthielten, um eine Selektion auf Plasmid-haltige Zellen zu erreichen. Erhaltene Kan-resistente Klone wurden gepickt und vereinzelt.

C. glutamicum Stämme, die eines dieser Plasmidkonstrukte enthielten, wurden in MMA-Medium ((40 g/l Saccharose, 20 g/l (NH₄)₂SO₄, 1 g/l KH₂PO₄, 1 g/l K₂HPO₄, 0,25g/l MgSO₄ x 7H₂O, 54 g Aces, 1 ml CaCl2 (10 g/I), 1 ml Protocatechoat (300 mg/10 ml), 1 ml Spurenelementelösung (10 g/l FeSO₄ x /H₂O, 10 g/l MnSO₄ x H₂O, 2 g/l ZnSO₄ x 7 H₂O, 0,2 g/l CuSO₄, 0,02 g/l NiCl₂ x 6 H₂O),100 µg/l Vitamin B₁₂, 0,3 mg/l Thiamin, 1 mM Leucin, 1 mg/l Pyridoxal HCl, 1 ml Biotin (100 mg/l), pH7,0) bei 30°C über Nacht angezogen. Die Zellen wurden bei 4°C abzentrifugiert und das zweimal mit kaltem Tris-HCl-Puffer (0,1 %, pH 8,0) gewaschen. Nach erneuter Zentrifugation wurden die Zellen in kalten Tris-HCl-Puffer (0,1 %, pH 8,0) aufgenommen und eine OD₆₀₀ von 160 eingestellt. Für den Zellaufschluß wurden 1 ml dieser Zellsuspension in 2 ml Ribolyserröhrchen der Fa. Hybaid überführt und in einem Ribolyser der Fa. Hybaid bei einer Rotationseinstellung von 6,0 dreimal für jeweils 30 sec lysiert. Das Lysat wurde durch 30minütige Zentrifugation bei 15.000 rpm bei 4°C in einer Eppendorfzentrifuge geklärt und der Überstand in ein neues Eppendororfcup überführt. Der Proteingehalt wurde nach Bradford, M.M. (1976) Anal. Biochem. 72:248-254 bestimmt.

Die Messung der enzymatischen Aktivität von MetA wurde wie folgt durchgeführt. Die Reaktionsansätze von 1 ml enthielten 100 mM Kaliumphosphatpuffer (pH 7,5), 5mM MgCl2, 100 µM Acetyl CoA, 5mM L-Homoserine, 500 µM DTNB (Ellmans Reagenz) und Zellextrakt. Der Test wurde durch Zugabe von dem jeweiligen Proteinlysat gestartet und bei Raumtemperatur inkubiert. Es wurde dann eine Kinetik bei 412 nm über 10 min aufgenommen.

Die Ergebnisse sind in Tabelle 3a gezeigt.

**Tabelle 3a**

| Stamm | spezifische Aktivität [nMol/mg/min] |
|---|---|
| ATCC 13032 pClik5MCS | 12,6 |
| ATCC 13032 pClik5MCS PmetA metA | 50,7 |
| ATCC 13032 pClik5MCS Psod metA | 100,7 |

Die Aktivität von MetA konnte durch die Verwendung der heterologen Expressionseinheit Psod erheblich gesteigert werden.

## Patentansprüche

1. Verwendung einer Nukleinsäure mit Promotoraktivität, enthaltend
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist oder
C) eine Nukleinsäuresequenz, die mit der Nukleinsäuresequenz SEQ. ID. NO. 1 unter stringenten Bedingungen hybridisiert oder
D) funktionell äquivalente Fragmente der Sequenzen unter A), B) oder C)
zur Transkription von Genen.

2. Verwendung einer Expressionseinheit, enthaltend eine Nukleinsäure mit Promotoraktivität gemäß Anspruch 1 und zusätzlich funktionell verknüpft eine Nukleinsäuresequenz, die die Translation von Ribonukleinsäuren gewährleistet, zur Expression von Genen.

3. Verwendung nach Anspruch 2, **dadurch gekennzeichnet, dass** die Expressionseinheit enthält:
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist oder
G) eine Nukleinsäuresequenz, die mit der Nukleinsäuresequenz SEQ. ID. NO. 2 unter stringenten Bedingungen hybridisiert oder
H) funktionell äquivalente Fragmente der Sequenzen unter E), F) oder G).

4. Verwendung nach Anspruch 3, **dadurch gekennzeichnet, dass** die Expressionseinheit aus einer Nukleinsäure der Sequenz SEQ. ID. NO. 2 besteht.

5. Nukleinsäure mit Promotoraktivität, enthaltend
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist oder
C) eine Nukleinsäuresequenz, die mit der Nukleinsäuresequenz SEQ. ID. NO. 1 unter stringenten Bedingungen hybridisiert oder
D) funktionell äquivalente Fragmente der Sequenzen unter A), B) oder C), mit der Maßgabe, dass die Nukleinsäure mit der Sequenz SEQ. ID. NO. 1 ausgenommen ist.

6. Expressionseinheit, enthaltend eine Nukleinsäure mit Promotoraktivität gemäß Anspruch 5 und zusätzlich funktionell verknüpft eine Nukleinsäuresequenz, die die Translation von Ribonukleinsäuren gewährleistet.

7. Expressionseinheit nach Anspruch 6, enthaltend
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist oder
G) eine Nukleinsäuresequenz, die mit der Nukleinsäuresequenz SEQ. ID. NO. 2 unter stringenten Bedingungen hybridisiert oder
H) funktionell äquivalente Fragmente der Sequenzen unter E), F) oder G), mit der Maßgabe, dass die Nukleinsäure mit der Sequenz SEQ. ID. NO. 2 ausgenommen ist.

8. Verfahren zur Veränderung oder Verursachung der Transkriptionsrate von Genen in Mikroorganismen im Vergleich zum Wildtyp durch
a) Veränderung der spezifischen Promotoraktivität im Mikroorganismus von endogenen Nukleinsäuren mit Promotoraktivität gemäß Anspruch 1, die die Transkription von endogenen Gene regulieren, im Vergleich zum Wildtyp oder
b) Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a), wobei die Gene in Bezug auf die Nukleinsäuren mit Promotoraktivität heterolog sind.

9. Verfahren nach Anspruch 8, **dadurch gekennzeichnet, dass** die Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a) dadurch erreicht wird, dass man
b1) eine oder mehrere Nukleinsäuren mit Promotoraktivität gemäß Anspruch 1, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität gemäß Anspruch 1, gegebenenfalls mit veränderter spezifischer Promotoraktivität, erfolgt oder
b2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 1, gegebenenfalls mit veränderter spezifischer Promotoraktivität, erfolgt oder
b3) ein oder mehrere Nukleinsäurekonstrukte, enthaltend eine Nukleinsäure mit Promotoraktivität gemäß Anspruch 1, gegebenenfalls mit veränderter spezifischer Promotoraktivität, und funktionell verknüpft eine oder mehrere, zu transkribierende Nukleinsäuren, in den Mikroorganismus einbringt.

10. Verfahren nach Anspruch 8 oder 9, **dadurch gekennzeichnet, dass** man zur Erhöhung oder Verursachung der Transkriptionsrate von Genen in Mikroorganismen im Vergleich zum Wildtyp
ah) die spezifische Promotoraktivität im Mikroorganismus von endogenen Nukleinsäuren mit Promotoraktivität gemäß Anspruch 1, die die Transkription von endogenen Genen regulieren, im Vergleich zum Wildtyp erhöht oder
bh) die Transkription von Genen im Mikroorganismus durch Nukleinsäuren mit Promotoraktivität gemäß Anspruch 1 oder durch Nukleinsäuren mit erhöhter spezifischer Promotoraktivität gemäß Ausführungsform a) reguliert, wobei die Gene in Bezug auf die Nukleinsäuren mit Promotoraktivität heterolog sind.

11. Verfahren nach Anspruch 10, **dadurch gekennzeichnet, dass** die Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit erhöhter spezifischer Promotoraktivität gemäß Ausführungsform a) dadurch erreicht wird, dass man
bh1) eine oder mehrere Nukleinsäuren mit Promotoraktivität gemäß Anspruch 1, gegebenenfalls mit erhöhter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität gemäß Anspruch 1, gegebenenfalls mit erhöhter spezifischer Promotoraktivität, erfolgt oder
bh2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 1, gegebenenfalls mit erhöhter spezifischer Promotoraktivität, erfolgt oder
bh3) ein oder mehrere Nukleinsäurekonstrukte, enthaltend eine Nukleinsäure mit Promotoraktivität gemäß Anspruch 1, gegebenenfalls mit erhöhter spezifischer Promotoraktivität, und funktionell verknüpft eine oder mehrere, zu transkribierende Nukleinsäuren, in den Mikroorganismus einbringt.

12. Verfahren nach Anspruch 8 oder 9, **dadurch gekennzeichnet, dass** man zur Reduzierung der Transkriptionsrate von Genen in Mikroorganismen im Vergleich zum Wildtyp
ar) die spezifische Promotoraktivität im Mikroorganismus von endogenen Nukleinsäuren mit Promotoraktivität gemäß Anspruch 1, die die Transkription der endogenen Gene regulieren, im Vergleich zum Wildtyp reduziert oder
br) Nukleinsäuren mit reduzierter spezifischer Promotoraktivität gemäß Ausführungsform a) in das Genom des Mikrorganismus einbringt, so dass die Transkription endogene Gene unter der Kontrolle der eingebrachten Nukleinsäure mit reduzierter Promotoraktivität erfolgt.

13. Verfahren zur Veränderung oder Verursachung der Expressionsrate eines Gens in Mikroorganismen im Vergleich zum Wildtyp durch
c) Veränderung der spezifischen Expressionsaktivität im Mikroorganismus von endogenen Expressionseinheiten gemäß Anspruch 2 oder 3, die die Expression der endogenen Gene regulieren, im Vergleich zum Wildtyp oder
d) Regulation der Expression von Genen im Mikroorganismus durch Expressionseinheiten gemäß Anspruch 2 oder 3 oder durch Expressionseinheiten gemäß Anspruch 2 oder 3 mit veränderter spezifischer Expressionsaktivität gemäß Ausführungsform c), wobei die Gene im Bezug auf die Expressionseinheiten heterolog sind.

14. Verfahren nach Anspruch 13, **dadurch gekennzeichnet, dass** die Regulation der Expression von Genen im Mikroorganismus durch Expressionseinheiten gemäß Anspruch 2 oder 3 oder durch Expressionseinheiten gemäß Anspruch 2 oder 3 mit veränderter spezifischer Expressionsaktivität gemäß Ausführungsform a) dadurch erreicht wird, dass man
d1) eine oder mehrere Expressionseinheiten gemäß Anspruch 2 oder 3, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten erfolgt oder
d2) ein oder mehrere Gene in das Genom des Mikrorganismus einbringt, so dass die Expression eines oder mehrerer der eingebrachten Gene unter der Kontrolle der endogenen Expressionseinheiten gemäß Anspruch 2 oder 3, gegebenenfalls mit veränderter spezifischer Expressionsaktivität, erfolgt oder
d3) ein oder mehrere Nukleinsäurekonstrukte, enthaltend eine Expressionseinheit gemäß Anspruch 2 oder 3, gegebenenfalls mit veränderter spezifischer Expressionsaktivität, und funktionell verknüpft eine oder mehrere, zu exprimierende Nukleinsäuren, in den Mikroorganismus einbringt.

15. Verfahren nach Anspruch 13 oder 14, **dadurch gekennzeichnet, dass** man zur Erhöhung oder Verursachung der Expressionsrate eines Gens in Mikroorganismen im Vergleich zum Wildtyp
ch) die spezifische Expressionsaktivität im Mikroorganismus von endogenen Expressionseinheiten gemäß Anspruch 2 oder 3, die die Expression der endogenen Gene regulieren, im Vergleich zum Wildtyp erhöht oder
dh) die Expression von Genen im Mikroorganismus durch Expressionseinheiten gemäß Anspruch 2 oder 3 oder durch Expressionseinheiten gemäß Anspruch 2 oder 3 mit erhöhter spezifischer Expressionsaktivität gemäß Ausführungsform a) reguliert, wobei die Gene im Bezug auf die Expressionseinheiten heterolog sind.

16. Verfahren nach Anspruch 15, **dadurch gekennzeichnet, dass** die Regulation der Expression von Genen im Mikroorganismus durch Expressionseinheiten gemäß Anspruch 2 oder 3 oder durch Expressionseinheiten gemäß Anspruch 2 oder 3 mit erhöhter spezifischer Expressionsaktivität gemäß Ausführungsform a) dadurch erreicht wird, dass man
dh1) eine oder mehrere Expressionseinheiten gemäß Anspruch 2 oder 3, gegebenenfalls mit erhöhter spezifischer Expressionsaktivität, in das Genom des Mikroorganismus einbringt, so dass die Expression eines oder mehrerer endogenen Gene unter der Kontrolle der eingebrachten Expressionseinheiten, gegebenenfalls mit erhöhter spezifischer Expressionsaktivität, erfolgt oder
dh2) ein oder mehrere Gene in das Genom des Mikrorganismus einbringt, so dass die Expression eines oder mehrerer der eingebrachten Gene unter der Kontrolle der endogenen Expressionseinheiten gemäß Anspruch 2 oder 3, gegebenenfalls mit erhöhter spezifischer Expressionsaktivität, erfolgt oder
dh3) ein oder mehrere Nukleinsäurekonstrukte, enthaltend eine Expressionseinheit gemäß Anspruch 2 oder 3, gegebenenfalls mit erhöhter spezifischer Expressionsaktivität, und funktionell verknüpft eine oder mehrere, zu exprimierende Nukleinsäuren, in den Mikroorganismus einbringt.

17. Verfahren nach Anspruch 13 oder 14, **dadurch gekennzeichnet, dass** man zur Reduzierung der Expressionsrate von Genen in Mikroorganismen im Vergleich zum Wildtyp
cr) die spezifische Expressionsaktivität im Mikroorganismus von endogenen, Expressionseinheiten gemäß Anspruch 2 oder 3, die die Expression der endogenen Gene regulieren, im Vergleich zum Wildtyp reduziert oder
dr) Expressionseinheiten mit reduzierter spezifischer Expressionsaktivität gemäß Ausführungsform cr) in das Genom des Mikroorganismus einbringt, so dass die Expression endogener Gene unter der Kontrolle der eingebrachten Expressionseinheiten mit reduzierter Expressionsaktivität erfolgt.

18. Verfahren nach einem der Ansprüche 8 bis 17, **dadurch gekennzeichnet, dass** die Gene ausgewählt sind aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Kohlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

19. Verfahren nach Anspruch 18, **dadurch gekennzeichnet, dass** die Proteine aus dem Biosyntheseweg von Aminosäuren ausgewählt sind aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

20. Expressionskassette, umfassend
a) mindestens eine Expressionseinheit gemäß Anspruch 2 oder 3 und
b) mindestens eine weitere, zu exprimierende Nukleinsäuresequenz, und
c) gegebenenfalls weitere genetische Kontrollelemente,
wobei mindestens eine Expressionseinheit und eine weitere, zu exprimierende, Nukleinsäuresequenz funktionell miteinander verknüpft sind und die weitere, zu exprimierende, Nukleinsäuresequenz in Bezug auf die Expressionseinheit heterolog ist.

21. Expressionskassette nach Anspruch 20, **dadurch gekennzeichnet, dass** die weitere, zu exprimierende Nukleinsäuresequenz ausgewählt ist der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen

22. Expressionskassette nach Anspruch 21, **dadurch gekennzeichnet, dass** die Proteine aus dem Biosyntheseweg von Aminosäuren ausgewählt sind aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serin-hydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase

23. Expressionsvektor enthaltend eine Expressionskassette gemäss einem der Ansprüche 20 bis 22.

24. Genetisch veränderter Mikroorganismus, wobei die genetische Veränderung zu einer Veränderung oder Verursachung der Transkriptionsrate von mindestens einem Gen im Vergleich zum Wildtyp führt und bedingt ist durch
a) Veränderung der spezifischen Promotoraktivität im Mikroorganismus von mindestens einer endogenen Nukleinsäure mit Promotoraktivität gemäß Anspruch 1, die die Transkription mindestens eines endogenen Gens reguliert oder
b) Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a), wobei die Gene in Bezug auf die Nukleinsäuren mit Promotoraktivität heterolog sind.

25. Genetisch veränderter Mikroorganismus nach Anspruch 24, **dadurch gekennzeichnet, dass** die Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a) dadurch erreicht wird, dass man
b1) eine oder mehrere Nukleinsäuren mit Promotoraktivität gemäß Anspruch 1, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität gemäß Anspruch 1, gegebenenfalls mit veränderter spezifischer Promotoraktivität, erfolgt oder
b2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 1, gegebenenfalls mit veränderter spezifischer Promotoraktivität, erfolgt oder
b3) ein oder mehrere Nukleinsäurekonstrukte, enthaltend eine Nukleinsäure mit Promotoraktivität gemäß Anspruch 1, gegebenenfalls mit veränderter spezifischer Promotoraktivität, und funktionell verknüpft eine oder mehrere, zu transkribierende Nukleinsäuren, in den Mikroorganismus einbringt.

26. Genetisch veränderter Mikroorganismus nach Anspruch 24 oder 25 mit erhöhter oder verursachter Transkriptionsrate von mindestens einem Gen im Vergleich zum Wildtyp, **dadurch gekennzeichnet, dass**
ah) die spezifische Promotoraktivität im Mikroorganismus von endogenen Nukleinsäuren mit Promotoraktivität gemäß Anspruch 1, die die Transkription von endogenen Genen regulieren, im Vergleich zum Wildtyp erhöht ist oder
bh) die Transkription von Genen im Mikroorganismus durch Nukleinsäuren mit Promotoraktivität gemäß Anspruch 1 oder durch Nukleinsäuren mit erhöhter spezifischer Promotoraktivität gemäß Ausführungsform ah) reguliert wird, wobei die Gene in Bezug auf die Nukleinsäuren mit Promotoraktivität heterolog sind.

27. Genetisch veränderter Mikroorganismus nach Anspruch 26, **dadurch gekennzeichnet, dass** die Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a) dadurch erreicht wird, dass man
bh1) eine oder mehrere Nukleinsäuren mit Promotoraktivität gemäß Anspruch 1, gegebenenfalls mit erhöhter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, gegebenenfalls mit erhöhter spezifischer Promotoraktivität, erfolgt oder
bh2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 1, gegebenenfalls mit erhöhter spezifischer Promotoraktivität, erfolgt oder
bh3) ein oder mehrere Nukleinsäurekonstrukte, enthaltend eine Nukleinsäure mit Promotoraktivität gemäß Anspruch 1, gegebenenfalls mit erhöhter spezifischer Promotoraktivität, und funktionell verknüpft eine oder mehrere, zu transkribierende Nukleinsäuren, in den Mikroorganismus einbringt.

28. Genetisch veränderter Mikroorganismus nach Anspruch 24 oder 25 mit reduzierter Transkriptionsrate von mindetstens einem Gen im Vergleich zum Wildtyp, **dadurch gekennzeichnet dass**,
ar) die spezifische Promotoraktivität im Mikroorganismus von mindestens einer endogenen Nukleinsäure mit Promotoraktivität gemäß Anspruch 1, die die Transkription von mindestens einem, endogenen Gen reguliert, im Vergleich zum Wildtyp reduziert ist oder
br) eine oder mehrere Nukleinsäuren mit reduzierter Promotoraktivität gemäßAusführungsform a) in das Genom des Mikrorganismus eingebracht wurden, so dass die Transkription mindestens eines endogenen Gens unter der Kontrolle der eingebrachten Nukleinsäure mit reduzierter Promotoraktivität erfolgt.

29. Genetisch veränderter Mikroorganismus, wobei die genetische Veränderung zu einer Veränderung oder Verursachung der Expressionsrate mindestens eines Gens im Vergleich zum Wildtyp führt und bedingt ist durch
c) Veränderung der spezifischen Expressionsaktivität im Mikroorganismus von mindestens einer endogenen Expressionseinheiten gemäß Anspruch 2 oder 3, die die Expression mindestens eines endogenen Gens reguliert, im Vergleich zum Wildtyp oder
d) Regulation der Expression von Genen im Mikroorganismus durch Expressionseinheiten gemäß Anspruch 2 oder 3 oder durch Expressionseinheiten gemäß Anspruch 2 oder 3 mit veränderter spezifischer Expressionsaktivität gemäß Ausführungsform a), wobei die Gene im Bezug auf die Expressionseinheiten heterolog sind.

30. Genetisch veränderter Mikroorganismus nach Anspruch 29, **dadurch gekennzeichnet, dass** die Regulation der Expression von Genen im Mikroorganismus durch Expressionseinheiten gemäß Anspruch 2 oder 3 oder durch Expressionseinheiten gemäß Anspruch 2 oder 3 mit veränderter spezifischer Expressionsaktivität gemäß Ausführungsform a) dadurch erreicht wird, dass man
d1) eine oder mehrere Expressionseinheiten gemäß Anspruch 2 oder 3, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten gemäß Anspruch 2 oder 3, gegebenenfalls mit veränderter spezifischer Expressionsaktivität, erfolgt oder
d2) ein oder mehrere Gene in das Genom des Mikrorganismus einbringt, so dass die Expression eines oder mehrerer der eingebrachten Gene unter der Kontrolle der endogenen Expressionseinheiten gemäß Anspruch 2 oder 3, gegebenenfalls mit veränderter spezifischer Expressionsaktivität, erfolgt oder
d3) ein oder mehrere Nukleinsäurekonstrukte, enthaltend eine Expressionseinheit gemäß Anspruch 2 oder 3, gegebenenfalls mit veränderter spezifischer Expressionsaktivität, und funktionell verknüpft eine oder mehrere, zu exprimierende Nukleinsäuren, in den Mikroorganismus einbringt.

31. Genetisch veränderter Mikroorganismus nach Anspruch 29 oder 30 mit erhöhter oder verursachter Expressionsrate mindestens eines Gens im Vergleich zum Wildtyp, **dadurch gekennzeichnet, dass** man
ch) die spezifische Expressionsaktivität im Mikroorganismus von mindestens einer endogenen Expressionseinheiten gemäß Anspruch 2 oder 3, die die Expression der endogenen Gene reguliert, im Vergleich zum Wildtyp erhöht oder
dh) die Expression von Genen im Mikroorganismus durch Expressionseinheiten gemäß Anspruch 2 oder 3 oder durch Expressionseinheiten gemäß Anspruch 2 oder 3 mit erhöhter spezifischer Expressionsaktivität gemäß Ausführungsform a) reguliert, wobei die Gene im Bezug auf die Expressionseinheiten heterolog sind.

32. Genetisch veränderter Mikroorganismus nach Anspruch 31, **dadurch gekennzeichnet, dass** die Regulation der Expression von Genen im Mikroorganismus durch Expressionseinheiten gemäß Anspruch 2 oder 3 oder durch Expressionseinheiten gemäß Anspruch 2 oder 3 mit erhöhter spezifischer Expressionsaktivität gemäß Ausführungsform a) dadurch erreicht wird, dass man
dh1) eine oder mehrere Expressionseinheiten gemäß Anspruch 2 oder 3, gegebenenfalls mit erhöhter spezifischer Expressionsaktivität, in das Genom des Mikroorganismus einbringt, so dass die Expression eines oder mehrerer endogenen Gene unter der Kontrolle der eingebrachten Expressionseinheiten gemäß Anspruch 2 oder 3, gegebenenfalls mit erhöhter spezifischer Expressionsaktivität, erfolgt oder
dh2) ein oder mehrere Gene in das Genom des Mikrorganismus einbringt, so dass die Expression eines oder mehrerer der eingebrachten Gene unter der Kontrolle der endogenen Expressionseinheiten gemäß Anspruch 2 oder 3, gegebenenfalls mit erhöhter spezifischer Expressionsaktivität, erfolgt oder
dh3) ein oder mehrere Nukleinsäurekonstrukte, enthaltend eine Expressionseinheit gemäß Anspruch 2 oder 3, gegebenenfalls mit erhöhter spezifischer Expressionsaktivität, und funktionell verknüpft eine oder mehrere, zu exprimierende Nukleinsäuren, in den Mikroorganismus einbringt.

33. Genetisch veränderter Mikroorganismus nach Anspruch 29 oder 30 mit reduzierter Expressionsrate von mindetstens einem Gen im Vergleich zum Wildtyp, **dadurch gekennzeichnet dass**,
cr) die spezifische Expressionsaktivität im Mikroorganismus von mindestens einer endogenen Expressionseinheit gemäß Anspruch 2 oder 3, die die Expression von mindestens einem edogenen Gen reguliert, im Vergleich zum Wildtyp reduziert ist oder
dr) eine oder mehrere Expressionseinheiten gemäß Anspruch 2 oder 3 mit reduzierter Expressionsaktivität in das Genom des Mikrorganismus eingebracht wurden, so dass die Expression mindestens eines Gens unter der Kontrolle der eingebrachten Expressionseinheit gemäß Anspruch 2 oder 3 mit reduzierter Expressionsaktivität erfolgt.

34. Genetisch veränderter Mikroorganismus, enthaltend eine Expressionseinheit gemäß Anspruch 2 oder 3 und funktionell verknüpft ein zu eprimierendes Gen, wobei das Gen im Bezug auf die Expressionseinheit heterolog ist.

35. Genetisch veränderter Mikroorganismus nach Anspruch 34, enthaltend eine Expressionskasette gemäß einem der Ansprüche 20 bis 22.

36. Genetisch veränderter Mikroorganismus nach einem der Ansprüche 24 bis 35, **dadurch gekennzeichnet, dass** die Gene ausgewählt sind aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

37. Genetisch veränderter Mikroorganismus nach Anspruch 36, **dadurch gekennzeichnet, dass** die Proteine aus dem Biosyntheseweg von Aminosäuren ausgewählt sind aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA248, Protein OpcA, 1-Phosphofruktokinase und 6-Phosphofruktokinase.

38. Verfahren zur Herstellung von biosynthetischen Produkten durch Kultivierung von genetisch veränderten Mikroorganismen gemäß einem der Ansprüche 24 bis 37.

39. Verfahren zur Herstellung von Lysin durch Kultivierung von genetisch veränderten Mikroorganismen gemäß einem der Ansprüche 24, 25, 31 oder 32, **dadurch gekennzeichnet, dass** die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Diaminopimelat- Dehydrogenase, Nukleinsäuren kodierend eine Diaminopimelat-Decarboxylase, Nukleinsäuren kodierend eine Dihydrodipicolinate-Synthetase, Nukleinsäuren kodierend eine Dihydridipicolinate-Reduktase, Nukleinsäuren kodierend eine Glycerinaldehyd-3-Phosphat Dehydrogenase, Nukleinsäuren kodierend eine 3-Phosphoglycerat-Kinase, Nukleinsäuren kodierend eine Pyruvat Carboxylase, Nukleinsäuren kodierend eine Triosephosphat-Isomerase, Nukleinsäuren kodierend einen Transkriptionellen Regulator LuxR, Nukleinsäuren kodierend einen Transkriptionellen Regulator LysR1, Nukleinsäuren kodierend einen Transkriptionellen Regulator LysR2, Nukleinsäuren kodierend eine Malat-Quinon-Oxodoreduktase, Nukleinsäuren kodierend eine Glucose-6-Phosphat-Deydrognease, Nukleinsäuren kodierend eine 6-Phosphogluconat-Dehydrognease, Nukleinsäuren kodierend eine Transketolase, Nukleinsäuren kodierend eine Transaldolase, Nukleinsäuren kodierend einen Lysin Exporter, Nukleinsäuren kodierend eine Biotin-Ligase, Nukleinsäuren kodierend eine Arginyl-t-RNA-Synthetase, Nukleinsäuren kodierend eine Phosphoenolpyruvat-Carboxylase, Nukleinsäuren kodierend eine Fruktose-1,6-bisphosphatase, Nukleinsäuren kodierend ein Protein OPCA, Nukleinsäuren kodierend eine 1-Phosphofructokinase und Nukleinsäuren kodierend eine 6-Phosphofructokinase,

40. Verfahren nach Anspruch 39, **dadurch gekennzeichnet, dass** die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Diaminopimelat- Dehydrogenase-Aktivität, Diaminopimelat-Decarboxylase-Aktivität, Dihydrodipicolinate-Synthetase-Aktivität, Dihydridipicolinate-Reduktase-Aktivität, Glycerinaldehyd-3-Phosphat Dehydrogenase-Aktivität, 3-Phosphoglycerat-Kinase-Aktivität, Pyruvat Carboxylase-Aktivität, Triosephosphat-Isomerase-Aktivität, Aktivität des Transkriptionellen Regulators LuxR, Aktivität des Transkriptionellen Regulators LysR1, Aktivität des Transkriptionellen Regulators LysR2, Malat-Quinon-Oxodoreduktase-Aktivität, Glucose-6-Phosphat-Deydrognease-Aktivität, 6-Phosphogluconat-Dehydrognease-Aktivität, Transketolase-Aktivität, Transaldolase-Aktivität, Lysin-Exporter-Aktivität, Arginyl-t-RNA-Synthetase-Aktivität, Phosphoenolpyruvat-Carboxylase-Aktivität, Fruktose-1,6-bisphosphatase-Aktivität, Protein OpcA-Aktivität, 1-Phosphofructokinase-Aktivität, 6-Phosphofructokinase-Aktivität und Biotin-Ligase-Aktivität aufweisen.

41. Verfahren nach Anspruch 39 oder 40, **dadurch gekennzeichnet, dass** die genetisch veränderten Mikroorganismen im Vergleich zum Wildtyp zusätzlich eine reduzierte Aktivität, mindestens einer der Aktivitäten, ausgewählt aus der Gruppe Threonin Dehydratase-Aktivität, Homoserin O-Acetyltransferase-Aktivität, O-Acetylhomoserin-Sulfhydrylase-Aktivität, Phosphoenolpyruvat-Carboxykinase-Aktivität, Pyruvat-Oxidase-Aktivität, Homoserine-Kinase-Aktivität, Homoserin-Dehydrogenase-Aktivität, Threonin-Exporter-Aktivität, Threonin-Efflux-Protein-Aktivität, Asparaginase-Aktivität, Aspartat-Decarboxylase-Aktivität und Threonin-Synthase-Aktivität aufweisen.

42. Verfahren zur Herstellung von Methionin durch Kultivierung von genetisch veränderten Mikroorganismen gemäß einem der Ansprüche 24, 25, 31 oder 32, **dadurch gekennzeichnet, dass** die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Homoserin Dehydrogenase, Nukleinsäuren kodierend eine Glycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin O-Acetyltransferase, Nukleinsäuren kodierend eine Cystahionin-gamma-Synthase, Nukleinsäuren kodierend eine Cystahionin-beta-Lyase, Nukleinsäuren kodierend eine Serin-Hydroxymethyltransferase, Nukleinsäuren kodierend eine O-Acetylhomoserin-Sulfhydrylase, Nukleinsäuren kodierend eine Methylen-Tetrahydrofolat-Reduktase, Nukleinsäuren kodierend eine Phosphoserin-Aminotransferase, Nukleinsäuren kodierend eine Phosphoserin-Phosphatase, Nukleinsäuren kodierend eine Serine Acetyl-TransferaseNukleinsäuren kodierend eine Cystein-Synthase I, Nukleinsäuren kodierend eine Cystein-Synthase II , Nukleinsäuren kodierend eine Coenzym B12-abhängige Methionin-Synthase, Nukleinsäuren kodierend eine Coenzym B12-unabhängige Methionin-Synthase, Nukleinsäuren kodierend eine Sulfat-Adenylyltransferase, Nukleinsäuren kodierend eine Phosphoadenosin-Phosphosulfat-Reductase, Nukleinsäuren kodierend eine Ferredoxin-Sulfit-Reduktase, Nukleinsäuren kodierend eine Ferredoxin NADPH-Reduktase, Nukleinsäuren kodierend eine Ferredoxin-Aktivität, Nukleinsäuren kodierend ein Protein der Sulfat-Reduktion RXA077, Nukleinsäuren kodierend ein Protein der Sulfat-Reduktion RXA248, Nukleinsäuren kodierend ein Protein der Sulfat-Reduktion RXA247, Nukleinsäuren kodierend einen RXA0655 Regulator und Nukleinsäuren kodierend einen RXN2910 Regulator.

43. Verfahren nach Anspruch 42, **dadurch gekennzeichnet, dass** die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Homoserin Dehydrogenase-Aktivität, Glycerinaldehyd-3-Phosphat Dehydrogenase-Aktivität, 3-Phosphoglycerat Kinase-Aktivität, Pyruvat Carboxylase-Aktivität, Triosephosphat Isomerase-Aktivität, Homoserin O-Acetyltransferase-Aktivität, Cystahionin-gamma-Synthase-Aktivität, Cystahionin-beta-Lyase-Aktivität Serin-Hydroxymethyltransferase-Aktivität, O-Acetylhomoserin-Sulfhydrylase-Aktivität, Methylen-Tetrahydrofolat-Reduktase-Aktivität, Phosphoserin-Aminotransferase-Aktivität, Phosphoserin-Phosphatase-Aktivität, Serin Acetyl-Transferase-Aktivität, Cystein-Synthase Aktivität I-Aktivität, Cystein-Synthase Aktivität II, Coenzym B12-abhängige Methionin-Synthase-Aktivität, Coenzym B12-unabhängige Methionin-Synthase-Aktivität, Sulfat-Adenylyltransferase-Aktivität, Phosphoadenosin-Phosphosulfat-Reductase-Aktivität, Ferredoxin-Sulfit-Reduktase-Aktivität, Ferredoxin NADPH-Reduktase Aktivität, Ferredoxin-Aktivität, Aktivität Proteins der Sulfat-Reduktion RXA077, Aktivität eines Proteins der Sulfat-Reduktion RXA248, Aktivität eines Proteins der Sulfat-Reduktion RXA247, Aktivität eines RXA655-Regulators und Aktivität eines RXN2910-Regulators aufweisen.

44. Verfahren nach Anspruch 42 oder 43, **dadurch gekennzeichnet, dass** die genetisch veränderten Mikroorganismen im Vergleich zum Wildtyp zusätzlich eine reduzierte Aktivität, mindestens einer der Aktivitäten, ausgewählt aus der Gruppe Homoserine-Kinase-Aktivität, Threonin-Dehydratase-Aktivität, Threonin Synthase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthase-Aktivität, Dihydrodipicolinat Reduktase-Aktivität und Diaminopicolinat Decarboxylase-Aktivität aufweisen.

45. Verfahren zur Herstellung von Threonin durch Kultivierung von genetisch veränderten Mikroorganismen gemäß einem der Ansprüche 24, 25, 31 oder 32, **dadurch gekennzeichnet, dass** die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Glycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin-Kinase, Nukleinsäuren kodierend eine Threonin Synthase, Nukleinsäuren kodierend einen Threonin Exporter Carrier, Nukleinsäuren kodierend eine Glucose-6-Phosphat-Dehydrogenase, Nukleinsäuren kodierend eine Transaldolase, Nukleinsäuren kodierend eine Transketolase,, Nukleinsäuren kodierend einer Malat-Quinon-Oxidoreductase, Nukleinsäuren kodierend eine 6-Phosphogluconat-Dehydrogenase, Nukleinsäuren kodierend einen Lysin-Exporter, Nukleinsäuren kodierend eine Biotin-Ligase, , Nukleinsäuren kodierend eine Phosphoenolpyruvat-Carboxylase, Nukleinsäuren kodierend ein Threonin Efflux-Protein, Nukleinsäuren kodierend eine Fruktose-1,6-bisphosphatase, Nukleinsäuren kodierend ein OpcA Protein, Nukleinsäuren kodierend eine 1-Phosphofructokinase, Nukleinsäuren kodierend eine 6-Phosphofructokinase, und Nukleinsäuren kodierend eine Homoserin-Dehydrogenase

46. Verfahren nach Anspruch 45, **dadurch gekennzeichnet, dass** die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Glycerinaldehyd-3-Phosphat Dehydrogenase-Aktivität, 3-Phosphoglycerat Kinase-Aktivität, Pyruvat Carboxylase-Aktivität, Triosephosphat Isomerase-Aktivität, Threonin Synthase-Aktivität, Aktivität eines Threonin Export-Carriers, Transaldolase-Aktivität, Transketolase-Aktivität, Glucose-6-Phosphat-dehydrogenase-Aktivität, Malat-Qinon-Oxidoreductase-Aktivität, Homoserin-Kinase-Aktivität, Biotin-Ligase-Aktivität, Phosphoenolpyruvat-Carboxylase-Aktivität, Threonin-Efflux-Protein-Aktivität, Protein OpcA-Aktivität, 1-Phosphofructokinase-Aktivität, 6-Phosphofructokinase-Aktivität, Fruktose 1,6 bisphosphatase-Aktivität, 6-Phosphogluconat-Dehydrogenase und Homoserin-Dehydrogenase-Aktivität aufweisen.

47. Verfahren nach Anspruch 45 oder 46, **dadurch gekennzeichnet, dass** die genetisch veränderten Mikroorganismen im Vergleich zum Wildtyp zusätzlich eine reduzierte Aktivität, mindestens einer der Aktivitäten, ausgewählt aus der Gruppe Threonin Dehydratase-Aktivität, Homoserin O-Acetyltransferase-Aktivität, Serin-Hydroxymethyltransferase-Aktivität, O-Acetylhomoserin-Sulfhydrylase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthetase-Aktivität, Dihydrodipicolinat Reduktase-Aktivität, Asparaginase-Aktivität, Aspartat-Decarboxylase-Aktivität, Lysin-Exporter-Aktivität, Acetolactat-Synthase-Aktivität , Ketol-Aid-Reductoisomerase- Aktivität, Branched chain aminotransferase- Aktivität, Coenzym B12-abhängige Methionin Synthase- Aktivität, Coenzym B12-unabhängige Methion Synthase-Aktivität, Dihydroxy-acid Dehydratase- Aktivität und Diaminopicolinat Decarboxylase-Aktivität aufweisen.

48. Verfahren nach einem der Ansprüche 38 bis 47, **dadurch gekennzeichnet, dass** die biosynthetischen Produkte nach und/oder während des Kultivierungsschrittes aus dem Kultivierungsmedium isoliert und gegebenenfalls aufgereinigt werden.

49. Verwendung der Nukleinsäuresequenz SEQ. ID. NO. 44 als ribosomale Bindungsstelle in Expressionseinheiten, die die Expression von Genen in Bakterien der Gattung Corynebacterium oder Brevibacterium ermöglichen.

50. Verwendung der Nukleinsäuresequenzen SEQ. ID. NOs. 42 oder 43 als -10-Region in Expressionseinheiten, die die Expression von Genen in Bakterien der Gattung Corynebacterium oder Brevibacterium ermöglichen.

51. Expressionseinheit, die die Expression von Genen in Bakterien der Gattung Corynebacterium oder Brevibacterium ermöglicht, enthaltend die Nukleinsäuresequenz SEQ. ID. NO. 44.

52. Expressionseinheit gemäß Anspruch 51, **dadurch gekennzeichnet, dass** die Nukleinsäuresequenz SEQ. ID. NO. 44. als ribosomale Bindungsstelle verwendet wird.

53. Expressionseinheit, die die Expression von Genen in Bakterien der Gattung Corynebacterium oder Brevibacterium ermöglicht, enthaltend mindestens eine der Nukleinsäuresequenzen SEQ. ID. NOs. 42 oder 43.

54. Expressionseinheit gemäß Anspruch 53, **dadurch gekennzeichnet, dass** eine der Nukleinsäuresequenzen SEQ. ID. NOs. 42 oder 43 als -10-Region verwendet wird.
